(19) Europäisches Patentamt
European Patent Office
Office européen des brevets

(11) **EP 1 344 059 B1**

(12) **EUROPEAN PATENT SPECIFICATION**

(45) Date of publication and mention of the grant of the patent:
**10.10.2007 Bulletin 2007/41**

(51) Int Cl.:
*G01N 33/52* (2006.01)    *G01N 33/543* (2006.01)
*G01N 33/58* (2006.01)

(21) Application number: **01986945.2**

(22) Date of filing: **21.12.2001**

(86) International application number:
**PCT/EP2001/015385**

(87) International publication number:
**WO 2002/052263 (04.07.2002 Gazette 2002/27)**

(54) **FLOW THROUGH ASSAY DEVICE, DIAGNOSTIC KIT COMPRISING SAID DEVICE AND USE IN THE DETECTION OF AN ANALYTE IN A SAMPLE**

DURCHFLUSSVORRICHTUNG, DIAGNOSTISCHER KIT DIESE ENTHALTEND UND GEBRAUCH FÜR DEN NACHWEIS VON ANALYTEN IN EINER PROBE

DISPOSITIF D'ESSAI EN CONTINU, TROUSSE DE DIAGNOSTIC COMPRENANT LEDIT DISPOSITIF ET UTILISATION DE CE DISPOSITIF POUR LA DETECTION D'UN ANALYTE DANS UN ECHANTILLON

(84) Designated Contracting States:
**AT BE CH CY DE DK ES FI FR GB GR IE IT LI LU MC NL PT SE TR**

(30) Priority: **22.12.2000 EP 00870321**
**02.02.2001 US 266236 P**

(43) Date of publication of application:
**17.09.2003 Bulletin 2003/38**

(60) Divisional application:
**07111462.3**

(73) Proprietor: **BIO A.R.T. SA**
**1070 Bruxelles (BE)**

(72) Inventor: **FANNES, France**
**B-1410 Waterloo (BE)**

(74) Representative: **Brants, Johan P.E. et al**
**De Clercq, Brants & Partners cv**
**Edgard Gevaertdreef 10a**
**9830 Sint-Martens-Latem (BE)**

(56) References cited:
**EP-A- 0 125 118        EP-A- 0 458 231**
**US-A- 5 958 790**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Field of the invention

The present invention relates to a method for the detection of an analyte present in a sample, and to a method to coat an insoluble porous material with an analyte binding compound or complex.

Background of the invention

The number of membrane-based rapid immunochromatographic devices on the market is continuing to increase at a very quick pace. Major factors that are contributing to this growth include improvements in conjugate technology and a growing understanding among product developers of the general design principles involved. Although today's immunochromatographic devices come in a wide variety of designs with a diverse assortment of housings, most commonly available tests are based on one of two simple formats.

The most common format is the lateral-flow or dipstick design, which has become familiar through its use in physician-office assays as well as in over-counter tests. A less widespread format is the flow-through or transverse-flow design. Regardless of the format being used, achieving a sensitive and reproducible test requires the manufacturer to have an efficient procedure for applying an analyte-binding compound. Said analyte-binding compound is able to specifically bind the analyte to be analysed. A rapid test is an inexpensive, disposable, membrane-based assay that provides visual evidence of the presence of an analyte in a sample.

By definition, rapid tests provide results in a short time, preferably minutes. It is an object of these tests to be convenient, accurate, reliable, inexpensive, disposable and foolproof. They are preferably also easily and unambiguously to interpret, even by users without much experience. Typically, as little as $200\mu l$ of liquid sample is required to perform the test, which is usually complete within 2-5 minutes. No instrumentation is required to perform these tests, which can be used in clinics, laboratories, field locations, and the home - often by inexperienced personnel. The base substrate of a known rapid test is typically a nitrocellulose strip onto which an analyte-binding compound is immobilised, usually an antibody or an antigen. A pad containing dried conjugate is attached to the membrane strip. For the majority of currently available tests, this conjugate pad contains gold particles absorbed with antibodies or antigens specific to the analyte being detected. When the sample is applied to the device, the liquid sample migrates by capillary diffusion through the conjugate pad, rehydrating the gold conjugate and allowing the interaction of the sample with the conjugate.

Gold labels were introduced into membrane-based rapid tests in the late 1980s. Its superior stability, sensitivity and precision make gold suitable for use in rapid test Nevertheless, high-quality gold conjugate requires the utmost care and attention in order to achieve a final stable and sensitive product. Indeed, many poor-quality, poorly characterized products leads to non-reproducible, non-reliable results. To prevent this, gold colloids have to be evaluated ultrastructurally using a transmission electron microscope (TEM). Such an evaluation should enable the manufacturer to compare the diameter of the colloids to that of calibrated standards and to obtain information about particle sphericity and the overall variance in particle diameter. Evenly shaped with an optimal particle size of 40 nm to 20 nm will allow to set up reliable assays. A mere 5% of odd-shaped particles can influence a test result, making it completely non-reproducible. It is for this reason that the production of such high-quality of gold particles is expensive. This stands in contrast with the above given definition that rapid test should be a low-cost assay.

EP 0 207 152 and US Patent No. 5,958,790 disclose a method for qualitative or semi-quantitative determination of an analyte in a test sample based on the flow-through principle comprising a second analyte-binding compound which becomes immobilized upon contact with the reaction zone whereby a colloidal gold label is being attached to the second analyte-binding compound. A color signal generated by the immobilized colloidal gold form the visual signal. In addition, US Patent No. 5,616,467 defines the optimal size of the particles to be 20 nm in order to increase sensitivity and reproducibility of the test.

However, as discussed above the reproducibility and sensitivity of such gold-based tests is quite low. Further optimization is needed to increase throughput, sensitivity and reproducibility and decrease costs of these *in vitro* assays.

Enzyme immunoassay devices whereby a sandwich complex is produced are known from EP 0 458 231. A flow through device is disclosed whereon a sandwich complex is produced using urease as a label. A cascade of reactions is disclosed in order to produce a colored deposit. A main problem is that said known enzyme immunoassays require several procedural steps such as a cascade of reactions in order to produce a colored deposit. It is an object of the present invention to reduce said multiple step reaction mechanism.

EP 0 125 118 discloses an immunoassay whereby enzyme-linked sandwiches are formed on a dipstick surface and whereby an insoluble deposit is formed. Several wash steps are required in obtaining said colored deposit.

US Patent No. 5,958,790 discloses a method for qualitative or semi-qualitative determination of an analyte in a test sample based on a flow-through principle whereby a visual signal is generated by the immobilized colloidal gold particles.

Object of the present invention

The aim of the present invention is thus to provide a cheap, easy, fast, reproducible, sensitive test which is reliable for the detection of analytes In a sample.

The present invention uses conjugates or analyte-binding compounds labeled with enzymes able to form insoluble precipitates when (an) analyte(s) is (are) present in a test solution in a flow-through set up. An object of the present invention is directed towards a further optimization of the rapid-test concept through a balanced consideration of each parameter resulting in a reproducible and sensitive test.

A main object of the present invention is to provide a reliable one-step procedure, in order to obtain a colored deposit, integrated in the flow-through assay device. The use of such a one-step procedure allows to generate a faster, easier, cheaper test system, but also to improve the reliability and reproducibility of the corresponding test. Indeed, by omitting procedural steps, errors, such as pipetting or handling errors, may become limited or even negligible. The use of a one-step detection procedure, as proposed in present invention, in contrast to the use of a multiple step reaction detection mechanism as described in EP 0 458 231 results in this effect. In addition, in the device/method/kit of present invention washing steps are omitted. Such washing steps are essential for the sensitivity/reproducibility for most prior art tests, e.g. EP 0 458 231 and EP 0 125 118. Besides the improvement of above-mentioned features, the device/ method/kit of present invention also allows a permanent record of the results which is not the case when using gold particles. A permanent record allows the person skilled in the art to use obtained results with results obtained from previous/later experiments. This makes comparison of inter-assay-results easier and more reliable. In addition, the production of a kit according to US Patent No. 5,958,790 requires more labour and is more expensive and corresponding test results are less reliable.

As a general conclusion, the present invention describes a faster, easier, cheaper method which is more comparable, reliable and reproducible than already known test methods.

These aims separately, partly combined or all together have been met by the present invention.

Summary of the invention

According to the present invention an assay device is provided for testing the presence of an analyte in a given sample comprising: a multilayer support whereon a first analyte-binding compound or analyte-binding complex, able to bind said analyte present in said sample, is immobilized, whereby said analyte is able to bind a second enzyme labeled analyte-binding compound or enzyme labeled analyte-binding complex forming a sandwich complex, whereby said sandwich complex is able to generate upon contact with a suitable precipitating substrate for said enzyme-label a colored deposit in a one step procedure.

In the present invention a 'complex' is to be understood such that the analyte-binding compound may form part of a cluster of molecules, whereby one or more compounds present in this cluster may bind the analyte separately or cooperatively. Alternatively, the analyte may also form part of complex comprising one or more which might be identical or different analytes. The other members in this cluster or complex may be peptides, proteins, lipids, nucleic acids or organic molecules.

By combining the flow through system with the use of a specific precipitating substrate the inventors found surprisingly that highly reliable, fast, sensitive and high-throughput test conditions could be created for the detection of analytes in a test sample, due to the fact that a color deposit is directly obtained in merely one step. In addition, the production of these assay devices are easy and cheap and does not demand extra high-quality of products.

Another object of the present invention Is directed towards a further optimization of the rapid-test concept through a balanced consideration of each parameter resulting in a reproducible and sensitive test such as composition of the membrane, pore size of the membrane, optimization of the labeled reagent system to detect the analyte, composition of the coating buffer, choice of the analyte-binding compound, application procedure, new blocking procedure (post-coating by immersion during a couple of hours has not been described before), storage and stability of the membrane, stability of the result, costs. Through this optimization procedure all washing steps which were previously needed to clear the background signals can be omitted which makes that the test is not only cheaper but also easier and faster than the test systems described until now.

The present flow-through set up allows a "high throughput" approach which may be necessary when a high number of samples needs to be analyzed. The described invention also allows reproducible testing of test samples which may be essential when used for clinical diagnosis. The Inventors found that the sensitivity of the assay using the device as described by the invention is comparable with the most optimized condition as described for the gold-based tests. These devices can be used for a wide range of applications not only for clinical applications but also for agricultural, environmental and veterinary applications. Such tests can be performed outside the laboratory without laboratory equipment by physicians, laboratory technicians or less trained personnel.

In summary, the present invention relates to an assay device complying with the requirements which are essential for the acceptance of such tests on the market: ease of use, small sample volumes, speed, i.e. within 3-5 minutes, reliability and low sales price. Moreover, these tests also feature the same technical performances, i.e. high sensitivity and specificity, long-term stability as those provided by instrumented testing in laboratory.

According to the present invention, said multilayer support of said assay device of the invention as described above comprises:

a) an upper cover layer of a water-impermeable material having at least one hole, whereby said hole at least partly exposing a test zone,
b) an intermediate porous layer comprising at least one insoluble porous material whereon the first analyte binding compound is able to bind in said test zone, and
c) a lower absorbent layer comprising at least one layer of a hydrophilic material.

According to the present invention the water-impermeable material of said upper cover layer as defined above is chosen from plastic adapted to the sample to be tested comprising polypropylene, polyvinylchloride or styrene-ethylene/butylene styrene (SEBS) (Rubin (1990), Schouten and van der Vegt (1987)). This prevents unwanted background signals caused by hydrophobic or hydrophylic interaction of some of the components present in a sample with the plastic.

The assay device of the present invention comprises at least one hole In the water-impermeable material having a diameter of at least 1 mm overlaying a test zone. The hole limits the surface of the porous membrane to which the sample Is exposed to and, when a large volumes are spotted onto this multilayer device, the hole also helps the liquid to be absorbed by the area defined by the hole. The hole is not limited by the size of the test zone. Both hole and test zone may have any form such as a circle, square, triangle, cross or any regular or irregular surface.

The test zone is part of the porous layer where the first-anayte-binding compound or capturing molecule has been spotted. The test zone may be between 1 to 10 mm wide. A test zone with a size smaller than 1 mm is possible but In such case interpretation, i.e. reading of results may be difficult. A test zone with a size higher than 10 mm is possible but in such case, more volume of reagents is needed. Nevertheless, in some cases the use of a large test zone is crucial, e.g. for the analysis of food samples. The soaking of larger sample volume, e.g. from 0,1 ml to 2 mls may allow concentration onto the membrane of the analyte to be detected. Said test zone may have a diameter of 1, 2, 3, 4, 5, 6, 7, 8, 9, 10, 11, 12, 13, 14, 15, 16, 17, 18, 19 or 20 mm. Preferentially said test zone has a diameter of 3 to 4 mm. For such a zone sample volumes between 5 to 500 $\mu$l are preferentially used. The volume of the sample added is preferentially equal or smaller than the volume of the reagent solution. E.g. if a 15$\mu$l sample is applied, a 25$\mu$l of the reagent solution is used; when a 25-30$\mu$l of sample is used, 50$\mu$l of reagent solution is applied. If the test zone is larger than 4 mm or if sample volumes are larger than 100 $\mu$l the composition of diluent buffer may be modified by the addition of sucrose going from 1% up to 40%. Possible concentrations are 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 and 40%. Sucrose may be added into the diluent buffer in order to slow down the flow rate phenomenon in order to get a lower detection limit and to avoid the appearance of a heterogeneous colored spot.

The assay device of the present invention comprises, preferably an intermediate insoluble porous layer to which an analyte-binding compound or to which a capturing molecule is bound. This layer is capable of filtering complex suspensions, e.g. cellular material from a sample being assayed if the analyte is associated with the cellular material. In the last case, the membrane or filter is selected to have a pore size which permits this separation. Any of a variety of filtering members may be used including glass fiber filters and filters of various synthetic or natural materials. According to present invention, the intermediate insoluble porous material is chosen from a group comprising nylon, nitrocellulose, cellulose, fiberglass, polysulfofone, polyvinylidene difluoride, polyester or any other polymeric material to which biological substances may bind. Preferentially, the intermediate insoluble porous material is nitrocellulose (Advanced Microdevices (PVT) LTD. 21, Industrial Area, Ambala Cantt, 133 001 India). In addition, the present invention suggests that the intermediate insoluble porous material has pores with a diameter between 0.1 and 12 $\mu$m and has a thickness up to 2500 $\mu$m. Membranes with pore sizes of 0.1, 0.2, 0.45, 0.8, 1.2, 3.0, 5.0 8.0 and 12 $\mu$m may be used. Membranes with pore sizes of 0.45, 0.8, 1.2 $\mu$m are the most relevant; membranes with pores of 0.45 $\mu$m and a thickness of 500 $\mu$m are preferentially used. The thickness of the membrane seems to influence the flow rate and the quality of the results.

All of these embodiments of the present invention are based on experimental evidence as described In the example section.

In the present invention the intermediate insoluble porous membrane is a layer such as membrane or filter to which a maximum of first-analyte-binding-compound or capturing molecule can be bound. In some cases, it may be necessary not to load a maximum of this first analyte binding compound or capturing molecule onto this surface. The term "bound" in the present invention is intended to embrace any means for fixing the first analyte-binding compound or capturing molecule to the porous member. In addition, this term includes all means that can be used for setting up covalent and non-covalent binding. The material of the porous member is selected from a material to which the analyte-binding compound or, if used, capturing molecule can be bound. The invention does not exclude the possibility to bind the analyte immediately onto this porous surface.

In case covalent linkage is chosen and the molecules to be bound are proteineous, e.g., antibodies or antigens, the porous membrane has amino group residues or into which such groups have been introduced by chemical means. Amino groups permit a protein to be coupled to it by the well known glutaraldehyde method. Alternatively, antibodies can be coupled to glass fibers through aminosilanes. Other natural or synthetic materials which can be coupled directly or through intermediates to an analyte-binding compound may also be used.

Although covalent linkage of the first analyte-binding compound or the capturing molecule might guarantee more stable situation of the assay, it is shown by he present invention that the use of non-covalent linkages in a flow-through setup also results in reliable assay conditions. As non-covalent linkages are based on charge or hydrophobic interaction, it is more easy and less laborious to perform such linkages. Therefore, a preferred embodiment of present invention is to coat the first analyte-binding compound or the capturing molecule non-covalently on said intermediate insoluble porous member of the assay device.

The coating of the porous layer with the capturing or first-analyte-binding compound is performed preferentially via immersion of the layer in coating and post coating solutions followed by a drying procedure. However, alternatively a spraying or a spotting process can be used for the binding of molecules with high affinity.

Preferably the antibody preparation comprises a monoclonal antibody even though polyclonal antibodies from antisera may be used. In this respect also crude antibody preparations may be used for this purpose. Techniques for polyclonal and monoclonal antibody preparation and/or purification are now well known and require no citation here.

In yet another embodiment of the invention, the porous member of the device of the present invention may have capturing molecules bound to it. As used herein, the term "capturing molecule" is intended to refer to agents which will bind selectively to the first analyte-binding compound. The use of a capturing molecule bound to the porous member makes it possible to simplify development and preparation of the porous member useful in ligand-receptor assays. Here "ligand" is defined as being the analyte, "receptor" can be defined as being the analyte-binding compound. For example, if an analyte-binding compound is bound to the porous member, it may be necessary to modify the binding procedure in order to optimize the binding of each analyte-binding compound required for a panel of assays. However, a single capturing molecule or a mixture of multiples capturing molecules or a mixture multiples analyte-binding compounds such as antigens or antibodies bound to the porous member may be employed in a plurality of assays. As a result, the development effort and manufacturing procedures may be greatly simplified when such a "universal" porous member is possible. Consequently, depending on the strategy used, the coating of the first analyte-binding compound may be situated before or after the assembly of the assay device and may be carried out by the manufacturer or by the person who performs the assay. In addition, assembly of the device itself can also be performed by the person who carries out the assay.

The lower layer of the device of the present invention is an absorbent member or layer having capillary passage ways generally transverse to the upper and lower surfaces. The lower absorbent layer is assembled with the intermediate porous layer in a manner which permits direct communication between the pores or interstices of the porous layer and the capillaries of the absorbent layer. Thus, as a liquid is applied onto the intermediate porous layer and is subsequently absorbed by the lower absorbent layer and saturates it, the liquid is drawn through capillary force into the absorbent member. As a result, flow can be induced through the lower absorbent layer when a liquid sample is applied to the surface of the intermediate porous layer even though the hydrostatic pressure of the fluid is so low that unaided it could not flow through the intermediate layer without the application of pressure to force it through or a vacuum to draw it through. The absorbent layer comprises at least one layer of hydrophilic material in contact with and positioned on the side of the Insoluble porous layer opposite the side of the cover layer.

The selection of material for absorbent layer is not critical and a variety of fibrous filter materials can be used. A useful material is cellulose acetate fibers oriented as in a cigarette filter. Those skilled in the art will appreciate that other absorbent members made of polyester, polyolefin or other materials may be used in place of cellulose acetate. Present invention suggests that the hydrophilic material of the device allows communication between the porous material and the absorbent layer and is preferentially AP120, provided by the company mdi (Advanced Microdevices (PVT) LTD. 21, Industrial Area, Ambala Cantt, 133 001 India). Alternatively, equivalent filter pads may be used. These absorbent layers or filter pads can be provided by several companies involved in membrane technology, e.g. Schleicher & Schuell, Sartorius and Millipore.

As already mentioned above, according to present invention said first and the second analyte-binding compounds are substances which specifically bind the analyte and are chosen from a group comprising peptides, proteins, lipids, nucleic acids and organic molecules. When the porous member has a first analyte-binding compound bound to it, the analyte-binding compound is selected for its ability to selectively bind directly with the analyte. For example, if the analyte is an antigen, the analyte-binding compound may be an antibody, monoclonal or polyclonal which specifically binds the analyte, preferably a monoclonal antibody. If the analyte is an antibody, the analyte-binding compound may be an antigen or anti-antibody. If the analyte is an enzyme, the analyte-binding compound may be a receptor or a substrate for the enzyme. If the analyte is a nucleic acid, for example, RNA or DNA, the receptor may be a complementary oligomer of DNA or RNA or a nucleic acid binding protein.

Preferentially, said first analyte-binding compound and/or said second analyte-binding compound are an antibody which binds specifically the analyte. In this respect, present invention also defines said antibody being preferentially a monoclonal or polyclonal or an antibody preparation thereof. The term "specific binding" implies that there is substantially no cross-reaction of the antibody with other proteins. The term "antibody preparation" covers any solution containing antibodies such as serum or solutions containing any antibody derivative. The antibodies according to the invention may be produced according to techniques which are known to those skilled in the art. Monoclonal antibodies may be prepared using conventional hybridoma technology as described by Kohler and Milstein (1979) (Kohler, F. and Milstein, C. 1995. Continuous cultures of fused cells secreting antibody of predefined specificity. Nature 256: 495-497.). Polyclonal antibodies may also be prepared using conventional technology well known to those skilled in the art, and which comprises inoculating a host animal, such as mouse, with a protein or epitope according to the invention and recovering the immune serum. The present invention also includes fragments of whole antibodies which maintain their binding activity, such as for example Fv, F(ab') and F(ab')2 fragments as well as single chain antibodies.

The present invention further discusses that the first analyte-binding compound might be directly or indirectly coupled to the porous layer of the device. When Indirectly coupled, the device is first coated with a capturing molecule as described above which specifically binds the first analyte-binding compound followed by the coating of the first analyte-binding compound. For example, if the analyte Is an antigen and the first analyte-binding compound is an antibody, for example, a mouse IgG antibody, preferably a monoclonal antibody, the capturing may be an antibody, preferably a monoclonal antibody, against murine IgG. In other cases the first analyte-binding compound may be conjugated with a moiety which binds selectively with the capturing molecule. For example, the moiety may be a hapten and the capturing molecule an antibody against said hapten. A possible hapten is fluorescein. In other cases, the capturing molecule may be avidin or streptavidin. In such case, the first analyte-binding compound will have biotin bound to it. In other cases, the first analyte-binding compound may be nucleic acid oligomer, or have such an oligomer bound to it, and the capturing molecule may be a nucleic acid segment complementary to a portion of the first analyte-binding oligomer which does not impair binding with the analyte. Those skilled in the art will appreciate from the foregoing that a variety of capturing-molecule/first-analyte-binding compound combinations may be employed.

According to the present invention the second analyte-binding compound is labeled with an enzyme which upon interaction with a precipitating substrate results in one step in a colored deposit. It is the use of such a precipitating substrate in such a defined assay device as described by the invention that makes the analysis of the analyte within a test sample cheap, easy, stable and reliable. This second analyte-binding compound can also be referred to as "conjugate".

In analogy with the use of a capturing molecules as described above, the present invention also relates to a diagnostic assay device wherein the analyte is detected indirectly by the use of a detection molecule. Indeed, it is possible that said second analyte-binding compound is further bound by a detection molecule labeled with a different enzyme (E2) than the enzyme present on the second analyte-binding compound (E1), whereby this E2 enzyme upon interaction with a precipitating substrate using a one step procedure results in a colored deposit. In this way, amplification of the signal can be obtained.

Alternatively, said second analyte-binding compound is not enzyme-labeled and is further bound by a detection molecule labeled with an enzyme which upon Interaction with a precipitating substrate using a one step procedure results in a colored deposit.

Indirectly coupling of the first-analyte-binding compound or the indirect detection of the second-analyte binding compound can be realized via an avidin/biotin, streptavidin/biotin antibody/antigen, antibody/hapten, receptor/ligand, sugar/lectin, complementary nucleic acid, i.e. RNA or DNA, or combination thereof, enzyme/substrate, enzyme/cofactor, enzyme/inhibitor or immunoglobulin/ Staphylococcal proteinA interaction.

According to the present invention, said test sample can be chosen from a group comprising cell fractions, serum, whole blood, urine, plasma i.e. for human or animal diagnostic testing; soil, mud, minerals, water, air i.e. for environmental testing; any food materials i.e. for food testing; or any other medium/suspension/hard material which can be used for one of these purposes. The medium analysed can be both solid or liquid in nature. It is evident when solid materials are used, these are first dissolved in a suitable solution. According to the invention, this solution is not always a real "buffer" with at least 2 well balanced components. It may be a strong hypotonic solution such as NaCl alone or an extraction solution such as with alcohol.

According to the present invention, said test sample can be applied undiluted or in a diluted form using a diluent buffer and for which the dilution factor is adapted to the analyte to be detected (claim 23). Said dilution may vary from ½ up to 100.000. Dilutions of ½, 1/3, ¼, 1/5, 1/6, 1/7, 1/8, 1/9, 1/10, 1/15, 1/20, 1/25, 1/30, 1/35, 1/40, 1/45, 1/50, 1/55, 1/60, 1/65, 1/70, 1/75, 1/80, 1/85, 1/90 1/95, 1/100, 1/150, 1/200, 1/250, 1/300, 1/350, 1/400, 1/450, 1/500, 1/550, 1/600, 1/650, 1/700, 1/750, 1/800, 1/850, 1/900, 1/950, 1/1000, 1/1250, 1/1500, 1/1750, 1/2000, 1/2250, 1/2500, 1/2750, 1/3000, 1/3250, 1/3500, 1/3750, 1/4000, 1/4250, 1/4500, 1/4750, 1/5000, 1/5250, 1/5500, 1/5750, 1/6000, 1/6250, 1/6500, 1/6750, 1/7000, 1/7250, 1/7500, 1/7750, 1/8000, 1/8250, 1/8500, 1/8750, 1/9000, 1/9250, 1/9500, 1/9750, 1/10.000, 1/11250, 1/11500, 1/11750, 1/12000, 1/12250, 1/12500, 1/12750, 1/13000, 1/13250, 1/13500, 1/13750, 1/14000, 1/14250, 1/14500, 1/14750, 1/15000, 1/15250, 1/15500, 1/15750, 1/16000, 1/16250, 1/16500, 1/16750, 1/17000, 1/17250, 1/17500, 1/17750, 1/18000, 1/18250, 1/18500, 1/18750, 1/19000, 1/19250, 1/19500, 1/19750, 1/20.000, 1/30.000, 1/40.000, 1/50.000, 1/60.000, 1/70.000, 1/80.000, 1/90.000 and 1/100.000 are possible. The dilution used depends on which sample is used. For example, for serological applications, i.e. samples coming from urine, serum, plasma or cell fractions the dilution range may be from undiluted to 1/200; for microbiological applications, e.g. detection of microbiological contamination in food and water the range of dilutions may be extended to 1/10.000. It is evident that for samples carrying high amounts of the analyte or when using an analyte-binding compound which has a high affinity for the analyte, dilutions up to 100.000 may be performed.

Said diluent buffer has the function to dilute the sample but in certain cases also the effect on the improved presentation of the analyte to be recognized by the analyte-binding compound. Therefore a solubilizing agent such as EDTA or SDS may be included.

In addition, according the analyte(s) to be detected, the composition of the diluent buffer of the sample(s) needs to be adapted for the detection of the analyte. For example, If the sample to be tested is isolated from food, a sonication process may be required in order to dissolve aggregates; if the sample to be tested Is isolated from urine, adjustment of pH may be needed; If the sample contains lipids i.e. "fat sample", delipidation may be requested. An hemolytic solution may be requested in order to hemolyse the red blood cells.In some cases the salinity needs to be adapted. All measures listed may be necessary to prevent clogging of the system or to optimize the condition wherein the analyte needs to bind the analyte-binding compounds. Other examples of such adaptations are for human application: a sample from synovial fluid for the detection of rheumatoid factors has to be treated with hyaluronidase. For microbiological application: a sample from food for the detection of contaminants have sometimes to be enriched through the addition of some nutriments or lysed by the addition of lytic agents into the diluent buffer. The diluent buffer may also comprise a preservative such as thimerosal or sodium azide.

According to the present invention, said analyte is a compound abnormally or normally present or absent in the sample. So, the devices according to the present invention can be used to detect the absence or presence of an analyte in a sample; nevertheless, they can also be applied to evaluate the quantity of a certain analyte into a medium whereby a decrease or an increase in analyte-concentration can be studied. If the device according to the invention is used to diagnose a disease then the analyte is a cellular compound either of intracellular, membrane or extracellular origin. In this case, the term "abnormally" implies that the presence *per se*, an increase or a decrease of the present level or the absence of the analyte is indicative for a disease. Examples of analytes which are "normally present" are LH and TSH; these are normally present and may abnormally increase or decrease in certain diseases. According to present invention, said compound is selected from the group comprising antigens and antibodies; whereby said antigen may be chosen from the group comprising any biological agent such as bacteria, viruses, molds, mycobacteria, parasites, pathogens; or molecules such as peptides, proteins, lipids, organic molecules, nucleic acid-oligomers.

According to the present invention, said antigen may be chosen from the group comprising proteins for which the level abnormally increases In certain diseased states or abnormally increases in food material.

According to the present invention said antigen may be a bacterium or a toxin produced by a bacterium in food material or in biological fluids. Examples are: *Salmonella sp., E.coli sp., Listeria sp., Clostridium sp., Stayphylococcus sp.,Campylobacter sp. ,Mycobacterium, Streptococcus*...; i.e. urine, blood, serum, feces, cerebral fluid. Such bacteria are normally absent in safe food or in healthy people.

More specifically, the present invention defines that said antigen may be chosen from the group comprising C-reactive protein (CRP), troponin, myoglobin, HCG (human chorionic gonadotrophin, LH (Luteinizing hormone), rheumatoid factors, cardiolipin, centromere (kinetochore proteins), histones,Jo-1 (eponymously named, same as histidyl tRNA transferase,), lupus coagulant, myeloperoxidase, nucleolair auto-antigens, e.a. PM-Scl = polymyositis-Scleroderma, RNP (ribonucleoproteins) (eg. U1RNP), Scl70 (same as topoisomerase 1), Sm (eponymously named as Smith antigen, same as nuclear antigen), SSA/Ro (Sjögren syndrome antigen), SSB/La (Sjögren syndrome antigen, thyroglobulin, cell surface lipoproteins, Thyroid auto-antigens, collagen, ANCA (anti-neutrophil cytyplamsic antibodies) (claim 29). CRP is increased in case of infections, myoglobin in traumatic states, troponin in case of myocardial infarction, rheumatoid factors in case of inflammatory states and HCG in case of pregnancy. Additionally, said protein may be chosen from the group comprising a beta-Adrenoreceptor, TSH-receptor, Insulin receptor, Acetylcholine receptor, Gastrin receptor, pyruvate dehydrogenase. Nevertheless, this assays device can be used for the detection of many other substances and is not limited to the listed examples.

The assay device of the invention can also be used to detect an enzyme by binding a compound carrying a high affinity for this enzyme to the porous member as the assay receptor. This compound might be the substrate of the enzyme or any other protein or molecule that binds specifically to this enzyme. A labeled antibody against the enzyme can be used to detect formation of a receptor-enzyme complex on the porous member. As known by the skilled person in the art also other compounds which have a specifically binding capacity towards this enzyme can be used.

These proteins are recognizable by analyte-binding compounds such as antibodies. Positive controls preferentially contain purified proteins from natural sources; nevertheless, recombinant proteins with a similar folding as found in the natural protein may be used. Consequently, the analyte-binding compound needs to preferentially recognize an analyte with a conformation as found in the natural protein; nevertheless, also analyte-binding compounds recognizing both correctly folded protein and denatured protein might be used.

According to the present invention the analyte might comprise an antibody belonging to any class of immunoglobulins such as IgE, IgG, IgM, IgA, IgD. The presence of a specific antibody in a sample might give indications on stage, location and nature of a disease. An increase of IgE is a measure for allergic reactions and presence of helminthic parasites; the increase of IgG confirms the presence of infections which is already in a extended stage; IgM indicates that infections are present in an early state. IgA can be especially detected in secretions and IgD are present on membranes of B cells. Present invention illustrates the detection of anti-mitochondial antibody type M2 and rheumatoid factors antibodies by the FT method of present invention. However, the FT method of present invention can be used to detect other antibodies, even to detect antibodies from a different Ig class or antibodies which are present on cell surfaces. The examples and figure legends serve to illustrate the invention and are by no way to be understood as limiting the present invention.

The assay device of this invention may also be used in assays for an antibody which employ an antigen as first analyte-binding compound on the solid phase and which use labeled antigen or labeled anti-antibody as the second analyte-binding compound. The latter is particularly suited to allergy specific assays where the first analyte-binding compound is an allergen bound to the porous member and the second analyte-binding compound is an antibody, preferably a monoclonal antibody to IgE. In other cases, the IgG response to allergens may be measured similarly, i.e., by using an antibody, such as a monoclonal antibody against IgG, as the second analyte-binding compound. Other antibody tests which can be carried out in this manner include tests for detection of viral infections, e.g. herpes, rubella, hepatitis, cytomegalovirus, rotavirus, RSV, HTLV-III, HIV or bacterial infections, e.g. *Streptococcus A&B, Chlamydia, M.tuberculosis, M.pneumonlae, H.pylori, Clostridium, E. coll, Listeria, Staphylococcus, Clostridium.*

Additionally, the present invention defines that said nucleic acid oligomer may be chosen from the group comprising DNA, RNA, DNA/RNA hybrid or chemically analogues thereof, genetically modified or not. With DNA or RNA molecules is meant complementary DNA (cDNA), genomic DNA (gDNA), double stranded-DNA (dsDNA), single stranded-DNA (ssDNA), nuclear RNA (nRNA), transfer RNA (tRNA), messenger RNA (mRNA) and ribosomal RNA (rRNA). RNA molecules may also include dsRNA. When nucleic acid oligomers are used which are not isolated from any living organisms, also RNA/DNA hybrid molecules or oligomers consisting of non-natural nucleotides such as inosines might be used. In these cases, the porous member can be coated with a nucleic acid oligomer as a first-analyte-binding compound for the detection of nucleic acid material in a sample. The first-analyte-binding compound may be an oligomer of DNA, for example, complementary to a sequence in the nucleic acid of interest and can be used to bind either RNA or DNA. Subsequently, the detection of the formed complex can be done using a second nucleic acid oligomer complementary to a non-interfering region of the nucleic acid ligand of interest, the second oligomer being labeled to permit detection.

According to present invention, the enzyme-label, which is coupled to said second analyte-binding compound or said detection molecule, reacts in one step with a precipitating substrate and may be chosen from the group comprising horse radish peroxidase (HRP), alkaline phosphatase (AP), and dehydrogenase. Dehydrogenase can be specified as being for instance glucose-6-phosphate dehydrogenase, lactate dehydrogenase or a malate dehydrogenase. For the enzymes glucose oxidase, cholesterol oxidase, urease, β-galactosidase, and lysozyme no precipitating substrates have been defined so far. Nevertheless, the principle for using these in the same context is similar as described for the above mentioned substrates.

In the present invention said enzyme-label is covalently or non-covalently bound to the second analyte-binding compound. For linkage, the second-analyte binding compound should have a reactive group that is complementary to a reactive group on the enzyme-label. For instance, a free carboxyl group of the second-analyte binding protein complements the amino terminus of enzyme-label such that an amide bond can be made. Alternatively, the analyte-binding compound should be capable of modification to have a reactive group that complements a reactive group of the enzyme label. The second analyte-binding compound can be linked to the linking moiety directly or via a spacer moiety. Those of skill in the art will recognize that, while in most instances the analyte-binding compound and enzyme-label will be linked directly, in some instances it may be desirable to space the linking moiety away from either or both parts with a spacing moiety. It will be recognized that virtually any linkage that is stable to the conditions of use and that can be readily formed without denaturing or otherwise degrading the analyte-binding compound and/or enzyme-label may be employed. Thus, the analyte-binding compound and enzyme-label may include virtually any reactive group that is complementary to, i.e. able to covalently react with, the respective terminus of the linking moiety to which it will be attached may be utilized. Suitable groups complementary to the linking moiety amino terminus include, for example, carboxy groups, esters including activated esters such as NHS-esters, acyl azides, acyl halides, acyl nitriles, aldehydes, alkyl sulfonyl halides, halotriazines, imidoesters, isocyanates, isothiocyanates, sulfonate esters, etc. Suitable reactive groups complementary to the linking moiety carboxy terminus include, for example, amines, alcohols, alkyl halides, thiols, hydrazines, diazoalkanes, sulfonate esters, etc. Conditions for forming covalent linkages between a plethora of complementary reactive group pairs are well known. Preferably, each linkage between the linking moiety and the biologically active agent and the masking moiety is an amide. Conditions for linking molecules together having complementary amino and carboxy groups to form amide linkages are well-known (see, e.g., Merrifield 1997 Merrifield, B. 1997. Concept and early development of solid-phase peptide synthesis. Methods Enzymol. 289: 3-13.). Examples of non-covalently linkages were already illustrated above.

According to a preferred embodiment of the present invention, said substrate of HRP may be chosen from the group comprising TMB (tetratmethylbenzidine) and AEC (3-amino-9 ethylcarbazole). When using the horse radish peroxidase, the enzymatic reaction consists of the reaction of 3-3',5-5' tetramethylbenzidine with the enzyme and hydrogen peroxidase forming in one step insoluble blue free radical cation 1-electron oxidation product.

The mentioned substrates are commercially available. Nevertheless, the inventors illustrate in the present invention that the source of the TMB solution may exert an influence on the quality of the obtained results. Also other reagents known in the art may form suitable substrates. AEC produces a red end product that is soluble in alcohol. This precipitable substrate is used mainly in immunoblotting and immunohistochemical staining techniques, cf. histology.

According to a preferred embodiment of the present invention for alkaline phosphatase said substrate may be chosen from the group comprising BCIP (5-bromo-4-chloro-3-indolylphosphate) and BCIP-NBT(5-bromo-4-chloro-3-indolylphosphate-nitrobluetetrazolium). The mentioned substrates are commercially available. Other reagents known in the art are also suitable. When BCIP reacts with alkaline phosphatase, the free halogenated indoxyl derivative dimerizes, forming an insoluble indigo dye. This product is commonly used for immunoblotting and immunohistochemical staining techniques, cf. histology. The BCIP-NBT system is based on the hydrolysis of BCIP and reduction of NBT producing a deep purple reaction product. This reagent may be used for immunoblotting, immunohistochemical staining techniques and *in situ* hybridisation techniques.

For a dehydrogenase, the substrate to be used according to the present invention may be NBT (nitrobluetetrazolium). Nitrobluetetrazollum is also used In analyte detection systems that employ dehydrogenase activity. The mentioned substrate is commercially available. Other precipitable substrates known In the art may be used as well.

Subsequently to the formation of the precipitation, the TMB precipitate may be fixed using a reagent comprising polyvinylic alcohol supplemented with dioctyl sulfosuccinate and dimethyl formamide. Such solution stops the enzymatic reaction and allows permanent record of the result.

The assay device according to the present invention may be used for qualitative (yes/no answer); semi-quantitative (-/++/+++/++++) or quantitative answer. In a preferred embodiment of the invention, the assay device contains at least 1 test zone which may be used for standard(s) and/or positive and/or negative and/or cut-off control(s). With standard is meant a calibrator. As calibrators, a set of at least one analyte solution with known concentration might be used. This helps to evaluate the analyte concentration into a sample quantitatively or semi-quantitatively. The use of a positive built-up control does show if the test works. The use of a built-up cut-off may be used for the qualitative interpretation of results (yes/no answer) control but no quantitative information can be retrieved from it. For specific tests, it might be advisable that more than one test zone is present in the assay device of the invention. For example when using it to test allergy, 1 card with 6 holes for each panel of allergens can be developed. Alternatively, to test the cardiac markers, e.g. troponin, myoglobin, a card whereon the several cardiac markers are tested in the same time would be optimal. Said test zone(s) can be located within the same hole of the upper cover layer or in separate holes.

The present invention also relates to the interpretation of the observed signal using a card system or using a reader. A "card system" can be defined as being a holder where colored spots of different color intensities are indicated. When the test is reproducible the visualized color can be linked to a concentration of the analyte into the tested sample. This holder might comprise holes within these color spots. In this way the color of the holder can be easily compared to the color formed in the assay device by overlapping both devices. Alternatively, a reader measuring the reflectance can be used to monitor color deposits. Any reader known by the skilled person in the art measuring reflectance can be used. In the examples the VISI-CHROMA™ VC-100 of Biophotonics S.A. (Lessines, Belgium) is used; nevertheless, other systems such as reflectance readers may be used, for example Nycomed readers.

The VISI-CHROMA reader which is used by the inventors for the interpretation of results uses a color CCD camera (charge coupled device) allowing an accurate measurement in a tri-stimulus way (3 filters) on a selected area of the membrane. Such technique allows a very accurate measurement of the colored deposit (dot).

The present assay device may be used for controlling/testing/determining food samples. Presence of bacteria, viruses, mycotoxins, toxins, residues of pesticides, residues of antibiotics, residues of chemical substances may be determined. Examples of bacteria are *Salmonella sp., E.coll sp., Staphylococcus sp., Clostridium sp., Campylobacter sp. Listeria sp., Streptococcus sp.*

The present invention illustrates the detection of *Salmonella* by the FT method of present Invention. However, the FT method of present invention can be applied to detect other DNA/RNA comprising biological agents, such as viruses, mycobacteria, all types of prokaryotic and eukaryotic cells. The examples and figure legends serve to illustrate the invention and are by no way to be understood as limiting the present invention.

Examples of toxins are endo- or exotoxins, thermostables or thermolabile. Examples of mycotoxins are aflatoxins type A&B, oxchratoxin. Examples of residues of pesticides are PCB, dithiocarbamates, propamocarbe, benzimidazoles, organocholides. Examples of residues of antibiotics are residues from beta-lactams, tetracyclines, macrolides, quinolones, sulfonamides, aminoglycosides but also antibiotics that are used as animal growth promoters such as bacitracin, tylosin, spiramycin, virginiamycin and avoparcin. For environmental purposes similar antigens may be controlled/tested/determined.

The present method may be applied for the diagnosis and/or monitoring of treatment of diseases. Examples are the detection of viral infections (virus family: adenoviridae, coronoviridae, papovaviridae, retrovoviridae, etc.) bacterial infections (*Yersinia, Aeromonas, Pasteurella, Vibrio, Helicobacter (H. pylori)* species, etc.) and the diagnosis of acute phase proteins (rheumatoid factors, CRP, serum amyloid A, etc.). Major examples of viral infection are HIV (human immunodeficiency virus) causing AIDS and HCV (Hepatitis C virus) infections resulting in cirrhosis and liver failure.

For example, according to present invention the method may be applied for the diagnosis and/or monitoring of treatment of auto-immune diseases induced by organ specific or non organ specific auto-antigens. Table 1 summarises the main organ and non-organ specific antibodies. Tables 2 and 3 list organ and non-organ specific auto-antigens. Non-organ specific auto-antigens generally induce an auto-immune response whereby different organs are involved.

Said non-organ specific (multisystem) auto-immune disease may be chosen from the group of diseases comprising systemic lupus erythematotus (SLE) and other rheumatic diseases, scleroderma with or without Crest syndrome, drug-induced lupus erythematosis (LE), polymyositis with or without scleroderma, primary Sjögren Syndrome, rheumatoid arthritis and other connective tissue diseases (see table 2).

Organ specific antigens primary induce organ specific auto-immune diseases. In such case clinical manifestations originally affect only one organ, e.g liver in case of Primary Biliary Chirrosis but the onset of the disease also may affect other organs.

Said organ specific auto-immune disease may be chosen from the group of diseases comprising Addison's disease, auto-immune haemolytic anemia, chronic active hepatitis, coeliac disease, Goodpasture's syndrome, Grave's thyrotoxicosis, Hashimoto's thyroiditis, idiopathic thrombocytopenic purpura, Juvenile-onset diabetes, late onset diabetes, lens induced uveitis, some male infertility, multiple sclerosis, myasthenia gravis, pemphigoid, primary biliary cirrhosis, pernicious anemia, primary myxoedema, , sympathetic ophtalmia, ulcerative colitis, vasculitis and Wegener's granulomatosis (see table 3) (Lemoine 1992; Humbel; Abuaf et al.).

According to the present invention, the method can be applied for the diagnosis and/or monitoring of treatment of infectious diseases included by viruses, bacteria, molds, mycobacteria or parasites. Examples of antigens which can be detected in case of infectious diseases are HIV, HbsAg, HbsAb, HbeAg, HbeAb, HbcIgM, Malaria, Chlamydia, StrepA, H.pylori, Lyme, Salmonella, E.coli, Syphilis, TB, Dengue and Chagas.

According to the present invention, the method can be applied for the diagnosis and/or monitoring of treatment of allergic diseases or intolerance manifestations Induced by numerous allergens from grasses, weeds, moulds, foods, trees, epidermals and dust. More than 2000 potential allergens have been identified. Examples of above mentioned allergens are: sweet vernal grass, thimothy grass, cultivated oat pollen; common ragweed, western ragweed, dandelion, *Penicillium notatum, Cladosporium herbarum, Candida albicans,* chicken egg white, cow milk, crab, egg yolk; maple, alder, birch, hazelnut, oak; cat and dog epithelium, horse and cow dander; greer or bencard dust.

According to present invention, the method can be applied for use in the testing of cardiac markers. Said cardiac markers may be chosen from the group comprising myoglobin, creatine kinase and troponin. Said inflammatory markers may be chosen from the group comprising C-reactive protein and

According to present invention, the method can be applied for use in testing of bacteria and/or toxins. With 'testing' is meant detecting the presence/absence, increase or decrease of certain antigens. Toxins may include mycotoxins. Examples of bacteria to be detected are *Salmonella sp., E. coli sp., Listeria sp., Clostridium sp., Stayphylococcus sp., Campylobacter sp., Mycobacterium, Streptococcus, Shigella sp, Bacillus sp.*

The present invention also relates to a method, according to the invention, for use in testing of tumor antigens. Examples of antigens which can be detected in case of tumors are AFP, PSA, CEA, CA-15-3 and ferritin.

Examples of antigens which can be detected, using the method in case of drug abuse are methamphetamine, barbiturates, benzodiazepine, amphetamine, morphine, THC, cocaine and profile.

Generally, the present invention also implies a method for the detection of an analyte present in a test sample.

Said method may comprise steps wherein the addition of the first analyte-binding compound, sample, second analyte-binding compound, substrate solution and fixation solution are performed subsequently, one after the other. Interestingly, no washing step is needed after the addition of the substrate. The omission of washing step is due to the specific blocking process of the membrane after the coating of the binding protein onto the membrane which block any remaining free site.

Alternatively, said method may comprise steps wherein the addition of the first analyte-binding compound, sample, second analyte-binding compound, substrate solution and fixation solution are not all performed subsequently, some of these may be premixed in advance before bringing them onto the device. The foregoing has stressed the application of the invention to sequential immunometric assays with monoclonal antibodies, i.e., an immunoassay using a first monoclonal antibody receptor on the porous member and a second monoclonal antibody receptor which is labeled. Sample is added to the porous member followed by labeled antibody. Other assay variants are possible. For example, in the case of an immunometric assay, the labeled antibody and sample may be admixed prior to addition to the porous member. In another embodiment of the invention, the assay device is used to perform competitive assays, i.e., assays in which the first analyte-binding compound is bound to the porous member and for which the analyte in the sample competes with a fixed quantity of labeled analyte added to the sample solution or added following sample addition. Competitive immunoassays are conveniently run in this fashion using an antibody, for example, a monoclonal or polyclonal antibody preparation as first analyte-binding compound bound to the solid phase. Labeled antigen can be added to the sample before the sample is added to the porous layer. Alternatively, it can be added subsequent to addition of the sample or concurrently therewith. When a capturing molecule is used, the sample may be assayed in a variety of ways. For example, in a "sandwich assay", a first analyte-binding compound and a second labeled analyte-binding compound may be combined with the sample to bind the analyte prior to addition to the porous member. Alternatively, a first analyte-binding compound and a sample may be combined prior to addition to the porous member, or added in the sequence of first an analyte-binding compound and then sample, to be followed by addition of a labeled second analyte-binding compound. In such sandwich assays, the capturing molecule is selected to bind the first analyte binding compound and not the labeled second analyte-binding compound. In a competitive assay format, the intensity of the coloration of the deposit is inversely proportional to the concentration of the marker to be detected.

The present invention also describes a method for coating the intermediate porous layer of the device of the present invention comprising following steps:

- cutting membranes into strips
- immersing said strips into an application buffer and the capturing agent or first analyte-binding compound,
- incubating the membrane,
- immersing the membrane in a blocking agent containing 0.2 to 10% blocking agent, whereby the blocking agent may be BSA or any other agent known to block free sites on membranes,
- incubating membranes,
- drying strips, and,
- when storage is needed, the strips are packaged in order to protect membranes from humidity. This is preferentially realized by applying vacuum. Subsequent storage is performed,

wherein the coating (application) buffer features preferentially very low salinity and basic pH (9.1+/-0.1) the analyte-binding compound is present in an excess. The structure of the binding protein may require other types of application buffer.

According to a preferred embodiment of the present invention the intermediate porous layer of the device according to the invention is coated comprising following steps:

- cutting membranes into strips of preferably 0.8 cm wide,
- immersing said strips into a bath brought at RT containing the application buffer and the capturing molecule or the first analyte-binding compound,
- incubating the membrane for 3 hours at RT under gentle agitation,
- immersing the membrane in a blocking agent containing 1% BSA,
- incubating during 3 hours at RT under gentle agitation,
- drying strips at 37°C in an Incubator for 1 hour to overnight, and,
- when storage is needed, packaging of the strips In order to protect membranes from humidity and stored at RT;

wherein the coating (application) buffer features preferentially very low salinity and basic pH (9.1+/-0.1), the analyte-binding compound is present in an excess. All parameters described here might be varied using conditions for the analyte to be detected by the flow-through assay device.

Preferentially the flow through method using a device as described by the invention is performed using the following steps:

- dilute sample 1/2 up to 1/100.000 into the diluent buffer, whereby the diluent buffer is a Tris buffer of low salinity and containing 1 to 5% BSA,. The composition of the diluent may be adapted to the analyte to be detected. Alternatively, an undiluted sample may be used.
- applying 15 μl or one drop) of diluted sample on the membrane,
- allowing the sample to soak for 1 minute at least, i.e. range from 30" up to 1'30" preferentially 45". The first soaking time depends upon the sample to be tested, for example a sample containing a high level of leukocytes or fibrin may soak more slowly,
- applying 25 μl or one drop of conjugate, i.e. anti-CRP coupled to HRP and allow to soak,
- applying 25 μl or one drop of precipitating TMB and allow to soak,
- applying 25 μl or one drop of fixative solution and allow to soak,
- waiting for 2 minutes before reading the result and read within 30 minutes, and,
- covering the colored spot with a scotch band, i.e. type 3M when a long-term storage of the result may be necessary.

As described In the example section, the present invention (example 1) also describes an optimized method for detecting analytes via dot blot based on an enzymatic assay as previously described for the flow-through concept. In this procedure coating a porous layer strip with a first analyte-binding compound or with a capturing molecule comprises the following steps:

- rinsing the membrane strips in alcohol for 1 to 60 seconds,
- incubating the membranes in a cold saline buffer between 1 to 60 minutes,
- adding 0.1 to 10 μl coating solutions,
- incubating the strips for 1 hour to overnight between 4 to 25 °C under agitation,
- drying the membranes for 1 hour to overnight at room temperature
- saturating the membranes with a blocking protein during 15 min to overnight at RT or 4°C, respectively, under agitation, and,
- drying the membranes at 37°C overnight or over weekend.

These strips can be subsequently used in a dot blot or in any equivalent assay procedure. The dot procedure can be performed comprising following steps:

1. diluting the analyte containing samples 1/2 or 1/10.000 into the Tris diluent buffer,
2. incubating 1 to 2 ml of this diluted sample for 5' to 45' (time X) at room temperature (RT) under agitation,
3. washing the membranes at least three times with 1 to 3 ml of Tris (0.01 M) & Tween 20 (0.5% to 1 %) solution for at least 3' each under agitation,
4. adding 1 ml to 2 ml of enzyme-labeled conjugate for which the dilution factor is adapted according to the method,
5. incubating the membrane for at least 5 to 30 minutes at RT (time Y) under agitation,
6. washing the membranes three times for at least 3' each under agitation,
7. adding 1 to 2 ml of chromogen solution (membrane precipitating substrate),
8. incubating the membrane for 1' to 15' (time Z) at RT under agitation,
9. stopping the reaction by addition of 1 ml of stopping solution,
10. analysing the appearance of a blue spot of variable intensity.

All parameters described here might be varied using conditions as described for the above developed flow-through assay device.

The following examples and figure legends merely serve to illustrate the invention and are by no way to be understood as limiting the present invention.

Brief description of the figures

Table 1: Main organ and non-organ specific antibodies present in autoimmune diseases.

Table 2: non organ-specific antigens.

Table 3: organ-specific antigens.

Table 4 (4a-4c): Evaluation of the flow-through method as described in the present invention and other existing methods of immunoturbidimetry and flow-trough method from Nycomed.

Table 5: Evaluation of different membrane lots.

Table 6: Testing of different lots of membranes.

Table 7 (7a-7h): Evaluation of intra-assay reproducibility.

Table 8 (8a-8f): Evaluation of the inter-assays reproducibility.

Table 9 (9a-9c): Evaluation of different TMB agents.

Table 10 (10a-10c): Evaluation of the lowest detection which can be detected.

Table 11 (11a-11b): Detection of anti-mitochondrial type M2 antibodies.

Table 12: Detection of Salmonella thyphimurium.

Table 13: Influence of the composition of the diluent buffer.

Table 14 (14a-14e): Correlation with another rapid method for the detection of CRP by Immunoturbidimetry.

Modes for carrying out the invention

The inventors developed a membrane based rapid test for the detection of analyte(s). The described test has been first developed and optimized for the detection of the *C-reactive protein* (CRP) which is an acute phase protein (examples 1-4, 8, 9, 16). As the inventors proved in the present invention, this test can be used to detect any other analyte in any other media.

Other assays also have been performed for the detection of rheumatoid factors (example 6), M2 antibodies (example 5) or *Salmonella typhimurium* (example 7).

In experiment 1 the presence of the analyte CRP was studied using a polyclonal antiserum anti-CRP (from rabbit origin) at a final concentration of 10μg/ml as capture antibody during the coating procedure.

In example 5 the presence of anti-M2 antibodies was studied; a purified organ specific autoantigen pyruvate dehydrogenase (PDH antigen =M2 antigen) with an enzymatic activity of 0.0634 units/ml was coated.

In example 6 the presence of rheumatoid factors was analysed using purified human immunoglobulins as capture antibody during the coating procedure; a solution of purified human immunoglobulins with a coating concentration of 48 μg/ml was applied.

In example 7 the presence of *Salmonella typhimurium* was studied using a monoclonal antibody as capture protein during the coating procedure at a final concentration of 0,1 mg/ml.

The serum or plasma levels of CRP rise in response to infectious and non-infectious inflammatory processes. The levels may rise from +/- 5 mg/L to 500 mg/L. Measurements of the CRP level are useful in distinguishing viral, i.e. mild increase up to 50 mg/L from severe bacterial infections, i.e. up to 500 mg/L and to check the efficiency of treatment. Detection of low levels of CRP ranging from 1 mg/L to 5 mg/L may be predictive for atherosclerosis.

Several methods can be used for the detection of CRP, such as Radio Immunoassay (RIA), Radial immunodiffusion, Latex agglutination, Turbidimetry, Nephelometry, Enzyme Immuno Assay, e.a. Enzyme Linked Immunosorbent Assay, Fluorescence polarimetry and Membrane based immuno-assays.Radial immunodiffusion or latex agglutination methods only allow a yes or no or semi-quantitative interpretation. These methods in general suffer for a lack of sensitivity or lack of precision.Other methods mentioned above are instruments based tests allowing a quantitative interpretation of results. However for such methods the time required to get the results is generally too long and often the procedure require several washing steps.

Therefore new reliable rapid test systems are needed for the diagnosis of especially inflammatory diseases.

**Example 1: Optimization of a dot method as rapid test.**

The aim of this feasibility study was to develop a rapid method allowing the interpretation of the results within a few minutes without any sophisticated lab instrumentation.

*1.1 Manufacturing of dot strips*

PolyVinyliDeneFluoride membranes from Millipore (cat. 00010 IPVH) with a pore diameter of 0.45 μm were used and cut into strips of 3 mm wide. Strips were rinsed with absolute ethanol (Merck 1.00.983) for 15 seconds and incubated in cold TBS (Tris buffer saline, 10mMTris, 150mM Nacl, pH 9.0+/-0.1) during 10 minutes before starting the coating (application) procedure. The coating volumes are small, i.e. 1 to 5 μl and requires an accurate pipettes or accurate spotting device, e.g. BIO dot system. In this experiment an accurate pipette was used for this purpose. After the coating using a "spotting procedure", the membranes were dried at 37°C followed by a saturation step using a blocking protein ranging from 1% to 5% in concentration. In this experiment BSA was used. This blocking step was performed for 1 hour at RT under agitation. The membranes were subsequently dried overnight at 37°C.

Three experiments were performed using these coated strips whereby for each experiment a different analyte was searched for and/or a different antibody was used.

*1.2 Dot procedure*

According the analyte to be detected, the dot procedures were performed as follows:

1. The samples containing CRP protein -experiment 1- or rheumatoid factors type IgM - experiment 2 - in anti-M2 antibodies -experiment 3 - were diluted 1/50 to 1/100 into Tris diluent buffer
2. 1 to 2 ml of this diluted sample was incubated for 5' to 45' (time X) at room temperature (RT) under agitation.
3. The membranes were washed three times using 1 to 3 ml of Tris (0.01 M) & Tween 20 (0.5% to 1%) solution for 3' each, under agitation.
4. 1 ml to 2 ml of conjugate was used for further incubation.
5. Incubation was performed for 5 to 30 minutes at RT (time Y) under agitation.
6. The membranes were washed for a second time three times for 3' each under agitation (see step 3).
7. 1 to 2 ml of chromogen solution, i.e. membrane precipitating TMB was added.
8. Incubation was performed for 1' to 15' (time Z) at RT under agitation.
9. The reaction was stopped by the addition of 1 ml of stopping or fixative solution10. The appearance of a blue spot of variable intensity was visually analysed.

The conjugate's specificity was chosen according to the analyte to be detected. In experiment 1 the HRP labeled anti-CRP was used at concentration of 0.65g/L; in experiment 2 the human anti-$\mu$ chain IgM (P0322 from Dako, Fab'2) was used at concentration of 1 g/L; in experiment 3: a mix of human anti-gamma chain IgG (P406 from Dako, Fab'2) and human anti-$\mu$ chain IgM (P 322 from Dako, Fab'2) was used at a final concentration of 1 g/L.

For all three experiments the composition of the fixative solution was as follows (for 100 ml):

| | | |
|---|---|---|
| 0.1 g dioctyl-sulfosuccinate | SIGMA | D 0887 |
| 0. 5 ml dimethylformamide | " | D 8654 |
| 1.9 ml polyvinylic alcohol 5% | " | P8136 |
| 76 ml $H_2O$ (filtrated) | | |

*1.3 Interpretation of results*

The color formation was qualitatively evaluated. When a color was formed it was interpreted that the sample is positive, meaning that the test sample contains the analyte.

*1.4 Final conclusion*

Precipitating TMB allowed the development of a blue coloration proportional to the concentration of CRP. Presence of solubilization reagents in the diluent buffer seemed to avoid interference due to other blood protein such as human albumin and immunoglobulins. The coating procedure and the blocking procedure were optimized decreasing the background. The use of new dot strips allowed good results with 5'5'1' incubation times using the enzymatic detection HRP-TMB system. Nevertheless, washing steps were still cumbersome and simple dippings which excludes the use of any washing steps and decreased incubation times 2'2'1' induced too high background.

In addition, with such method, a gentle agitation is required in order to optimize the Immunologic interaction (Ag-Ab). Also, washing steps are obligatory in order to separate the free fraction from the bound fraction. Moreover, this washing procedure may be performed under agitation in order to avoid background such as blue background coloration of the strips.

Finally, this method requires some equipment lab, e.g. rocking agitator and some procedure steps are cumbersome, e.g. washing procedure. Moreover, large volumes of reagents may be used, i.e. 1 ml or more which do not meet the volume requirements for the rapid tests (see introduction).

Due to the above-mentioned arguments, the requirements related to the dot procedure did not meet the specifications of rapid tests.

Technical parameters which should be improved:

1. incubation times

- the target time is 5 min total eg 2':2':1'
- a titration of the conjugate may be performed in order to avoid false positive or false negative results
- current sensitivity (5':5':1') is +/- 10mg/Liter but should be improved
- blue coloration allows a good visual discrimination, a seml-quantitative determination is possible.

2. washing steps: too cumbersome.
3. test design in order to avoid lab equipment

**Example 2: optimization of the flow-through concept**

During the development of a rapid test for the detection of CRP, the inventors focused on the optimization of following technical parameters: composition, quality and pore size of the membrane (see 2.1), optimization of the enzyme labeled reagent system to detect the analyte (here CRP) (see 2.2), composition of the coating buffer (application buffer) (see 2.3), composition and titration of the analyte-binding compound agent, here anti-CRP HRP labeled antibody, application of the binding procedure (coating procedure), blocking procedure (post-coating procedure) (see 2.4), storage/ stability of the membrane, stability of the result retaining of (coloration) and costs.

*2.1 Selection of the membrane (composition - pore size)*

Quality and composition of membrane do exert an influence on the binding of the capturing agent or the first analyte-binding compound (anti-CRP antibody) and the flow rate (sample, conjugate and precipitating substrate). It is thus important to achieve a high consistent binding of the protein in order to get a very good sensitivity, a high specificity and a good reproducibility. Also the pore diameter of the membrane is of great importance.
The inventors found that membranes with pore-diameters of 1.2 $\mu$m and 0.8 Induce a too fast flow rate (within a few seconds) which give a diffuse colored spot difficult to Interpret.

*Brief description of the experiments*

Membranes from S&S with a pore diameter of 1.2 $\mu$m and membrane from mdi with a pore diameter of 0.8 $\mu$m were coated via a spotting procedure using 3 or 5 $\mu$l of an anti-CRP antibody. A sample positive in CRP (concentration 40 mg/L) was diluted 1/25 into a Tris diluent buffer (10mM) containing 1% sucrose. The inventors noticed that a too rapid, i.e. less than 10 seconds flow rate and a large diffusion process. Moreover, after addition of the conjugate, i.e. anti-CRP antibody HRP labeled) the background, i.e. blue background coloration was too intensive in order to allow a good visual reading. The background coloration was tested by the use of the diluent buffer only. In contrast, a membrane from mdi with a pore diameter of 0.45 $\mu$m showed acceptable backgound signals and was subsequently selected. Here the flow rate was slower, i.e. +/- 30 seconds.
In addition, a smaller pore size, i.e. 0.45 versus 0.8 and 1.8 $\mu$m induces a greater surface area, thus a higher binding capacity. Also, a small pore size allows to avoid the radial diffusion of the reagents during the flow through resulting in the formation of a well shaped colored spot.
The final selection was made between type CN and type CLN provided by the company mdi (Advanced Microdevices (PVT) LTD. 21, Industrial Area, Ambala Cantt, 133 001 India), both nitrate cellulose membranes.
Membrane type CN, is a plain membrane which is not supported and is relatively fragile. The inventors observed heterogeneous colorations probably due to air pockets that developed between the absorbent pads and the membrane. Moreover, the flow rate for sample was "too slow", more than 3 minutes.
Membranes type CLN are supported, paper backed meaning "supported" by paper, easy to handle and induce the appearance of a homogeneous coloration. Because the nature of support, there is always a good contact between the absorbent and the membrane, i.e. no air entrapment. Further, the structure helps to regulate the flow rate of the membrane to a low value resulting in an enhanced sensitivity. The CLN-040-SL53 has a pore size of 45 $\mu$m, a thickness of approximately 480 $\mu$m and a protein binding capacity of 103 $\mu$g/cm$^2$.
The absorbent pad (AP 120) used in the absorbent layer has a special property and is partial swelling on wetting in order to ensure good contact with membrane. This pad was bought from the same company mdi and has a thickness of approximately 1550 $\mu$m.

*2.2. Selection of the labeled system*

The method was optimized such that an easy visual interpretation of results, meaning a clear colored signal for the positive samples but also a very low signal for the negative samples, i.e. low background. Also, the visual discrimination between low-positive results observed in case of viral and mild-bacterial infections, i.e. CRP concentration up to 50 mg/L and strong positive results observed during severe bacterial infections, i.e. from +/- 60 up to 200 mg/L had to be clear. A reproducible test system, i.e. consistent results within different batches of membranes was obtained. Indeed, the determination of CRP concentration is useful in order to evaluate the follow-up of the treatment, i.e. antibiotics. An assay device is of low cost.

The end formed visual signal on membrane based tests can be formed by colored latex particles, gold particles, dyes or enzymes. The use of latex particles may lead to problems of interpretation and does not allow a permanent result. Self-agglutination also may occur, affecting the specificity of the method. Gold particles of good quality are relatively expensive. Some problems of reproducibility manufacturing in terms of spherical shape, size also may be encountered. Literature showed that the size of gold particles was a critical factor.

The present inventors decided to use a conjugate, i.e. purified anti-human CRP antibodies from rabbit origin coupled to Horse Radish Peroxidase -HRP- and a TMB precipitating system. Contrary to the use of gold particles, the presence of CRP is thus revealed by a one-step enzymatic reaction which is stopped by the use of a fixative, i.e. stopping solution.

The use of a fixative solution allows a permanent record of the result. This is not the case with gold particles for which the reading may be performed within 5 minutes and for which the result cannot be retained. The enzymatic reaction consists of the reaction of 3-3',5,5' tetramethylbenzidine with horse radish peroxidase and hydrogen peroxide forming a blue free radical cation 1-electron oxidation product.

Advantages of such combination include commercial ready to use TMB preparations of high consistent quality and with a long shelf life are available on the market. Manufacture of such reagent is easy and reproducible. The use of such precipitating system allows the development of a blue, i.e. aquamarine coloration easy to read. The conjugate used In this experimental setup can be titrated so that the best dilution of this compound can be selected allowing a correct, reproducible and accurate reading. In combination with a reflectance reader this assay device allows an accurate standardization of each batch of membrane, which guarantees a good reproducibility.

*2.3 Optimization of components*

*Membrane*

The membrane is the most critical factor in order to get a good test result. Even if exact mechanisms of binding are still unknown, e.g. hydrophobic interactions, hydrogen bonding, electrostatic interactions, high binding level is obtained through optimized application procedure and application (coating) buffer. Nitrocellulose is preferably chosen due to its neutral charge.

In the coating procedure membranes were cut into strips of 0.8 cm wide as it fitted to the flow-through device and slowly immersed into a bath brought at RT and containing the application buffer and the analyte-binding compound. It can be remarked that if the strips are immersed into a cold application buffer, e.g. 4°C strips tend to "retract" and the binding efficiency is decreased.

The coating (application) buffer features very low salinity and basic pH (9.1 +/-0.1) character. The first-analyte binding compound was applied in excess using a polyclonal antiserum anti-CRP with high affinity. An antibody stock having a titer concentration of 10.0 mg/ml +/-1.0 was diluted 1/50 in the application solution resulting in a binding concentration of 0.2 mg/ml.

| Composition of application buffer for 1 liter | |
| --- | --- |
| 1.2 g Tris | ICN 103133 |
| 8.8 g Nacl | Merck Eurolab (ME)1723 |

Several experiments showed that the reactive zone (cellulose nitrate) may be in direct contact with the application solution. Immersion gave the best results. A spotting coating procedure method gave less good results due to the formation of inhomogeneous and diffuse spots.

Several incubation, i.e. immersion times have been tested from 1 hour to 5 hours. An incubation of 3 hours at RT (18-22°C) under gentle agitation (rocking agitator) gave the best results. After the application, membranes are not washed off in order to avoid "desorption" of the analyte-binding compound due to the presence of surfactant agents in the washing buffer. Indeed, presence of a detergent such as Tween 20 which is usually present in the wash buffer might cause this effect.

*2.4 Post-coating - blocking -procedure:*

Such procedure was introduced in the concept and optimized in order to get a low background but also in order to avoid a washing step usually necessary during the FT (flow through) method. The aim was to block any free remaining sites on the membrane. The post-coating or blocking procedure is widely used in the ELISA methods, i.e. using soluble substrates in order to block the remaining free sites after the coating procedure.

The strips used in the dot method are generally, but not always, blocked in order to avoid a too high background induced by the non-specific adsorption of the conjugate onto the strip. In FT methods washings are obligatory in order to eliminate the unbound gold particles conjugate, i.e. gold antibody conjugate which may be adsorbed onto the membranes.

In the method according to present invention, the remaining free sites after the coating procedure are blocked using very purified BSA (bovine serum albumin) at a concentration of 1% in order to prevent any non-specific adsorption of the conjugate during the flow-through. Nevertheless, other proteins or polymer components such polyvinylic alcohol, polyvinylic pyrrolidone may be used for the same purpose.

After the coating procedure, strips are thus immediately immersed into a second bath containing the blocking agent. Strips are placed under gentle agitation (rocking agitator) during 3 hours at RT (18-24°C).

Composition of the blocking buffer (for 1 liter)

| | | |
|---|---|---|
| 8.5 g Nacl | MERCK EUROLAB | 1723 |
| 1.25 g Na$_2$HPO$_4$ | " | 1770 |
| 0.160 g NaH$_2$PO$_4$.2H2O | " | 1769 |
| 1 g thimerosal | " | 818957 |
| 10 g BSA | ICN | 105133 |

Due to the high binding of the analyte-binding compound onto the membrane this compound was not "displaced" by the BSA which blocks the remaining free sites. Strips were immersed into the blocking solution during 3 hours under gentle agitation. Thimerosal was added as a preservative.

Also after the blocking procedure, membranes were not washed off in order to avoid "desorption" of the analyte-binding compound and the blocking agents due to the presence of surfactant agents. Indeed, presence of a detergent such as Tween 20 which is usually present in the wash buffer might cause this effect.

Strips were dried at 37°C during 1 hour and then placed overnight in an Incubator at 18-22°C in order to avoid dust. The drying step may also be performed overnight at 37° without having any affect on the final result. Strips were then packaged in order to protect them against humidity under vacuum and stored at RT.

The handling procedure described above gave the best results with the flow through procedure described hereafter (see section 2.5).

The analyte-binding compound is used in excess; the application buffer supplemented with the analyte-binding compound may be reused 2 times if frozen at -20°C between two handling steps.

Similarly, the blocking solution also may be reused 2 times within 1 month, if stored at 4°C, between 2 handling steps without affecting the efficiency of the blocking procedure.

*2.5. FT procedure*

The sample, i.e. serum or whole blood was diluted 1/50 into the diluent buffer. The diluent buffer used was a Tris buffer of low salinity and containing BSA, i.e. 3 or 5%. The dilution was performed whereby 10 μl of the blood sample was added to the test tube containing 500 μl of diluent buffer. This test tube as subsequently closed and mixed.

Preparation for 1 liter diluent buffer

| | | |
|---|---|---|
| 1.2 g Tris | ICN | 103133 |
| 8.8 g Nacl | ME | 1723 |
| 50 g BSA ref. | ICN | 105033 |
| 1 g thimerosal | ME | 818957 |

15 μl or a drop of diluted sample was applied onto the membrane and the sample was allowed to soak through the membrane for at least 1 minute. Usually, the absorption time is around 45", i.e. range 30" up to 1'30". Such time is slower than those observed with other FT method. It is known that the sensitivity of the test increase with decreasing flow rate. Indeed, too fast flow rates, i.e. a few seconds does affect the sensitivity of the method.

This was followed by the addition of 25 $\mu$l or one drop of conjugate, i.e. anti-CRP coupled to HRP, 25 $\mu$l or one drop of precipitating TMB, 25 $\mu$l or one drop of fixative solution. After each addition, the solution was allowed to soak through the membrane. The result was read after minimal 2 minutes. Indeed, it may be necessary to wait at least 2 minutes before stopping the enzymatic reaction. For a long-term storage of the result, it is advised to cover the coloured spot with a scotch band, i.e. type 3M.

The procedure with the mentioned volumes 15-25-25-25 $\mu$l induce a homogenous colored spot of 3-4 mm of diameter. If a bigger diameter must be obtained > 4 mm or if volumes are higher, e.g. droplet dispenser of 50$\mu$l, the composition of diluent buffer has to be modified by the addition of sucrose, i.e. 10% up to 40% which slow the flow rate and let avoid heterogeneous coloration.

| Composition of the fixative solution (for 100 ml) | | |
|---|---|---|
| 1 g dioctyl-sulfosuccinate | SIGMA | D 0887 |
| 5 ml dimethylformamide | " | D 8654 |
| 19 ml polyvinylic alcohol 5% | " | P8136 |
| 76 ml H2O (filtrated) | | |

*2.6. Interpretation of result*

*2.6.1 visual reading = semi-quantitative reading*

The CRP concentration was estimated by comparing the test response with a standard curve prepared with highly purified CRP. The six zones of the chart correspond to the following concentrations: 11 mg/L, 27.5mg/L, 69mg/L, 91 mg/L, 137 mg/L and 275mg/L. Alternatively, other concentrations covering the range from 1 mg/L to 500 mg/L may be used.

Samples were interpreted as follows:

| Color of test response | CRP concentration |
|---|---|
| - lighter than the 11 mg/L zone | < 11 mg/L |
| - identical to a zone | in agreement with the zone |
| - between 2 zones | estimate a value between the zones |
| - darker than the 275 mg/L zone | > 275 mg/L |

In the kit, a colour chart will be included allowing the comparison of the coloration of the sample with the coloration of known concentrations of CRP. Alternatively, the colored spots might be scanned using a reflectance reader as discussed below.

*2.6.2 Interpretation with use of a reader*

Using a reflectance reader measurement of the intensity of the coloration is more accurate and allows to determine the concentration in CRP. The concentration of an unknown sample can be calculated by interpolation on a standard curve obtained with several concentrations of purified CRP.

**Example 3: Comparison with other existing method.**

*3.1. Aim of the study*

The optimized FT method was compared with 2 commercially available methods.

*Experimental conditions*

The inventors analysed 50 serum samples ranging from very low to high concentration in CRP which have been tested by the nephelometry method (quantitative method) using the Bio ART's flow through method, i.e. semi-quantitative method applying precipitating TMB and using the Nycomed method, I.e. semi-quantitative FT method applying gold particles.

*3.2. Procedure*

Samples have been tested according to the following procedure:

a) FT BIO-ART (according to present invention):

1. Using a pipette, 15µl of diluted sample (1/50) was applied onto the coated membrane and allowed to soak for at least 45 seconds.
2. Using a pipet (see table 4a) or a droplet dispenser (see table 4b) 25 µl of conjugate, i.e. anti-human CRP from a rabbit origin HRP labeled with horse radish peroxidase (HRP) with a concentration of 0.9 mg/L was applied to the coated membrane and allowed to soak.
3. Using a pipette (see table 4a) or a droplet dispenser (see table 4b) 25 µl of chromogen (precipitating TMB) was applied to the coated membrane and allowed to soak.
4. Using a pipette (see table 4a) or a droplet dispenser (see table 4b), 25 µl of fixative solution was applied to the coated membrane and allowed to soak.
5. Visual reading and/or measurement of the reflectance was performed after 2 minutes of stabilization.

b) FT Nycomed:

The kit from Nycomed has been used according the recommended procedure described in the package insert. Kit with lot n°10092464 was used.

1. Using a pipette, 25µl of diluted sample sample (1/40) or positive control was applied to the coated membrane. The sample was allowed to soak for at least 45 seconds.
2. One drop of conjugate was applied to the coated membrane and allowed to soak.
3. One drop of washing solution was applied to the coated membrane and allowed to soak
4. Reading, i.e. visual or by the use of the reflectance reader was performed after 30 seconds of stabilisation.

In order to validate the reflectance reader, both BIO ART and Nycomed results were read with the reflectance reader. Using a standard curve, the concentration of the sample was calculated in order to foresee the feasibility of a real quantitative method.

*3.3. Reagents:*

membrane lot n° : 00 6 4 (table 4a) and 00 1 15 (table 4b)
precipitating TMB lot n° : 5 DT 0629 Exp. Date: 06/01
fixative solution lot n°: 0051 Exp. Date: 05/01
conjugate: anti-CRP -HRP labeled diluted 1/700 (0.9mg/L)

*3.4. Interpretation of results*

*3.4.1 Nycomed : visual reading = semi-quantitative reading*

The CRP concentration was estimated by comparing the test response with the reference color chart provided with the kit. The five zones of the chart corresponded to the following concentrations: 10 mg/L, 25mg/L, 50mg/L, 100mg/L and 200mg/L.
Signals were interpreted as follows:

| Color of test response | CRP concentration |
|---|---|
| - lighter than the 10mg/L zone | < 10 mg/L |
| - identical to a zone | in agreement with the zone |
| - between 2 zones | estimate a value between the zones |
| - darker than the 200 mg/L zone | > 200 mg/L |

*3.4.2. BIO ART: visual reading = semi-quantitative reading*

The CRP concentration present in the samples was estimated by comparing the test response with a standard curve prepared with highly purified CRP. The six zones of the chart representing the standard curve corresponded to the following concentrations: 11 mg/L, 27.5mg/L, 69mg/L, 91 mg/L,137 mg/L, 275mg/L. Other concentrations covering the range from 10 mg/L to 250 mg/L may be used.

Interpretation was guided using following criteria:

| Color of test response | CRP concentration |
|---|---|
| - lighter than the 11mg/L zone | < 11 mg/L |
| - identical to a zone | in agreement with the zone |
| - between 2 zones | estimate a value between the zones |
| - darker than the 275 mg/L zone | > 275 mg/L |

*3.4.3 Calculation of results with the reflectance reader*

The end-coloration was read by using the reflectance reader as described hereafter.

The CRP concentration was calculated from the standard curve. Results are expressed In mg/L as listed in tables 4a and 4b.

*3.5.Results :*

See tables 4a and 4b.

*3.6.Conclusions :*

From table 4a it may be concluded that there is no discrepancy in terms of negative versus positive result, 3 samples * (30, 34, 43) are classified into different classes and according to the lot membrane, 5 samples** (25/26/30/35/37) are differently classified. In order to always get the same signal for the same level of concentration, the conjugate may be titrated, e.g. this titration gives allows to determine the best final dilution that might be used in the respective experiment.

In table 4b the first results show that the quantification is feasible. As the concentration is calculated by interpolation of the standard curve, no accurate concentration can be calculated for values lower than 10 mg/L. Additional standards, i.e. with values of 2 and 5 mg/L are needed in order to calculate the concentration with accuracy. For samples varying between 10 to 50 mg/L, correlation between both methods is good. Sample n° 31 has to be tested again For samples varying between 51 to 150 mg/L, correlation is also good except that sample 39 has to be tested again. For the visual reading (see table 1a) the inventors noticed that both the Nycomed and method according to present invention lacks discrimination for samples having an analyte concentration higher than 150 mg/L. By adjustment of the conjugate's dilution, such problem can be overcome. By adjustment of the TMB concentration, such problem can be overcome.

Further to this preliminary study, reagents have been optimized and a second series of samples with concentrations ranging from 0,4 mg/L to 200 mg/L have been tested according the BIO ART FT procedure. Results were compared with Nycomed method and with nephelometry. A standard curve prepared with purified CRP was prepared in order to interpret the results. The six concentrations of the Bio ART standard curve were 0 mg/L, 1 mg/L, 10 mg/L, 25 mg/L, 75 mg/L, 200 mg/L.

a) FIT BIO-ART (according to present invention):

1. Using a pipette, 20µl of diluted sample (1/50) was applied onto the coated membrane and allowed to soak for at least 45 seconds.

2. Using a droplet dispenser (see table 4c) 25 µl of conjugate, i.e. anti-human CRP from a rabbit origin HRP labeled with horse radish peroxidase (HRP) with a concentration of 0.9 mg/L was applied to the coated membrane and allowed to soak.

3. Using droplet dispenser (see table 4c) 25 µl of chromogen (precipitating TMB) was applied to the coated membrane and allowed to soak.

4. Using a droplet dispenser (see table 4c), 25 µl of fixative solution was applied to the coated membrane and allowed to soak.

5. Measurement of the reflectance was performed after 2 minutes of stabilization.

The results are shown in table 4 c.

The addition of a standard 1 mg/L allows a better discrimination In low values, i.e. < 10 mg/L. For samples varying between 10 to 75 mg/L, correlation between both methods is good. Sample n° 31 was tested again and showed a conc. of 43,8 mg/L which is more consistent with the result from nephelometry. For samples varying between 75 to 150 mg/L, correlation is also good except sample 39 which still showed a result lower than nephelometry (67 mg/L). As for the Nycomed method, value higher than 200 mg/L cannot be discriminated easily.

**Example 4 : evaluation of the reproducibility of results by the use of a reflectance reader**

*4.1 Introduction*

The reader is an imaging reader allowing the quantification of the colored signal by reflectance. The reader has been adapted to be used to read the signals obtained by the flow through CRP method as described in present invention. This reader allows the measurement of a specific area. This area is not limited to a specific form such as a circle.

Advantages of such quantification are evaluation of the inter-batches reproducibility, titration of the conjugate in order to get the same signal for the same concentration, evaluation of the sensitivity, evaluation of the intra-assay reproducibility and standardization of the quality control procedure (quantitative "acceptance/reject" specifications).

*4.2.. Experiments conditions*

The FT procedure has been performed according procedure described in example 3 and 4. For each experiment the membrane and sample used are indicated. The dilution performed for the conjugate is additionally mentioned.

a) Membrane lot n° 0064 sample 48 conjugate 1/700

The fourth lot of membrane produced in June (lot 00 6 4) was tested using a conjugate with a concentration of 0.9mg/L.A serum sample n° 48 having a concentration of 258 mg/L CRP was diluted 1/50 into the Tris diluent buffer containing 5% BSA and tested 5 times on 5 separate pieces of membrane (Replicate 1 (R1) to Replicate 5 (R5)).The procedure previously described (experiment 3 and 4) was used and reading was performed 2 minutes after fixation. The reflectance was measured using a red (R), a green (G) and a blue (B) filters. Results were interpreted according the measurement using the red filter since the difference In intensities between the different spots between 0 and higher values is more pronounced using this filter. The 5 replicates gave the following results:R1: 60.65 reflectance units (RU); R2 59.47 RU;R3 61.30 RU;R4 59.68 RU andR5 63.24 RU. The mean of the signals was 60.8 RU showing astandard deviation of 1.5 RU. The coefficient of variation (CV) was 2.5%.

b) Membrane lot 0093

The third lot produced in June (00 6 3) was tested under conditions as previously described for lot n° 0064. Results for the 5 replicates by using the red filter were: R1 66.72 RU;R2 70.05 RU;R3 68.10 RU;R4 68.54 RU andR5 68.02 RU. The mean of the signals was 68.2 RU with a standard deviation of 1.2 RU. The coefficient of variation was 1.8%.

c) Membrane lot 0094 sample 48 conj. 1/700

The fourth lot produced in September (00 9 4) was tested under conditions described for lot n° 0064. Results for the 5 replicates by using the red filter were R1 73.67 RU;R2 71.14 RU;R3 73.74 RU;R4 78.42 RU andR5 74.35 RU. The mean of the signals was 74.3 RU with a standard Deviation of 2.6 RU. The coefficient of variation was 3.5%.

d) Membrane 0064 sample 30 conj. 1/700

The fourth lot produced in June (00 6 4) was tested under conditions described for lot n° 0064 but with an hemolysed sample (n°30) with a concentration of 52.7mg/L. A hemolysed sample contains remains of blood cells which might influence reading of the obtained signals. In this way, it was tested if contaminating compounds Influence the reading of the assay. Results for the 3 replicates by using the red filter were: R1 76.38 RU;R2 67.60 RU andR3 78.56 RU. The mean of the signals was 74.2 RU with a standard deviation of 5.9 RU. The coefficient of variation (CV) was 7.8%. The inventors concluded that a higher CV as obtained with hemolysed sample using this method.

e) Membrane 0093 sample n° 30 (hemolysed) conj. 1/700

The third lot produced in September (00 9 3) was tested under conditions described for lot n° 0064 but with an hemolysed sample (n°30) with a concentration of 52.7 mg/L. Results for the 3 replicates by using the red filter were: R1 83.30 RU;R2 82.34 RU and R3 81.73 RU. The mean of the signals was 82.4 RU with astandard deviation of 0.8 RU.The coefficient of variation was 0.9%. From these results the inventors concluded that not the method "as such" but the membrane used for this method influenced the variation.

f.) Membrane 0064 sample 6 conj.1/700

The fourth lot produced in June (00 6 4) has been tested under conditions described for lot n° 0064 but using sample (n°6) with a CRP concentration of 2.0 mg/L. Results for the 4 replicates by using the red filter were: R1 129.6 RU;R2 126.6 RU;R3 131.8 RU andR4 126.6 RU. The mean of the signals was128.7 RU with astandard deviation of 2.5 RU. The coefficient of variation for this experiment was 1.96%.

g) Membrane 0081 sample 6 conj.1/700

The first lot produced in August (00 6 1) was tested under conditions described for lot n° 0064 with sample (n°6) carrying a CRP concentration of 2.0 mg/L. In this experiment different volumes of reagents were tested and its influence of the detected signals analyzed.

g.1 Reagents volumes used: 15-25-25-25 $\mu$l

Signals that were obtained were: R1 122.7 RU and R2137.1 RU.

g.2 Reagents volumes used: 15-15-15-15 $\mu$l

Signals that were obtained were: R3141.5 RU andR4150.6 RU.
The inventors observed a small difference in the intensity of the coloration confirmed by the measurement of the reflectance.

h) testing of standard curve on different lot of membranes

*Experimental conditions*

A standard curve containing highly purified CRP was prepared in the Tris diluent buffer containing 3% BSA. The flow through procedure was performed according previous description (experiment 3 and 4) and the measurement of reflectance with the red filter was performed on several lots of membranes

*Aim of the experiment*:

The inventors observed a poor visual discrimination for membrane lots 00102 and 00107 and a good discrimination for lot 00103 and 00106. The coating procedure was different according lot n° lot number and manufacturing (coating process). Lots 00103 and 00106 were made on strips of 0.8 cm wide; Lots 00102 and 00107 were made on larger strips 8.5 cm wide. According the quality control procedure, the accurate measurement of the reflectance will allow to accept or reject the membranes.
Results are shown in table 5. Sizes of membrane strips used are indicated. The reflectance units are shown for each standard concentration used.
Due to the lack of discrimination in the color intensity, membranes lot n° 00102 and 00107 were rejected. Membranes 001030 and 00106 featured a good visual discrimination, confirmed by the measurement of reflectance and passed the quality control procedure.

i) testing of samples on different lots of membranes

*Experimental conditions*

Samples with various concentrations in CRP were tested according the FT procedure as previously described (experiment 3 and 4) in order to confirm which size of membrane strips are optimal to use to get the best color discrimination.

Following samples were used and diluted 1/50 into Tris diluent buffer with 3% BSA: sample n° 45 (208 mg/L); sample n° 41 (157 mg/L);sample n° 37 (94 mg/L);sample n° 29 (48 mg/L);sample n° 21 (20 mg/L) and sample n° 9 (15 mg/L)..

Results are presented in table 6. Sizes of the strips used are indicated; reflectance units for each concentration tested are mentioned for each tested membrane.

The inventors concluded that there exists no relationship between the size of the strips during the coating process and the quality of results.

*j) Evaluation of the intra-assay reproducibility additional data*

*Preliminary experimental conditions*

The FT was performed according the previous description (experiment 3 and 4). 3 samples carrying different concentrations in CRP have been tested in 4 replica. The reflectance was measured and the coefficient of variation calculated.

Following samples were used and diluted 1/50 into Tris diluent buffer supplemented with 3% BSA: sample n° 41 (157 mg/l);sample n° 29 (47.8 mg/L) and sample n° 15 (9 mg/L).

Results are presented in table 7a. Reflectance units are indicated for each sample used. The mean of the signals determined, the standard deviation and the coefficient of variation for each group of measurements are shown.

*Experimental conditions additional data*

Further to this, extensive tests have been performed in order to study the intra-assay reproducibility. The FT was performed according the previous description (experiment 3 and 4) on different lot numbers of membranes. Different samples with concentrations ranging from 1 to 368 mg/L and the standard curve (prepared with highly purified BSA) with standard from 1 to 200 mg/L have been diluted in Tris buffer supplemented with 3% BSA and tested in different replicates on different membranes. The reflectance was measured and the mean, standard deviation and CV's have been calculated. For each experiment the lot numbers of the reagents are indicated. Results are presented in tables 7b to 7g.

The intra-assay reproducibility is lower than 5% and can be as low as 1 %.

k) Evaluation of the inter-assays reproducibility

*Preliminary experimental conditions*

The FT procedure was performed according previous description (experiment 3 and 4). The standard curve was tested on different lot of membranes, i.e. strips at different dates in order to check the reproducibility of the method (accurate results) independently from the test conditions such as ambient temperature. Results are presented in table 8a.

*Experimental conditions additional data*

Further to this, extensive tests have been performed in order to study the inter-assay reproducibility. The FT was performed by different users according the previous description (experiment 3 and 4) on different lot numbers of membranes at different days. Different samples with concentrations ranging from 1 to 368 mg/L and the standard curve (prepared with highly purified BSA) with standard from 1 to 200 mg/L have been diluted in Tris buffer supplemented with 3% BSA and tested in different replicates on different membranes. The reflectance was measured and the mean, standard deviation and CV's have been calculated. For each experiment the lot numbers of the reagents are indicated. Results are presented in tables 8b to 8f. Cv's are lower than 10 % and can b as low as 1 %.

I) Evaluation of another precipitating TMB additional data

*Aim 1: Another source of precipitating TMB (provided by another company) was tested in order to overcome the lack of discrimination for high values in CRP as shown In table 4b.*

Two different solutions were compared. A TMB 'A' solution was bought from Seramun (15754 Dolgenbrodt, Germany), and compared to a TMB 'B' solution obtained from D-TEK (7000 Mons, Belgium).

*Experimental conditions*

A standard curve has been prepared In Tris diluent buffer and the FT procedure using droplet dispenser has been performed as previously described (experiment 3 and 4). Results are shown in table 9a. Reflectance units are indicated for each concentration used using the different TMB agents (TMB "A"and "B").

*Conclusion*

The results showed that TMB "B" induces a better discrimination compared to the TMB "A". Such finding confirmed the variation of quality according source.

*Aim 2: In order to guarantee a good reproducibility, a new lot of TMB has been compared with the previous lot in the same test condition on standard curve.*

*Experimental conditions*

2 lots number of TMB have been tested on a purified standard curve ranging from 5 to 100 mg/L and 2 samples on membrane lot 1051.

**The variation between 2 lots was calculated by the ratio :** $\dfrac{\text{reflectance reference lot}}{\text{reflectance of the new lot}}$

Results are shown in table 9b.

*Conclusion*

The new lot gives comparable results to the previous one (1 % variation between both lots).

*Aim 3: In order to guarantee a good reproducibility, a new lot of TMB has been compared with the previous lot in the same test condition on samples.*

The FT was performed according the previous description (experiment 3 and 4) on membrane lot 1065 (produced in June 2001). 2 lots number of TMB have been tested on 2 samples and the diluent buffer only (blank value) in triplicates with a different dispensing device for each of the replicates. The reflectance was measured and the mean, standard deviation and CV's have been calculated. Results are shown In table 9c.

*Conclusion*

The new lot gives comparable results to the previous one within the inter-assays variation.

m1). Preliminary determination of the lowest concentration which can be detected.

A standard curve ranging from 2.5 mg/L to 360mg/L was prepared in a Tris diluent buffer containing 3% BSA. The FT procedure was performed according to previous description (experiment 3 and 4).

*Experimental condition:*

Membrane 00 6 4, conjugate anti-CRP HRP labeled with a dilution of 1:700 was used resulting in a final concentration of 0.9 mg/L 2 commercial TMB were compared: TMB-A from D-TEK (Belgium) and TMB-B from Seramun (Germany). Results are shown in table 10 a. Reflectance units measured for each standard value (mg/L are indicated.

*Conclusion:*

On this lot, the lowest value, i.e. different from 0 which could be detected is 2.5 mg/ml. Values between 180 and 360 mg/L can be discriminated.

m2). Additional data related to the lowest concentration which can be detected.

*Experimental conditions*

A standard curve ranging from 0 mg/L (blank) to 10 mg/L was prepared in a diluent buffer containing 3% BSA. Each concentration has been tested in 4 replicates on different lot of membranes. The FT procedure was performed according to previous description. Mean, standard deviation and CV have been calculated A statistical analysis (mean +/- 1,5 standard deviation (SD)) determined the lowest value different from zero which can be detected. Results are shown in table 10 b and 10c.

*Conclusions*

The lowest value which can be detected with a reflectance reader is 1 mg/L.

*4.3 General conclusions*

By an accurate measurement of the colored spot, the reflectance reader allow the implementation of quality control procedure and requested specifications. The intra-assays coefficient of variation may be as low as 1% up to 7% which is totally acceptable for a rapid test. Using the same final dilution of conjugate, the inter-assays coefficient of variation is lower than 10% for the different lot of membranes. By titration of conjugate, lower coefficient of variation can be obtained. A poorly visual discrimination is always confirmed by the reflectance values got with the reader.

**Example 5. *Detection of anti-mitochondrial antibodies (type M2) by the FT method.***

*5. 1 Introduction*

M2 antigen, i.e. pyruvate dehydrogenase is a specific marker for the diagnostic of Primary Biliary Cirrrhosis (PBC). The detection of anti-M2 antibodies is a specific tool for the diagnostic of PBC from other hepatic diseases.

*5.2 Experiment A:*

Membranes type CLN from mdi (Advanced Microdevices) with a pore diameter of 0.45 $\mu$m have been coated with highly purified dehydrogenase complex (Sigma ref. P5194) and blocked according procedures described in headings 2.3 and 2.4. Human anti-M2 antibodies (IgG/IgM) are revealed by the use of anti-human IgG Fab'2 HRP labeled (Dako ref. P406) and anti-human IgM Fab'2 HRP labeled (Dako ref. P322), with concentrations ranging from 0.2 to 0.3 mg/L, followed by the addition of precipitating TMB. Samples have been diluted in 1/100 in a Tris diluent buffer containing 5% BSA and the FT performed as previously described (Experiment 3 and 4).

*Results*

A coating concentration of 0.115 mg/ml corresponding to an enzymatic activity of 0.54 units/ml gave good visual discrimination between negative, low positive (+) and positive result (++). Background (diluent buffer only) was acceptable.

*5.2 Experiment B*

Membranes type CLN from mdi (Adanced Microdevices with a pore diameter of 0.45$\mu$m have been coated with highly purified dehydrogenase complex at a concentration of 0,24 mg/ml (Sigma ref. P5194) and blocked according procedure described in headings 2.3 and 2.4.
Human anti-M2 antibodies (IgG/IgM) are revealed by the use of anti-human IgG Fab'2 HRP labeled (Dako ref. P406) and anti-human IgM Fab'2 HRP labeled (ref. Dako P322) with concentration ranging from 0.2 to 1.0 mg/L, followed by the addition of precipitating TMB. Samples have been diluted 1/50 (as this dilution gave the best discrimination between positive and negative results) in a Tris diluent buffer containing 3% BSA and the FT performed as previously described (experiment 3 and 4). Results have been Interpreted by comparison of the intensity of the coloration with the intensity of the cut-off (limit of positivity). Reflectance has also been measured. As there is no correlation between the severity of the disease and the level of anti-M2 antibodies, a "yes/no", i.e. presence of absence of anti-M antibodies is sufficient and no quantitation is needed.

Tested samples featured a positive typical cytoplasmic pattern with the immunofluorescent method. However such immunofluorescent method does not allow to differentiate anti-M2 from other types of anti-mitochondrial antibodies (anti-M4, M5, M9).

*Aim of the study*

a) Results of the FT were compared with the ELISA method for the detection of anti-M2 antibodies.
ELISA M2 method is an automated method for which the result is obtained within 2 h.
Inter-assays reproducibility also has been studied.

b) inter-assay reproducibility (inter and intra-assay) has been studied. Results are shown in table 11 a and 11 b
Interpretation of results:

ELISA : positive result if the Optical Density is > than the OD for the cut-off control.
FT : positive result if the RU is higher then the RU value for the cut-off control.

There is a good correlation (100%) with the ELISA method. No discrepancy in terms of positive versus negative results is observed. The inter-assays reproducibility is lower than 10%.

**Example 6. *Detection of rheumatoid factors by the FT method.***

*6.1 Introduction.*

Rheumatoid factors are human immunoglobulins, i.e. mainly belonging to the IgM isotype directed against human or animal immunoglobulins. Detection of rheumatoid factors is very useful in the diagnosis of Rheumatoid Arthritis.

*6.2 Experiment*

Membranes type CLN from mdi (Advanced Microdevices) with pore diameter of 0.45μm have been coated with purified human immunoglobulins (Sigma ref. 068H4858) and blocked according procedures described in headings 2.3 and 2.4. Rheumatoid factors type M were revealed by the use of anti-human IgM Fab'2-HRP labeled (Daka ref P322), with a concentration +/- 1.5 mg/L, followed by the addition of precipitating TMB. Samples have been diluted 1/100 In a Tris diluent buffer containing 5% BSA and the FT performed as previously described.

*6.3 Results*

With a coating concentration of 0.53 mg/ml, the visual discrimination between negative samples (<25 IU/ml) and positive result (+/- 200 IU/ml) is acceptable.

**Example 7: *Detection of Salmonella***

*7.1 Introduction*

Detection of possible bacterial food contamination is the aim of the Hazard Analysis Critical Control Point (HACCP) recommendations. *Salmonella* is one of the main bacteria responsible for food mass poisoning. Classical methods such as culture, selection by growth on specific media and biochemical identification are labour and time consuming. Moreover, different bacteria may share same biochemical properties which may induce a wrong identification. Specific antibodies directed against bacterial antigens have now made possible to develop methods for rapid detection of food-borne pathogens. The use of such rapid detection method could allow a faster identification and detection. The aim was to develop a FT for the detection of *Salmonella sp.*.

*7.2 Experimental conditions.*

Membrane type CLN from mdi (Advanced Microdevices) with a pore diameter of 0.45 μm have been coated with a monoclonal capture antibody purified by A chromatography, specific to *Salmonella typhimurium* (Biodesign ref.

C65636M) with a concentration of 0.1 mg/ml. The coating and post-coating procedures are those described in sections 2.3 and 2.4., except that the incubation time for the coating procedure was 5 hours under gentle agitation at RT (18-24°C) and 1 or 2 hour(s) for the post-coating procedure.

In the meantime, a reference *Salmonella typhimurium sp.* (American Type culture collection ref. ATCC 14028 provided by the Laboratory of Microbiology Of the University of Gent) was plated on specific medium (nutrient agar) and incubated in aerobic conditions during 24 hours at 37°c.

a) Testing procedure for identification

For such procedure, a larger test device has been used (test zone of 1 cm) with a larger number of filters -8- (adsorbent pads). 1 pure culture of *Salmonella typhimurium* was diluted in physiologic water. 200 $\mu$l of such suspension were transferred to the membrane and let soak. The presence of *Salmonella* was revealed by 200$\mu$l of a second antibody HRP labeled (rabbit anti-salmonella peroxidase conjugated polyvalent for Salmonella "O" & "H" antigens (conc. 4$\mu$g/ml Biodesign ref. B65704R) followed by the addition of 200 $\mu$l of precipitating TMB. Coloration was stopped by 200 $\mu$l of fixative solution. There was a clear blue spot indicating the presence of *Salmonella typhimurium*.

b) Possible quantification

In order to evaluate a possible quantification, a standard curve of *Salmonella tiphymurium* ranging from 5,3 $\times 10^7$ to 7,0$\times 10^1$ was prepared hypotonic solution and tested with the FT. The different dilutions were counted for quantification. For the performance of the FT procedure, a larger test device has been used, i.e. test zone of 1 cm with a larger number of filters -8-(adsorbent pads). 500 $\mu$l of each dilutions were transferred to the membrane and let soak. 2 procedures for sample dispensing were used being 2 X 250 $\mu$l of sample, i.e. meaning a first soaking of 250 $\mu$l followed by a second deposit of 250 $\mu$l of sample and a flush of 500 $\mu$l of sample at once.

The presence of *Salmonella* was revealed by 500$\mu$l of a second antibody HRP labeled (rabbit anti-salmonella peroxidase conjugated polyvalent for Salmonella "O" & "H" antigens (conc. 4$\mu$g/ml Biodesign ref. B65704R) followed by the addition of 200 $\mu$l of precipitating TMB.

Coloration was stopped by 500 $\mu$l of fixative solution. Reflectance was measured. Results are shown in table 12.

There is a decrease in the intensity of the blue coloration measured by reflectance units according bacterial concentration meaning that quantification is possible. A flush of 500 $\mu$l of sample at once induced a more colored spot.

**Example 8: *Use of nylon membrane for the detection of CRP protein.***

Nylon is a linear synthetic polyamide terminated with either primary amine (positively-charged) or carboxylate (negatively-charged) end groups. Because the nylon membranes possess different surface chemistries from nitrocellulose (which is neutral), the binding properties are different.

*Experimental conditions*

In order to study the binding capacities of nylon, membranes from the company CUNO have been coated according the procedure described in section 2.3, with 10mmol or 25mmol Tris buffer. Different blocking procedures have been tested, either with BSA (condition A), either with polymer solutions such as polyvinylic alcohol (max. 0.1%) or polyvinyl pyrolidone (max. 1%) combined with non fat dry milk proteins (condition B). Nylon type C from the company Cuno of 0.45 $\mu$ diameter with a lowest ratio of amine to carboxylate end groups resulting in low positive charge have been used. Samples have been tested according procedure described In examples 3 and 4.

*Results*

By the use of BSA as blocking agent (condition), a colored spot was observed for the positive samples but some significant background was observed for the negative sample. Discrimination between positive and negative results was poor. Membranes blocked with polymer solutions and with non-fat dry milk protein also gave poor results (speckled spot). The first soaking time was too fast and a diffusion phenomenom was observed.

As different binding processes are involved for the binding on nylon membrane, a new optimization of reagents may be performed if such membranes are used. Nevertheless, the general concept as presented in present invention applies.

**Example 9: Influence of the molarity in Tris and pH.**

The influence of the composition (molarity) and the pH of the applied diluent buffer was studied.
Composition of the diluent buffer varied according conditions A, B and C (see below).

*Experimental conditions and procedure*

Samples have been diluted 1/50 into the diluent A, B and C and have been further tested according example 3 and 4.

Composition of the diluent (for 1 l):
Condition A

| Lot 1111 A & 1112 : | 0.12 g Tris |
| --- | --- |
| | 0.88 g. NaCl $\Rightarrow$ pH 7.36 |
| | 30 g. BSA |

This buffer is adapted to 'whole blood sample' and allows a complete hemolysis of the red cells due to the low concentration of NaCl which induces hypotonic conditions leading to hemolysis. The pH is neutral.

Condition B

| Lot 1051 : | 1.2 g. Tris |
| --- | --- |
| | 8.8 g NaCl $\Rightarrow$ pH 9.6 |
| | 30 g. BSA |

This buffer is adapted for serum samples but does.not allow a complete hemolysis of red blood cells in case of whole blood is tested. The pH is basic.

Condition C

| Lot 1113: | 1.2 g Tris |
| --- | --- |
| | 0.88 g. Nacl $\Rightarrow$ pH 10.5 |
| | 30 g. BSA |

This buffer induces hemolysis of the red blood cells but with a basic pH. Results are shown in table 13.
There is no statistical difference in the results according buffer composition. However a basic pH increases the flow rate through the membrane. Composition C has been selected.

Reagents

| A | Membrane: 1062 | B | Membrane: 1062 | C | Membrane: |
| --- | --- | --- | --- | --- | --- |
| | Conjugate : 1083 | | Conjugate: 1051 | | Conjugate: 1083 |
| | Diluent : 1111 & 1112 | | Diluent : 1051 | | Diluent: 1113 |
| | TMB :1021 | | TMB 1021 | | TMB : 1021 |
| | Fixative: 1081 | | Fixative : 1081 | | Fixative: 1081 |

**Example 10: Comparison with another rapid method for the detection of CRP**

*Introduction*

The CRP FT kit from has been benchmarked against the rapid method for the detection of CRP by immunoturbitimetry from the company ORION. The end-user was the emergency unit of a hospital. Samples were coming from patients manifesting signs of viral or bacterial infections. In case of a significant concentration in CRP (> 50 mg/L) the patient was treated with antibiotics. The ORION method is a rapid quantitative method based upon immunoturbidimetry but which requires a reader for the interpretation or results. Such method does not allow visual interpretation of result.

*Aim of the study*

47 samples ranging from negative in CRP concentration to high positive have been tested according Orion kit instructions.

*Number of positive samples tested :* 17 (36% of the total number of samples)
*Number of negative samples tested :* 30 (64% of the total number of samples)

The FT method from BIO ART has been performed according experiment described in section 3.

*Interpretation of results*

Results have been interpreted quantitatively with Orion (measurement of the concentration) and semi-quantitatively with BIO ART (comparison of the blue coloration with a reference color chart with the following concentrations 10-25-75 and 200 mg/L). Results are shown in table 14a-14e. There is a good correlation for the negative samples (table 14a). The concordance is 90% (27/30 samples well correlate). 3 samples (12/15/3) are interpreted as low positive with BIO ART and negative with Orion. As no clinical data were available for these 3 samples, no definitive conclusion can be drawn regarding the possibility of either false positive results for BIO ART either false negative results for Orion. For the low positive samples (table 14b), there is a correlation of 92% between both methods (11/12 samples). One sample (31) features a conc. of 25-50 mg/L with BIO ART method and 25 mg/L with Orion. Such difference however, does not induces any misclassification or misdiagnose. The four positive samples (table 14c) are similarly classified either with BIO ART, either with Orion. Only one high positive sample (n°37 see table 14d) has been tested and has a concentration of 200 mg/L with BIO ART and 112 mg/L with Orion. The cut-off level was 8 mg/L for Orion and 10 mg/L for BIO ART meaning that samples with a concentration lower than 8 or 10 mg/L feature normal levels in CRP.

*Conclusion:*

*1. Correlation on the positive samples*

There is no false negative result with the BIO ART method (all the samples which are positive with Orion are found positive with the BIO ART method). Correlation on positive samples is thus 100%. Moreover, 16 samples on 17 (except n°31) are classified into the same range of concentration

*2. Correlation on negative samples see table 4a)*

3 samples (n°12/15/3) are slightly positive with BIO ART and negative with Orion. In absence of clinical data or additional third method, no conclusion can be drawn regarding the possibility of either false positive results (BIO ART method) either false negative result (Orion). Moreover, these 3 low positive results from BIO ART do not induce erroneous diagnosis.

*3. General conclusion*

Such study indicated that the visual interpretation of results with the BIO ART FT method is valid for the interpretation. The FT from BIO ART features the same technical performances than the other rapid method. However, further to this study, it has been decided to include an additional standard of 1 mg/L in order to improve the visual discrimination between 0 and 10 mg/L

References

***Auto-Immune diseases***

Lemoine F.M. Intérèts clinique de la recherche des auto-anticorps. Medical trends 6/ 1992.

Humbel RL. Centre hospitalier du Luxembourg. Cources related to auto-immunity. 1990-1994. Université de Liège.

Abuaf N., Johanet C. and Homberg JC. "Les autoanticorps anti-tissues non spécifiques d'organe". 2e journée d'immunopathologie de l'hôpital Rothschild OPTION BIO n°51 (supplement)

Sturgess, A. -Recently characterised autoantibodies and their clinical significance Aust NZ J Med 1992; 22 : 279-289.

Mc Neil HP and Krilis SA Antiphospholipid antibodies Aust Nz J Med 1991; 21 : 463-475.

de Rooij et al. Use of recombinant RNP peptides 70K and A in an ELISA for measurement of antibodies in mixed connective tissue disease : a longitudinal follow up of 18 patients. Ann Rheum Dis 1990; 49 : 391-395.

Whittigham S. et al. Serological diagnosis of primary Sjögren syndrome by means of recombinant La (SSB) as nuclear antigen. Lancet, 1987; 8549:1-3.

Mathews MB and Bernstein RM. Myositis autoantibody inhibits histidyl tRNA synthetase. A model for autoimmunity. Nature, 1983; 304: 177-179.

Le Roy et al. Scleroderma (systemic sclerosis); classification, subsets and pathogenesis. J Rheumatol. 1988; 15 : 202-205.

Tan et al. Anti-nuclear antibodies (ANA's) : diagnosistically specific immune markers and clues towards the understanding of systemic auto-immunity. Clin Immunol Immunopath 1988; 47 : 121-141.

Tan Eng. Antinuclear antibodies : diagnostic markers for auto-Immune diseases and probes for cell biology. Advances in Immunology vol 44; 93-151, 1989 Academic Press.

Hochberg MC et al. Systemic Lupus Erythematosus : a review of clinico-laboratory features and immunogenetic markers in 150 patients with emphasis on demographic subsets. Medicine 1985; 64 : 285-295.

***CRP protein***

Lindback S et al. The value of C-reactive protein as a marker of bacterial infection in patients with septicaemia, endocarditis and influenza. Scand J Infect Dis 1989 ; 21 : 543-9.

Morley J and Kushner I. Serum C-reactive protein levels in disease. Ann NY Acad Sci 1982. 389: 406-18.

Whicher J et al. Acute phase response of serum amyloid A protein and C-reactive protein to the common cold and influenza. J Clin Pathol 1985; 38 : 312-6.

Pepys M and Baltz M. Acute phase proteins with special reference to C-reactive protein and related proteins (pentaxins) and serum amyloid A protein. Adv Immunol 1983; 34 : 141-212.

Chambers RE et al. Acute phase protein in inflammatory disease. Lab 1988; 1 : 29-37.

Mc Cabe Re and Remington JS. C-reactive protein in patients which bacteremia. J Clin Microbiol 1984; 20 : 317-9.

Mackie et al. C-reactive protein for rapid diagnosis of infection in leukaemia. J Clin Pathol 1979; 32 : 1253-6.

Angerman N et al. C-reactive protein in the evaluation of antibiotic therapy for pelvic infections J Reprod Med 1980; 25 : 65-7.

Philips A and Andrews P. Rapid determination of C-reactive protein levels; semi-quantitative versus quantitative. J. Paediatrics 1987 ; 110 : 263-8.

Komorski et al. Quantitative measurement of C-reactive protein in acute otitis media. Clin Lab Observ 1987; 111 : 81-4.

Claus DR et al. Radioimmunoassay of human C-reactive protein and levels in normal sera. J lab Clin Med 1976;

87 : 120-8.

Grützeimer S and von Schenk H. Four immunochemical methods for measuring C-reactive protein in plasma compared. Clin Chem 1989; 35 : 461-3.

Wadsworth C and Wadsworth E. Efficacy of latex agglutination and quantification methods for determination of C-reactive protein in pediatric sera. Clin Chem Acta 1984; 138 : 309-18.

Collet-Cassart D et al. A quantitative C-reactive protein assay using latex agglutination in microtiter plates. J Immunol Methods 1989 ; 125:137-41.

Hulman G et al. An accurate, simple and rapid test for detecting elevated levels of C-reactive protein in serum by agglutination of fat emulsion. Clin Chim Acta 1986; 156 : 337-40.

Price CP et al. Development and validation of a particle enhanced turbidimetric assay for C-reactive protein. J Immunol Methods 1987; 99 : 205-11.

Highton J and Hessian P. A solid-phase enzyme immunoassy for C-reactive protein : clinical value and the effect of rheumatoid factor. J Immunol Methods 1984; 68 : 185-92.

Käpyaho K et all Rapid determination of C-reactive protein by enzyme immunoassay using two monoclonal antibodies. Scand J Clin Lab Invest 1989; 49 : 389-93.

Buitago MG et al. Clinical evaluation of a fluorescence polarization immunoassay for quantifying C-reactive protein (Tech Brief). Clin Chem 1988; 34 : 595-6.

Hjortdahl P et al. C-reactive protein : a new rapid assay for managing infectious disease in primary health care. Scand J Prim Health Care 1991; 8 : 20-6.

Urdal P et al. Rapid Immunometric measurement of C-reactive protein in whole blood. Clin Chem 1992; 38 : 580-4.

**M2**

P.A. Berg and R. Klein. Autoantibody patterns in Primary Biliary Cirrhosis. Auto-immune liver diseases. Krawitt E.L. and Wiesner editors. Raven Press. 1991. Pages 121-142.

Berg E.L and R. Klein. Antimitochondrial antibodies in primary Biliary Cirrhosis and other disorders: definition and clinical relevance. Dig. Dis. 1992.10: 857-907

**Rheumatoid factors**

Bampton et al. 1985. Measurement of Rheumatoid factors by an anzyme-linked immunosorbent assay and comparison with other methods. Annals of Rheumatic Diseases. 44: 13-19.

Stone R. et al. 1987. Clinical value of ELISA assays for IgM and IgG Rgeumatic factors. J. Clin. Pathol. 40: 107-111.

**Allergy**

Kuby J. Immunology (Chapter 16) Eds. W.H. Freeman and Company, 1992.

Mygind N. Essential Allergy. Blackwell Scientific Publications, 1986.

Dodet Allergy: une cascade de reactions. BIOFUTUR juillet/aout 1989.

David B., Allergenes et desensibilisation. Rev.Fr.Allergol. 1989, 26: 29-37.

O'Byrne P.M. Allergen-induced airway hyperresponsiveness. J.Allergy Clin Immunol. 1988, 81: 119-127.

Cockcroft DW et al. Allergen-induced increase in non-allergic bronchial reactivity. Clin Allergy. 1977. 7: 503-513.

Platts-Mills Tae et al. Bronchial hyper-reactivity and allergen exposure. Prog.Resp.Res. 1985. 19: 276-284.

Bellanti JA. Prevention of food-allergies. Ann of Allergy 1984. 53: 683-688.

**Food applications**

Leclercq, A. Validation of analyses methods in food microbiology. Proceedings of the sixth conference in food microbiology, University of Liège, 21 & 22 June, 2001.

Leclerc,V; Vincent-Race,C et al. Recherche et dénombrement de Listeria monocytogenes : critères microbiologiques et méthodes. Proceedings of the sixth conference in food microbiology, University of Liège, 21 & 22 June, 2001.

Danan,C; Cornu,M; et al. Travaux de l'Agence française de sécurité sanitaire des aliments sur les tests de croissance de Listeria monocytogenes dans les denrées alimentaires. Proceedings of the sixth conference in food microbiology, University of Liège, 21 & 22 June, 2001.

Coia, J. (1998) Clinical, microbiological and epidemiological aspects of Escherichia coli O157 infection. FEMS Immunology and Medical Microniology 20:1-9.

Kerr, M.; Fitzgerald, M. et al. Survival of Escherichia coli 0157:H7 in bottled natural mineral water J.Appl.Microbiol. 87;833-841.

Smith, P.; Illingworth,D.S. and Coates, D. Evaluation of the EiaFoss assay for the detection of the Escherichia coli 0157 in food, faeces and environmental samples. Conference proceedings on "methods for Verocytotoxigenic E. coli" organised by an EU concerted Action on VTEC (CT 98-3935) at Western General Hospital, Edinburgh, November 25-26 th, 1998 ISBN 1 84170103 3

Vernozy-Rozand, C. Detection of Escherichia coli O157:H7 and other verocytotoxin producing E.coli (VTEC) in food. J.Appl.Microb. 82;537-551

François, JY; Jacob, B; et al. Salmonella spp. detection In foods : validation study of the Transla ™ plate salmonella Gold.Proceedings of the sixth conference in food microbiology, University of Liège, 21 & 22 June, 2001.

Van Der Zee H, Huis In't Veld JHj. Rapid and alternative screening methods for microbiological analysis. J AOAC Int , 4, 934-940 ,1997.

Rombouts FM et al. Rapid detection of foodborne pathogens. Med Fac Landbouww. Univ gent 60/4a, 1771-1776 (1995).

Paffard Sm, Miles Rj et al. A rapid and sensitive enzyme linked immunofilter assay (ELIFA) for whole bacterial cells. Journal of Immunological methods 192, 133-136,1996.

Bernal Cs, Robinson G et al. Development of a sensitive and quantitative enzyme-linked immunofilter assay (ELIFA) for whole bacterial cells. Journal of microbiological methods 19,135-143,1994.

Andrews W.H. Evolution of methods for the detection of Salmonella in food. J AOAC Int 1,4-12, 1996.

Blackbum C. Rapid and alternative method for the detection of Salmonella in food.J App Bacteriol 75, 199-215, 1993.

Feldslne, PT; Falbo-Nelson, MT et al. Visual immunoprecipitate assay (VIP) for the detection of enterohemorrhagic Escherichia Coli (EHEC) 0157:H7 in selected foods : collaborative study. J AOAC Int, 80, 517-529, 1997.

Chapamn PA, Ellin M, Ashton R. A comparison of immunomagnetic separation and culture Reveal and VIP for the detection of E.coli 0157 in enrichment cultures of naturally-contaminated raw beef, lamb and mixed meat products.

Feldsine, PT;Lienau et al. Visual immunoprecipitate assay (VIP) for the detection for Listeria monocytogenes and related Listeria species in selected food. : collaborative study. J AOAC Int, 80, 517-529, 1997.

Brakstad OG, Maeland JA. Detection of staphylococcus aureus with biotinylated monoclonal antibodies directed against staphylococcal Tnase complexed to avidin-peroxidase in a rapid sandwich linked immunofiltration assay (sELIFA). J Med Microbiol 39,128-134,1993.

Dupont H, Therasse J et al. Detection of staphylococcal enterotoxin - B A comparative study of ELISA and ELIFA systems. Journal Of Immunological methods 128,287-291,1990.

**Environment**

Schenider E, Usleber E & Martlauber E. Rapid detection of Fumonisin B-1 in corn-based food by competitive direct dipstick enzyme-immunoassay, enzyme-linked immunofiltration assay with integrated negative control reaction. Journal of Agricultural and FoodChemistry,43,2548-2552,1995.

**DNA applications**

Aubert D, Lepan H et al. Rapid detection of toxoplasmic nucleic-acid by enzyme-linked immunofiltration- assay after membrane transfer. Electrophoresis, 16, 354-356,1995.
Aubert D, Toubas D et al. Accelerated detection of DNA on membranes by automated enzyme-linked immunofiltration assay. Analytical Biochemistry, 247,25-29,1997.

**Material for medical devices (adapted to biological material)**

Rubin II. Handbook of plastic materials and technology 1990 pages 575-589.

Schouten AE. and van der Vegt. Plastics 1987 pages 106-124. Delta Press bv. Overburg, The Netherlands.

Tables

**Table 1:** Main organ and non-organ specific antibodies present in autoimmune diseases.

| General classification | |
|---|---|
| **Organ specific antibodies** | **Non-organ specific antibodies** |
| Anti-parietal cells (eg. Biermer's disease) | Anti-smooth muscle (Eg. chronic active hepatitis CAH type 1) |
| Anti-thyroid microsomes (eg. Hashimoto's thyroiditis) Grave's thyrootoxicosis | Anti-Liver Kidney microsomes (eg. chronic active hepatitis CAH type 2) |
| Anti-thyroglobulins (eg. Hashimoto's thyroiditis) Grave's thyrootoxicosis | Anti-mitochondria (Eg. Primary Biliary Cirrhosis |
| Anti-Langerhans islets (eg. diabetes) | Anti-reticulin (eg. coeliac disease) |

(continued)

| General classification | |
|---|---|
| **Organ specific antibodies** | **Non-organ specific antibodies** |
| Anti-skin (eg pempihgoid) | Anti-endomysium (Eg. coeliac disease) |
| Anti-striated muscle (ex. Myastenia gravis) | Anti-nuclear (Eg. System autoimmune diseases: SLA, Sjögren syndrome, scleroderma, dermatomyositis, polymyositis ..) |

**Table 2:** Auto-immune diseases induced by non-organ specific autoantigens

| Diseases | Autoantigen |
|---|---|
| Systemic Lupus Erythematosus | ds-DNA, ss DNA, RNP, Sm, cardiolipin, (SSA)(SSB) |
| Drug induced lupus erythematosus | histones |
| Scleroderma | nucleolar, Scl 70 |
| Crest syndrome | centromere |
| Other rheumatic diseases | ss and/or ds DNA |
| Polymyositis | Jo1 |
| Polymyositis + scleroderma | PM-Scl |
| Rheumatoid arthritis | Immunoglobulins (IgG) |
| Mixed connective tissue diseases | RNP |
| Sjögren syndrome | SSA/SSB |

**Table 3:** Auto-immune diseases induced by organ specific autoantigens

| Disease | non organ specific autoantigens |
|---|---|
| Addison's disease | adrenal cell cytoplasm |
| Auto-immune haemolytic anemia | erythrocytes |
| Chronic active hepatitis | cell surface lipoproteins, smooth muscles, Nuclear laminins |
| Coeliacdisease | endomysium |
| Goodpasture's syndrome | basement membrane (glomerulair and lung) |
| Graves'thyrotoxicosis | TSH receptor |
| Hashimoto's thyroiditis | thyroglobulin |
| Idiopathic thrombocytopenic purpura | platelets |
| Juvenile onset diabetes | islet cell cytoplasm and surface |
| Late onset diabetes | insulin receptor |
| Lens induced uveitis | lens |
| Male infertility (some) | spermatozoa |
| Multiple sclerosis | brain |
| Myasthenia gravis | skeletal and heart muscle |
| Pemphigoid | basement membrane (skin) |
| Pernicious anemia | parietal cell, gastrin receptor, intrinsic factor |
| Primary biliary cirrhosis | mitochondria, pyruvate dehydrogenase |
| Primary myxoedema | thyroid |
| Sympathetic ophtalmia | uvea |
| Ulcerative colitis | colon lipopolysaccharide |
| Vasculitides | p ANCA |
| Wegener's granulomatosis | c aNCA |

**Table 4:** Evaluation of the flow-through method as described in the present invention and other existing methods of Nephelometry and Nycomed.

**Table 4a:** Semi-quantitative method

| Ranking | Ident. | Nephelometry | BIO ART FT | NYCOMED | Int. |
|---|---|---|---|---|---|
| | | [mg/L] | [mg/L] | [mg/L] | |
| 1 | 114467 | 0.4 | < 11 <br> <11* | <10 | **Neg.** |
| 2 | 114083 | 0.6 | < 11 <br> <11* | <10 | Neg. |
| 3 | 114196 | 1.2 | < 11 <br> <11* | <10 | Neg. |
| 4 | 114187 | 1.7 | < 11 <br> <11* | <10 | Neg. |
| 5 | 114169 | 1.9 | < 11 <br> <11* | <10 | Neg. |
| 6 | 114058 | 2.0 | < 11 <br> <11* | <10 | Neg. |
| 7 | 114188 | 2.1 | < 11 <br> <11* | <10 | Neg. |
| 8 | 114035 | 2.5 | < 11 <br> <11* | <10 | Neg. |
| 9 | 114125 | 2.5 | < 11 <br> <11* | <10 | Neg. |
| 10 | 114086 | 3.2 | < 11 <br> <11* | < 10 | Neg. |
| 11 | 114206 | 4.8 | < 11 <br> <11* | =10 | Neg./D. |
| 12 | 114201 | 5.3 | < 11 <br> <11* | < 10 | Neg. |
| 13 | 114161 | 6.2 | < 11 <br> <11* | = 10 | Neg./D. |
| 14 | 114197 | 8.9 | < 11 <br> <11* | slightly > 10 | Neg./D. |
| 15 | 114057 | 9.3 | +/-20 <br> +/- 15* | slightly > 10 | D./D. |
| **16** | 114011 | 10.7 | lightly > 11 <br> = 11* | darker >10 +/- 20 | **D./Pos.** |
| 17 | 114085 | 11.9 | lightly > 11 <br> < 11* | darker >10 +/- 20 | D./Pos. |
| **18** | 114118 | 13.0 | = 11 <br> = 11* | 25 | Pos. + |
| 19 | 114192 | 14.2 | > 11 but <27.5 <br> > 11 but <27.5* | 25 | Pos. + |

(continued)

| Ranking | Ident. | Nephelometry | BIO ART FT | NYCOMED | Int. |
|---|---|---|---|---|---|
| | | [mg/L] | [mg/L] | [mg/L] | |
| 20 | 114190 | 17.8 | > 11 but <27.5<br>> 11 but <27.5* | 25 | Pos. + |
| 21 | 114084 | 20.2 | +/- 20<br>[11-27.5]* | slightly > 25 +/- 30 | Pos. + |
| 22 | 114065 | 24.7 | 27.5<br>[11-27.5]* | 25-50 | Pos. + |
| 23 | 114015 | 28.2 | 27.5<br>27.5* | 25-50 | Pos. ++ |
| 24 | 114061 | 31.7 | 27.5\|<br>27.5* | 50 | Pos. ++ |
| 25** | 115066 | 34.9 | +/- 69 [50-100]<br>[27.5-69] * | 50 | Pos. ++ |
| 26** | 115165 | 38.5 | +/- 69 [50-100]<br>+/- 40* | 50 | Pos. ++ |
| 27 | 114066 | 43.0 | +/- 69 [50-100]<br>+/-50 | darker> 50 but < 100 | Pos. ++ |
| 28 | 115166 | 45.4 | +/- 69 [50-100]<br>+/-50 | darker> 50 but < 100 | Pos. ++ |
| 29 | 114081 | 47.7 | +/- 50<br>+/-50 | +/- 80 | Pos. ++ |
| 30 | 115019 | 52.7 | [27.5-69]<br>+/-50 | lightly < 100 +/- 90 | Pos.++(+) |
| 31 | 114039 | 54.7 | < 69 [50-100]<br>< 69* | +/- 90 | Pos.++(+) |
| 32 | 114177 | 60.5 | < 69 [50-100]<br>+/ - 69* | +/-100 | Pos.++(+) |
| 33 | 115083 | 61.9 | < 69 [50-100]<br>< 69* | 100 | Pos.++(+) |
| 34* | 114016 | 67.6 | +/- 27.5 /<br>+/- 27.5* | 100 | Pos.++(+) |
| 35** | 115026 | 75.1 | lightly > 91 [100-150]<br>lightly < 69 * | lightly > 100 | Pos.++(+) |
| 36 | 114068 | 84.0 | lightly > 91 [100-150]<br>= 91* | 100 | Pos.++(+) |
| 37** | 114179 | 94.0 | = 69<br>= 91* | lightly > 100 | Pos. +++ |
| 38 | 115079 | 109.0 | = 91<br>= 91* | > 100 | Pos. +++ |
| 39 | 115022 | 122.0 | = 91<br>= 91* | > 100 | Pos. +++ |
| 40 | 114073 | 133.0 | [91-137]<br>= 137* | darker > 100 +/-160 | Pos.+++ |

(continued)

| Ranking | Ident. | Nephelometry | BIO ART FT | NYCOMED | Int. |
|---|---|---|---|---|---|
| | | [mg/L] | [mg/L] | [mg/L] | |
| 41 | 114076 | 157.0 | [91-137] = 137* | 200 | Pos.++++ |
| 42 | 115034 | 166.0 | +/- 137 +/- 137* | 200 | Pos.++++ |
| 43* | 114185 | 181.0 | [91-137] [91-137]* | 200 | Pos.++++ |
| 44** | 115042 | 203.0 | = 91 (heterog.spot) = 137* | 200 | Pos.++++ |
| 45 | 114181 | 208.0 | > 137.5 > 137.5* | 200 | Pos.++++ |
| 46 | 114019 | 218.0 | slightly < 275 [138-275] | > 200 sat. | Pos.++++ |
| 47 | 115016 | 231.0 | +/- 150 +/-150* | > 200 sat. | Pos.++++ |
| 48 | 114087 | 258.0 | +/-150 +/-150* | > 200 sat. | Pos.++++ |
| 49 | 115064 | 312.0 | +/- 275 +/-275* | > 200 sat. | Pas.++++ |
| 50 | 115084 | 368.0 | +/- 275 +/-275* | > 200 sat. | Pos.++++ |

**Table 4b:** Feasibility study of a quantitative method

| 4.b.1. Standard curve | | |
|---|---|---|
| Standards mg/L | Reflectance Red filter BIO ART | Reflectance Red Filter Nycomed |
| 275 | 77.2 | 69.9 |
| 120 | 84.3 | 92.3 |
| 60 | 95.4 | 112.5 |
| 40 | 101.2 | 131.4 |
| 20 | 137.0 | 152.1 |
| 10 | 153.6 | 161.5 |

| 4.b.2. Samples | | | | | |
|---|---|---|---|---|---|
| Sample | Nephelometry mg/L announced conc. | Reflectance Red filter BIO ART | Conc. mg/L | Reflectance Red filter Nycomed | Conc. mg/L |
| 1 | 0.4 | 168.4 | <10 | 190.4 | < 10 |
| 2 | 0.6 | 169.1 | <10 | 192.4 | < 10 |
| 3 | 1.2 | 161.3 | <10 | np | |
| 4 | 1.7 | 171.9 | <10 | 190.5 | <10 |
| 5 | 1.9 | 171.3 | <10 | np | |

(continued)

| 4.b.2. Samples | | | | | |
|---|---|---|---|---|---|
| Sample | Nephelometry mg/L announced conc. | Reflectance Red filter BIO ART | Conc. mg/L | Reflectance Red filter Nycomed | Conc. mg/L |
| 6 | 2.0 | 175.4 | <10 | 189.1 | <10 |
| 7 | 2.1 | 158.3 | <10 | np | |
| 8 | 2.5 | 164.2 | <10 | 190.2 | <10 |
| 9 | 2.5 | 150.1 | < 5 | np | |
| 10 | 3.2 | 157.4 | <10 | 190.5 | <10 |
| 11 | 4.8 | 164.8 | <10 | np | |
| 12 | 5.3 | 162.0 | <10 | 186.7 | <10 |
| 13 | 6.2 | 173.8 | <10 | np | |
| 14 | 8.9 | 155.9 | < 5 | 174.0 | <10 |
| 15 | 9.3 | 153.8 | 9 | np | |
| 16 | 10.7 | 155.2 | < 5 | 170.1 | 10 |
| 17 | 11.9 | 151.2 | 11 | np | |
| 18 | 13.0 | 148.0 | 12 | 164.7 | 11 |
| 19 | 14.2 | 130.2 | 20 | np | |
| 20 | 17.8 | 140.7 | 15 | 154.3 | 16 |
| 21 | 20.2 | 132.9 | 19 | np | |
| 22 | 24.7 | 127.6 | 23 | 146.2 | 24 |
| 23 | 28.2 | 109.6 | 32 | np | |
| 24 | 31.7 | 123.6 | 24 | 140.5 | 28 |
| 25 | 34.9 | 110.6 | 33 | np | |
| 26 | 38.5 | 114.9 | 30 | 131.4 | 36 |
| 27 | 43.0 | 106.8 | 38 | np | |
| 28 | 45.4 | 99.7 | 40 | 121.9 | 52 |
| 29 | 47.7 | 94.7 | 50 | np | |
| 30 | 52.7 | 104.1 | 37 | 117.1 | 56 |
| 31 | 54.7 | 142.6 | 15? | 119.9 | 50 |
| 32 | 60.5 | 98.2 | 60 | 114.1 | 64 |
| 33 | 61.9 | 101.4 | 52 | 111.2 | 70 |
| 34 | 67.6 | 100.8 | 56 | 113.1 | 68 |
| 35 | 75.1 | 98.9 | 60 | 110.2 | 72 |
| 36 | 84.0 | 102.3 | 52 | 103.0 | 92 |
| 37 | 94.0 | 94.9 | 78 | 100.4 | 100 |
| 38 | 109.0 | 94.6 | 78 | 87.4 | 160 |
| 39 | 122.0 | 97.6 | 64? | 88.5 | 150 |
| 40 | 133.0 | 83.9 | 140 | 81.8 | 190 |
| 41 | 157.0 | 84.9 | 135 | 80.9 | 200 |

(continued)

| 4.b.2. Samples | | | | | |
|---|---|---|---|---|---|
| Sample | Nephelometry mg/L announced conc. | Reflectance Red filter BIO ART | Conc. mg/L | Reflectance Red filter Nycomed | Conc. mg/L |
| 42 | 166.0 | 85.5 | 120 | 83.8 | 175 |
| 43 | 181.0 | 84.4 | 136 | 75.0 | 240 |
| 44 | 203.0 | 101.9 | 52 ? | 74.0 | 260 |
| 45 | 208.0 | 83.1 | 150 ? | 72.6 | 250 |
| 46 | 218.0 | 101.6 | 52? | 68.8 | 300 |
| 47 | 231.0 | 99.1 | 54? | 68.9 | 300 |
| 48 | 258.0 | 106.4 | 58? | 71.6 | 280 |
| 49 | 312.0 | 91.3 | 91? | 70.9 | 280 |
| 50 | 368.0 | 88.6 | 100 ? | 60.5 | 400 |

## Table 4 C: Feasibility of a quantitative method

### BIO ART standard curve :

| standard | Ru's |
|----------|------|
| mg/L     |      |
| 0        | 189  |
| 1        | 183  |
| 10       | 151  |
| 25       | 132  |
| 75       | 108  |
| 200      | 74   |
|          |      |

*Curve :*

y = -0,8465x + 3,0706

$R^2 = 0,991$

### Nycomed standard curve :

| standard | Ru's |
|----------|------|
| mg/L     |      |
| 0        | 170  |
| 10       | 162  |
| 40       | 131  |
| 60       | 113  |
| 120      | 92   |
| 275      | 70   |
|          |      |

*Curve :*

y = -1,2818+ 5,4987

$R^2 = 0,995$

| sample | Nephelometry Ann.conc.mg/L | BIO ART RU's | BIO ART Conc.mg/L | NYCOMED RU's | NYCOMED Conc. mg/L |
|---|---|---|---|---|---|
| **0 – 5 mg/L** | | | | | |
| 1 | 0,4 | 176 | 2,6 | 190 | <10 |
| 2 | 0,6 | 176 | 2,6 | 192 | <10 |
| 3 | 1,2 | 178 | 2,1 | np | |
| 4 | 1,7 | 169 | 4,6 | 190 | <10 |
| 5 | 1,9 | 164 | 6,3 * | np | |
| 6 | 2,0 | 173 | 3,4 | 189 | <10 |
| 7 | 2,1 | 176 | 2,6 | np | |
| 8 | 2,5 | 180 | 1,6 | 190 | <10 |
| 9 | 2,5 | 166 | 5,6 * | np | |
| 10 | 3,2 | 170 | 4,3 | 191 | <10 |
| 11 | 4,8 | 156 | 9,5 * | np | |
| **5,1 -10 mg/L** | | | | | |
| 12 | 5,3 | 152 | 11,4 | 187 | <10 |
| 13 | 6,2 | 147 | 14,0 | np | |
| 14 | 8,9 | 156 | 9,5 | 174 | <10 |
| 15 | 9,3 | 158 | 8,6 | np | |
| **10,1- 25 mg/L** | | | | | |
| 16 | 10,7 | 148 | 13,4 | 170 | 10 |
| 17 | 11,9 | 141 | 17,7 | np | |
| 18 | 13,0 | 141 | 17,7 | 165 | 6,3 |
| 19 | 14,2 | 151 | 11,9 | np | |
| 20 | 17,8 | 133 | 23,6 | 154 | 17 |
| 21 | 20,2 | 133 | 23,6 | np | |
| 22 | 24,7 | 148 | 13,4 | 146 | 24,8 |
| **25,1 – 75 mg/L** | | | | | |
| 23 | 28,2 | 132 | 24,5 | np | |
| 24 | 31,7 | 127 | 29,1 | 140 | 31,0 |
| 25 | 34,9 | 127 | 29,1 | np | |
| 26 | 38,5 | 112 | 48,6 | 131 | 41,3 |

| 27 | 43,0 | 131 | 25,3 | np | |
|----|------|-----|------|-----|------|
| 28 | 45,4 | 123 | 33,4 | 122 | 53,3 |
| 29 | 47,7 | 124 | 32,3 | np | |
| 30 | 52,7 | 112 | 48,6 | 117 | 60,9 |
| 31 | 54,7 | 115 | 43,8 | np | |
| 32 | 60,5 | 117 | 40,9 | 120 | 56,2 |
| 33 | 61,9 | 112 | 48,6 | np | |
| 34 | 67,6 | 106 | 59,9 | 113 | 67,6 |
| **75,1 – 200 mg/L** | | | | | |
| 35 | 75,1 | 111 | 50,3 | 110 | 73,1 |
| 36 | 84 | 104 | 64,3 | 103 | 87,8 |
| 37 | 94 | 107 | 57,8 | 100 | 95,1 |
| 38 | 109 | 98 | 80,1 | 87 | 137,3 |
| 39 ** | 122 | 103 | 66,6 | 89 | 129,3 |
| 40 | 133 | 112 | 48,6 | 82 | 161,0 |
| 41 | 157 | 85 | 136,2 | 81 | 166,5 |
| 42 | 166 | 81 | 164,3 | 84 | 150,8 |
| 43 | 181 | 77 | 201,9 | 75 | 207,2 |

***Assay protocol***

| comparative study (_Table 4 c_) |
|---|
| lot 1062 |

| STD (x) | lnx | Interpol. | Ecart% | Courbe moyenne | Y Moy. | Probit | Logit | Extrapol. | Measure (y) |
|---|---|---|---|---|---|---|---|---|---|
| | | 0 | 0 | 0 | -38 | | | 0 | |
| 1 | 0,000 | 1,0 | -3,3 | 183 | 98 | 22,167 | 3,099 | 183 | 183 |
| 10 | 2,303 | 11,9 | 18,5 | 151 | 73 | 2,658 | 0,978 | 155 | 151 |
| 25 | 3,219 | 24,6 | -2,1 | 132 | 59 | 1,439 | 0,384 | 131 | 132 |
| 75 | 4,317 | 55,8 | -25,6 | 108 | 42 | 0,716 | -0,334 | 100 | 108 |
| 200 | 5,298 | 239,4 | 19,7 | 74 | 17 | 0,209 | -1,567 | 77 | 74 |

| Meas. tot | 189 |
|---|---|
| NS | 60 |
| 50% Int. | 37,8 |
| S | -0,847 |
| lnA | 3,07061 |
| r2 | 0,99101 |

| Smp | Y | Probit | Valeur |
|---|---|---|---|
| 37 | 107 | 0,695 | 67,8 |
| 38 | 98 | 0,527 | 80,1 |
| 39 | 103 | 0,616 | 68,6 |
| 40 | 87 | 0,363 | 124,6 |
| 41 | 85 | 0,337 | 136,2 |
| 42 | 81 | 0,287 | 164,3 |
| 43 | 77 | 0,241 | 201,9 |
| 0 | 0 | -0,265 | ######## |
| 0 | 0 | -0,265 | ######## |
| 0 | 0 | -0,265 | ######## |
| 0 | 0 | -0,265 | ######## |
| 0 | 0 | -0,265 | ######## |
| 0 | 0 | -0,265 | ######## |
| 0 | 0 | -0,265 | ######## |
| 0 | 0 | -0,265 | ######## |
| 0 | 0 | -0,265 | ######## |
| 0 | 0 | -0,265 | ######## |

**Assay protocol**

| | | | | | comparative study (table 4c) | | | | |
|---|---|---|---|---|---|---|---|---|---|
| | | | | | lot 1062 | | | | |

| STD (x) | lnx | Interpol. | Ecart% | Courbe moyenne | Y Moy. | Probit | Logit | Extrapol. | Measure (y) |
|---|---|---|---|---|---|---|---|---|---|
| | | 0 | 0 | 0 | -38 | | | 0 | |
| 1 | 0,000 | 1,0 | -3,3 | 183 | 98 | 22,167 | 3,099 | 183 | 183 |
| 10 | 2,303 | 11,9 | 18,5 | 151 | 73 | 2,658 | 0,978 | 155 | 151 |
| 25 | 3,219 | 24,5 | -2,1 | 132 | 59 | 1,439 | 0,384 | 131 | 132 |
| 75 | 4,317 | 55,8 | -25,6 | 108 | 42 | 0,716 | -0,334 | 100 | 108 |
| 200 | 5,298 | 239,4 | 19,7 | 74 | 17 | 0,209 | -1,567 | 77 | 74 |

| Measure | 189 |
|---|---|
| NS | 50 |
| 50% int. | 97,6 |
| S | -0,847 |
| lnA | 3,07081 |
| r2 | 0,99101 |

| Smp | Y | Probit | Valeur |
|---|---|---|---|
| 19 | 151 | 2,658 | 11,9 |
| 20 | 133 | 1,482 | 23,6 |
| 21 | 133 | 1,482 | 23,6 |
| 22 | 148 | 2,390 | 13,4 |
| 23 | 132 | 1,439 | 24,5 |
| 24 | 127 | 1,242 | 29,1 |
| 25 | 127 | 1,242 | 29,1 |
| 26 | 112 | 0,805 | 48,6 |
| 27 | 131 | 1,397 | 25,3 |
| 28 | 123 | 1,106 | 33,4 |
| 29 | 124 | 1,138 | 32,3 |
| 30 | 112 | 0,805 | 48,6 |
| 31 | 115 | 0,878 | 43,8 |
| 32 | 117 | 0,931 | 40,9 |
| 33 | 112 | 0,805 | 48,6 |
| 34 | 106 | 0,676 | 59,9 |
| 35 | 111 | 0,782 | 50,3 |
| 36 | 104 | 0,635 | 64,3 |

**_Assay protocol_**

comparative study (Table 4 c)
lot 1082

| STD (x) | lnx | Interpol. | Ecart% | Courbe moyenne | Y Moy. | Probit | Logit | Extrapol. | Measure (y) |
|---|---|---|---|---|---|---|---|---|---|
| | | 0 | 0 | 0 | -36 | | | 0 | |
| 1 | 0,000 | 1,0 | -3,3 | 183 | 96 | 22,167 | 3,099 | 183 | 183 |
| 10 | 2,303 | 11,9 | 18,5 | 151 | 73 | 2,658 | 0,978 | 155 | 151 |
| 25 | 3,219 | 24,5 | -2,1 | 132 | 59 | 1,439 | 0,364 | 131 | 132 |
| 75 | 4,317 | 55,8 | -25,6 | 108 | 42 | 0,716 | -0,334 | 100 | 108 |
| 200 | 5,298 | 239,4 | 19,7 | 74 | 17 | 0,209 | -1,567 | 77 | 74 |

| Measure | 189 |
|---|---|
| NS | 50 |
| 50% int. | 37,8 |
| S | -0,847 |
| lnA | 3,07061 |
| r2 | 0,99101 |

| Smp | Y | Probit | Valeur |
|---|---|---|---|
| 1 | 176 | 9,692 | 2,8 |
| 2 | 176 | 9,692 | 2,8 |
| 3 | 178 | 11,636 | 2,1 |
| 4 | 169 | 5,950 | 4,6 |
| 5 | 164 | 4,560 | 6,3 |
| 6 | 173 | 7,688 | 3,4 |
| 7 | 176 | 9,692 | 2,8 |
| 8 | 180 | 14,444 | 1,6 |
| 9 | 168 | 5,043 | 5,6 |
| 10 | 170 | 6,318 | 4,3 |
| 11 | 158 | 3,212 | 9,6 |
| 12 | 152 | 2,757 | 11,4 |
| 13 | 147 | 2,310 | 14,0 |
| 14 | 158 | 3,212 | 9,5 |
| 15 | 158 | 3,484 | 8,6 |
| 16 | 148 | 2,390 | 13,4 |
| 17 | 141 | 1,896 | 17,7 |
| 18 | 141 | 1,896 | 17,7 |

**Table 5:** Evaluation of different membrane lots.

| Standard concentration mg/L | Membrane lot 00102 | Membrane lot 00103 | Membrane lot 00106 | Membrane lot 00107 |
|---|---|---|---|---|
| *dimensions* | *8.5 cm X 17 cm* | *0.8 cm X 17 cm* | *0.8 cm X 17.5 cm* | *8.5 cm X 17 cm* |
| 275 | 65.2 | 67.5 | 62.9 | 68.6 |
| 137.5 | 65.4 | 75.8 | 76.3 | 76.6 |
| 91 | 93.7 | 78.7 | 88.1 | 90.9 |
| 69 | 88.6 | 84.8 | 90.6 | 86.9 |
| 27.5 | 122.4 | 113.5 | 116.5 | 120.7 |
| 11 | 119.9 | 136.7 | 144.0 | 113.6 |

**Table 6:** Testing of different lots of membranes

| Concentration mg/L | Membrane lot 0092 | Membrane Lot 0093 | Membrane Lot 0094 | Membrane Lot 00102 | Membrane Lot 00104 |
|---|---|---|---|---|---|
| *strips* | *8.5 cm X 17.5 cm* | *8.5 cm X 17.5 cm* | *8.5 cm X 17.5 cm* | *8.5 cm X 17.5 cm* | *8.5 cm X 17.5 cm* |
| 208 | 78.7 | 80.7 | 81.0 | 76.1 | 73.1 |
| 157 | 77.8 | 93.7 | 90.0 | 83.6 | 82.7 |
| 94 | 79.5 | 100.3 | 98.8 | 96.9 | 87.1 |
| 48 | 106.1 | 107.7 | 107.8 | 93.5 | 109.1 |
| 20 | 126.8 | 131.5 | 138.2 | 104.4/121.7 | 132.6 |
| 15 | np | 146.7 | 147.6 | 117.7 | 137.3 |
| *QC* | QC rejected | QC passed | QC passed | QC rejected | QC passed |

**Table 7:** Evaluation of intra-assay reproducibility

**Table 7 a**

| CRP concentration mg/L | Reflectance lot 00106 | Reflectance lot 0064 | Reflectance lot 00111 | Reflectance lot 00112 |
|---|---|---|---|---|
| 157 | 96.9 | 80.7 | 92.2 | 72.6 |
| | 93.9 | 82.8 | 79.2 | 91.2 |
| | 100.9 | 81.5 | 81.7 | 84.1 |
| | 109.4 | 80.4 | 89.4 | 84.6 |
| *Mean* | *100.3* | *81.3* | *85.6* | *83.1* |
| *Standard deviation* | *6.7* | *1.2* | *6.15* | *7.7* |
| *Coefficient of variation* | *6.6 %* | *1.5 %* | *7.2 %* | *9.3 %* |
| | | | | |
| 47.8 | 108.5 | 103.6 | 93.3 | 101.5 |
| | 122.2 | 104.3 | 101.4 | 98.8 |
| | 131.4 | 101.1 | 106.8 | 100.6 |

(continued)

| CRP concentration mg/L | Reflectance lot 00106 | Reflectance lot 0064 | Reflectance lot 00111 | Reflectance lot 00112 |
|---|---|---|---|---|
| | 120.9 | 101.0 | 109.4 | 97.1 |
| *Mean* | *120.8* | *102.2* | *102.7* | *99.5* |
| *Standard deviation* | *9.4* | *2.0* | *7.1* | *2.0* |
| *Coefficient of variation* | *7.7 %* | *1.9 %* | *6.9 %* | *2.0 %* |
| | | | | |
| 9 | 168.2 | 153.2 | 133.6 | 144.9 |
| | 156.5 | 154.4 | 139.4 | 152.3 |
| | 171.0 | 155.1 | 139.1 | 140.8 |
| | 168.5 | 154.5 | 136.4 | 145.7 |
| *Mean* | *166.0* | *154.3* | *137.1* | *144.7* |
| *Standard deviation* | *6.5* | *0.8* | *2.7* | *5.4* |
| *Coefficient of variation* | *3.9 %* | *0.5 %* | *2.0 %* | *3.7 %* |

**Table 7:** Evaluation of intra-assay reproducibility

**Table 7 b**

| Reagents (lot n°) | | | |
|---|---|---|---|
| Membrane | 1062 | | |
| Conjugate | 1083 | | |
| Diluent buffer | 1111 A | | |
| TMB | 1021 | | |
| Fixative | 1081 | | |
| | | | |
| sample | RU's | sample | RU's |
| 03 (1mg/L) | 175 | 10 (3 mg/L) | 158 |
| | 166 | | 163 |
| | 167 | | 167 |
| | 175 | | 162 |
| Mean | 170 | Mean | 162 |
| SD | 4,9 | SD | 4 |
| %CV | 2,8 | %CV | 2,5 |
| 15 (9 mg/L) | 152 | 21 (20 mg/L) | 142 |
| | 155 | | 128 |
| | 158 | | 134 |
| | 165 | | 137 |
| Mean | 157 | Mean | 134 |

(continued)

| sample | RU's | sample | RU's |
|---|---|---|---|
| SD | 5,2 | SD | 5,5 |
| %CV | 3,3 | %CV | 4,1 |
| 29 (48 mg/L) | 123 | 36 (84 mg/L) | 101 |
| | 111 | | 103 |
| | 128 | | 102 |
| | 115 | | 101 |
| Mean | 118 | Mean | 101 |
| SD | 6,7 | SD | 0,9 |
| %CV | 5,7 | %CV | 1,0 |
| 38 (109 mg/L) | 100 | 50 (368 mg/L) | 80 |
| | 95 | | 79 |
| | 90 | | 81 |
| | 96 | | 77 |
| Mean | 94 | Mean | 79 |
| SD | 3,7 | SD | 2,1 |
| %CV | 3,9 | %CV | 2,7 |

**Table 7:** Evaluation of intra-assay reproducibility

**Table 7c**

| Reagents (lot n°) | | | |
|---|---|---|---|
| Membrane | 1063 | | |
| Conjugate | 1083 | | |
| Diluent buffer | 1081 | | |
| TMB | 1021 | | |
| Fixative | 1081 | | |
| | | | |
| sample | RU's | sample | RU's |
| 02 (0,6 mg/L) | 178 | 17(12 mg/L) | 134 |
| | 180 | | 147 |
| | 169 | | 146 |
| | 168 | | 144 |
| | 170 | | 152 |
| | 176 | | 146 |
| Mean | 174 | | 145 |
| SD | 5,2 | | 5,9 |
| %CV | 2,9 | | 4,1 |

(continued)

| sample | RU's | sample | RU's |
|---|---|---|---|
| 22 (24,7 mg/L) | 118 | 35 (75 mg/L) | 103 |
| | 122 | | 99 |
| | 120 | | 105 |
| | 113 | | 103 |
| | 125 | | 100 |
| | 126 | | 98 |
| Mean | 121 | Mean | 102 |
| SD | 4,6 | SD | 2,7 |
| %CV | 3,8 | %CV | 2,6 |
| 44 (203 mg/L) | 77 | | |
| | 81 | | |
| | 73 | | |
| | 72 | | |
| | 75 | | |
| | 70 | | |
| Mean | 75 | | |
| SD | 3,9 | | |
| %CV | 5,3 | | |

**Table 7:** Evaluation of intra-assay reproducibility

**Table 7d**

| Reagents | | | |
|---|---|---|---|
| Membrane | 1062 | | |
| Conjugate | 1083 | | |
| Diluent buffer | 1081 | | |
| TMB | 1021 | | |
| Fixative | 1081 | | |
| | | | |
| sample | RU's | sample | RU's |
| 02 (0,6 mg/L) | 178 | 17(12 mg/L) | 134 |
| | 180 | | 147 |
| | 169 | | 146 |
| | 168 | | 144 |
| | 170 | | 152 |
| | 176 | | 146 |
| Mean | 174 | Mean | 145 |

(continued)

| sample | RU's | sample | RU's |
|---|---|---|---|
| SD | 5,2 | SD | 5,9 |
| %CV | 2,9 | %CV | 4,1 |
| 22 (24,7 mg/L) | 118 | 35 (75 mg/L) | 103 |
| | 122 | | 99 |
| | 120 | | 105 |
| | 113 | | 103 |
| | 125 | | 100 |
| | 126 | | 98 |
| Mean | 121 | Mean | 102 |
| SD | 4,6 | SD | 2,7 |
| %CV | 3,8 | %CV | 2,6 |
| 44 (203 mg/L) | 77 | | |
| | 81 | | |
| | 73 | | |
| | 72 | | |
| | 75 | | |
| | 70 | | |
| Mean | 75 | | |
| SD | 3,9 | | |
| %CV | 5,3 | | |

**Table 7:** Evaluation of intra-assay reproducibility

**Table 7e**

| Reagents | | | |
|---|---|---|---|
| Membrane | 1061 b | | |
| Conjugate | 1083 | | |
| Diluent buffer | 1081 | | |
| TMB | 1021 | | |
| Fixative | 1081 | | |
| | | | |
| sample | RU's | sample | RU's |
| 02 (0,6 mg/L) | 169 | 17 (12 mg/L) | 127 |
| | 167 | | 132 |
| | 168 | | 140 |
| | 165 | | 139 |
| | 169 | | 127 |

(continued)

| sample | RU's | sample | RU's |
|---|---|---|---|
| | 170 | | 140 |
| Mean | 168 | | 134 |
| SD | 2,1 | | 6,8 |
| %CV | 1,2 | | 4,7 |
| 22 (32 mg/L) | 111 | 35 (75 mg/L) | 99 |
| | 110 | | 95 |
| | 112 | | 98 |
| | 113 | | 100 |
| | 112 | | 98 |
| | 111 | | 100 |
| Mean | 111 | Mean | 98 |
| SD | 0,8 | SD | 1,9 |
| %CV | 0,7 | %CV | 1,9 |
| 44 (203 mg/L) | 84 | | |
| | 80 | | |
| | 80 | | |
| | 85 | | |
| | 88 | | |
| | 81 | | |
| Mean | 83 | | |
| SD | 3,2 | | |
| %CV | 3,9 | | |

**Table 7:** Evaluation of intra-assay reproducibility

**Table 7f**

| Reagents | | | |
|---|---|---|---|
| Membrane | 1066 | | |
| Conjugate | 1083 | | |
| Diluent buffer | 1081 | | |
| TMB | 1021 | | |
| Fixative | 1081 | | |
| | | | |
| sample | RU's | sample | RU's |
| 02 (0,6 mg/L) | 176 | 17(12 mg/L) | 139 |
| | 166 | | 132 |
| | 164 | | 138 |

(continued)

| sample | RU's | sample | RU's |
|---|---|---|---|
| | 163 | | 130 |
| Mean | 167 | Mean | 135 |
| SD | 6,0 | SD | 4,4 |
| %CV | 3,6 | %CV | 3,3 |
| 21 (20mg/L) | 119 | 36 (84 mg/L) | 93 |
| | 119 | | 101 |
| | 114 | | 96 |
| | 114 | | 96 |
| Mean | 116 | Mean | 96 |
| SD | 2,9 | SD | 3,3 |
| %CV | 2,5 | %CV | 3,4 |
| 50 (368 mg/L) | 75 | | |
| | 77 | | |
| | 83 | | |
| | 86 | | |
| Mean | 80 | | |
| SD | 5,1 | | |
| %CV | 6,3 | | |

**Table 7:** Evaluation of intra-assay reproducibility

**Table 7g**

| Reagents | | | |
|---|---|---|---|
| Membrane | 1071 | | |
| Conjugate | 1083 | | |
| Diluent buffer | 1081 | | |
| TMB | 1021 | | |
| Fixative | 1081 | | |
| | | | |
| sample | RU's | sample | RU's |
| 02 (0,6 mg/L) | 171 | 17(12 mg/L) | 125 |
| | 174 | | 139 |
| | 178 | | 132 |
| | 167 | | 132 |
| Mean | 172 | Mean | 132 |
| SD | 4,7 | SD | 5,7 |
| %CV | 2,7 | %CV | 4,3 |

(continued)

| sample | RU's | sample | RU's |
|---|---|---|---|
| 21 (20 mg/L) | 111 | 36 (84 mg/L) | 85 |
| | 117 | | 100 |
| | 106 | | 79 |
| | 107 | | 89 |
| Mean | 110 | Mean | 88 |
| SD | 5,0 | SD | 8,8 |
| %CV | 4,5 | %CV | 10,0 |
| 50 (368 mg/L) | 67 | | |
| | 73 | | |
| | 63 | | |
| | 67 | | |
| Mean | 68 | | |
| SD | 4,1 | | |
| %CV | 6,1 | | |

**Table 7:** Evaluation of intra-assay reproducibility

**Table 7h**

| Reagents | | | |
|---|---|---|---|
| Membrane | 1062 | | |
| Conjugate | 1083 | | |
| Diluent buffer | 1113 | | |
| TMB | 1021 | | |
| Fixative | 1081 | | |
| | | | |
| sample | RU's | sample | RU's |
| 03 (1,2 mg/L) | 182 | 10 (3,2 mg/L) | 165 |
| | 182 | | 164 |
| | 177 | | 172 |
| | 178 | | 165 |
| Mean | 180 | | 167 |
| SD | 2,6 | | 3,7 |
| %CV | 1,5 | | 2,2 |
| 21 (20 mg/L) | 123 | 29 (48 mg/L) | 110 |
| | 136 | | 103 |
| | 143 | | 108 |
| | 130 | | 97 |

(continued)

| sample | RU's | sample | RU's |
|---|---|---|---|
| Mean | 132 | Mean | 104,5 |
| SD | 9,7 | SD | 5,8 |
| %CV | 7,4 | %CV | 5,5 |
| 36 (84 mg/L) | 89 | 38 ( 109 mg/L) | 89 |
| | 95 | | 93 |
| | 94 | | 81 |
| | 101 | | 82 |
| Mean | 95 | Mean | 86 |
| SD | 4,9 | SD | 5,7 |
| %CV | 5,2 | %CV | 6,7 |

**Table 8:** Evaluation of the inter-assay reproducibility

**Table 8a:**

| Std conc. mg/L | | | | | | | Mean | SD | CV % |
|---|---|---|---|---|---|---|---|---|---|
| Lot | 00106 | 00106 | 00111 | 00112 | 00114 | 00111 | | | |
| 275 | 62.9 | 58.4 | 68.7 | 68.2 | 75.4 | 70.1 | 67.3 | 5.9 | 8.7 |
| 137.5 | 76.3 | 75.7 | 73.0 | 78.2 | 82.6 | 77.8 | 77.3 | 3.2 | 4.1 |
| 91 | 88.1 | 78.8 | 85.6 | 89.2 | 84.6 | 80.0 | 84.4 | 4.2 | 5.0 |
| 69 | 90.6 | 89.6 | 99.5 | 95.1 | 93.6 | 86.6 | 92.5 | 4.6 | 4.9 |
| 27.5 | 116.5 | 102.4 | 115.3 | 108.6 | 112.9 | 102.4 | 109.7 | 6.2 | 5.7 |
| 11 | 114.1 | 123.2 | 137.1 | 130.9 | 134.9 | 122.9 | 127.2 | 8.7 | 6.8 |

**Table 8b:** Evaluation of the inter-assay reproducibility.

```
Reagents
·Membrane       1062
Conjugate       1083
Diluent buffer 1081 &1111A
TMB             1021
Fixative        1081
```

| sample | RU's | standard | RU's |
|---|---|---|---|
| 02 (0,6 mg/L) | 174 | 1mg/L | 173 |
| | 178 | | 172 |
| | 174 | | 172 |
| | 176 | | 182 |
| Mean | 176 | Mean | 175 |
| SD | 2 | SD | 4,9 |
| %CV | 1,1 | %CV | 2,8 |
| | | | |
| 17 (12 mg/L) | 137 | 10mg/L | 147 |
| | 150 | | 154 |
| | 147 | | 141 |
| | 141 | | 150 |
| Mean | 144 | Mean | 148 |
| SD | 5,8 | SD | 5,5 |
| %CV | 4,1 | %CV | 3,7 |
| | | | |
| 22 (24,7 mg/L) | 118 | 25 mg/L | 125 |
| | 113 | | 135 |
| | 120 | | 121 |
| | / | | 131 |
| Mean | 117 | Mean | 128 |
| SD | 3,6 | SD | 6,2 |
| %CV | 3,1 | %CV | 4,9 |
| | | | |
| 35 (75 mg/L) | 106 | 75 mg/L | 104 |
| | 104 | | 103 |
| | 101 | | 97 |
| | 111 | | 108 |
| Mean | 106 | Mean | 103 |
| SD | 4,2 | SD | 4,5 |
| %CV | 4,0 | %CV | 4,4 |
| | | | |
| 44 (203 mg/L) | 76 | 200 mg/L | 89 |
| | 78 | | 90 |
| | 74 | | 74 |
| | 85 | | 73 |
| Mean | 78 | Mean | 82 |
| SD | 4,8 | SD | 9,2 |
| %CV | 6,2 | %CV | 11,3 |
| | | blanc | 180 |

EP 1 344 059 B1

| | | | 181 |
|---|---|---|---|
| | | | 181 |
| | | | 181 |
| | | Mean | 181 |
| | | SD | 0,5 |
| | | %CV | 0,3 |

**_Assay protocol_**

| inter-assays table 8b |
|---|
| standard curve lot 1062 |

| STD (x) | lnx | Interpol. | Ecart% | Courbe moyenne | Y Moy. | Probit | Logit | Extrapol. | Measure (y) |
|---|---|---|---|---|---|---|---|---|---|
| | | 0 | 0 | 0 | -38 | | | 0 | |
| 1 | 0,000 | 0,9 | -7,9 | 175 | 95 | 20,833 | 3,037 | 175 | 175 |
| 10 | 2,303 | 11,1 | 10,6 | 148 | 75 | 2,970 | 1,088 | 150 | 148 |
| 25 | 3,219 | 27,1 | 8,3 | 128 | 60 | 1,472 | 0,388 | 130 | 128 |
| 75 | 4,317 | 72,6 | -3,2 | 103 | 40 | 0,679 | -0,388 | 102 | 103 |
| 200 | 5,298 | 187,3 | -6,4 | 82 | 24 | 0,323 | -1,129 | 81 | 82 |

| Max 50% | 181 |
|---|---|
| NS | 60 |
| 50% int. | 44,3 |
| S | -0,784 |
| lnA | 2,97213 |
| r2 | 0,99827 |

56

**Table 8c:** Evaluation of the inter-assay reproducibility

| Reagents |
|---|
| Membrane 1063 |
| Conjugate  1083 |
| Diluent buffer 1081 &1111A |
| TMB          1021 |
| Fixative 1081 |

| sample | RU's | standard | RU's |
|---|---|---|---|
| 02 (0,6 mg/L) | 169 | 1mg/L | 176 |
| | 170 | | 170 |
| | 173 | | 169 |
| | 173 | | 174 |
| Mean | 171 | Mean | 173 |
| SD | 2,1 | SD | 3,3 |
| %CV | 1,2 | %CV | 1,9 |
| | | | |
| 17 (12 mg/L) | 132 | 10mg/L | 141 |
| | 143 | | 135 |
| | 141 | | 153 |
| | Np | | 141 |
| Mean | 139 | Mean | 143 |
| SD | 5,9 | SD | 7,5 |
| %CV | 4,2 | %CV | 5,3 |
| | | | |
| 22 (24,7 mg/L) | 113 | 25 mg/L | 137 |
| | 122 | | 121 |
| | 122 | | 115 |
| | 118 | | 124 |
| Mean | 119 | Mean | 124 |
| SD | 4,3 | SD | 9,3 |
| %CV | 3,6 | %CV | 7,5 |
| | | | |
| 35 (75 mg/L) | 94 | 75 mg/L | 110 |
| | 111 | | 100 |
| | 101 | | 98 |
| | 91 | | 104 |
| Mean | 99 | Mean | 103 |
| SD | 8,9 | SD | 5,3 |
| %CV | 8,9 | %CV | 5,1 |
| | | | |
| 44 (203 mg/L) | 75 | 200 mg/L | 91 |
| | 96 | | 87 |
| | 83 | | 80 |
| | 82 | | 79 |
| Mean | 84 | Mean | 84 |
| SD | 8,8 | SD | 5,7 |
| %CV | 10,4 | %CV | 6,8 |
| | | | |
| | | blanc | 183 |
| | | | 179 |

**_Assay protocol_**

| inter-essays table 8c |
|---|
| standard curve lot 1083 |
| |
| |
| |
| |
| |
| |
| |
| |
| |

| STD (x) | lnx | Interpol. | Ecart% | Courbe moyenne | Y Moy. | Probit | Logit | Extrapol. | Measure (y) |
|---|---|---|---|---|---|---|---|---|---|
| | | 0 | 0 | 0 | -38 | | | 0 | |
| 1 | 0,000 | 0,9 | -9,9 | 173 | 93 | 13,667 | 2,615 | 172 | 173 |
| 10 | 2,303 | 11,3 | 13,1 | 143 | 70 | 2,385 | 0,869 | 145 | 143 |
| 25 | 3,219 | 28,0 | 12,0 | 124 | 56 | 1,276 | 0,244 | 127 | 124 |
| 76 | 4,317 | 71,1 | -5,2 | 103 | 40 | 0,671 | -0,399 | 102 | 103 |
| 200 | 5,298 | 184,8 | -7,6 | 84 | 28 | 0,347 | -1,059 | 83 | 84 |

| Mean 50% | 182 |
|---|---|
| NS | 50 |
| 50% Int. | 39,9 |
| S | -0,680 |
| lnA | 2,54298 |
| r2 | 0,99711 |

**Table 8 d:** Evaluation of the inter-assay reproducibility

Reagents
Membrane 1066
Conjugate : 1083 & 1081
Diluent buffer : 1081 &1111A
TMB : 1021
Fixative : 1081

| standard | RU's |
|---|---|
| 200 mg/L | 83 |
| | 76 |
| | 81 |
| | 95 |
| | 78 |
| | 74 |
| | 67 |
| Mean | 79 |
| SD | 8,1 |
| %CV | 10,2 |
| 75 mg/L | 104 |
| | 93 |
| | 92 |
| | 104 |
| | 95 |
| | 92 |
| | 87 |
| Mean | 95 |
| SD | 6,4 |
| %CV | 6,4 |
| 25 mg/L | 125 |
| | 119 |
| | 109 |
| | 126 |
| | 110 |
| | 110 |
| | 108 |
| Mean | 115 |
| SD | 7,9 |
| %CV | 6,8 |
| 10 mg/L | 130 |
| | 130 |
| | 127 |
| | 131 |
| | 135 |
| | 132 |
| | 148 |
| Mean | 133 |
| SD | 6,9 |

| %CV | 5,2 |
|---|---|
| 1 mg/L | 159 |
| | 160 |
| | 162 |
| | 168 |
| | 161 |
| | 167 |
| | 163 |
| Mean | 163 |
| SD | 3,4 |
| %CV | 2,1 |

**Assay protocol**

reproducibility *(Table 8 d)*
lot 1066

| STD (x) | lnx | Interpol. | Ecart% | Courbe moyenne | Y Moy. | Probit | Logit | Extrapol. | Measure (y) |
|---|---|---|---|---|---|---|---|---|---|
| | 0 | 0 | 0 | 0 | -41 | | | 0 | 0 |
| 1 | 0,000 | 0,9 | -10,8 | 163 | 93 | 12,556 | 2,530 | 162 | 163 |
| 10 | 2,303 | 11,4 | 14,2 | 133 | 68 | 2,128 | 0,755 | 135 | 133 |
| 25 | 3,219 | 28,0 | 11,9 | 115 | 53 | 1,140 | 0,131 | 117 | 115 |
| 75 | 4,317 | 73,1 | -2,8 | 95 | 37 | 0,584 | -0,537 | 94 | 95 |
| 200 | 5,288 | 180,1 | -9,9 | 79 | 24 | 0,312 | -1,165 | 77 | 79 |

| Meas. Y0 | 172 |
|---|---|
| NS | 50 |
| 50% int. | 33,8 |
| S | -0,696 |
| lnA | 2,45064 |
| r2 | 0,9967 |

**Table 8 e:** Evaluation of the inter-assay reproducibility

| Reagents |
|---|
| Membrane 1066 |
| Conjugate : 1083 & 1081 |
| Diluent buffer: 1081 &1111A |

(continued)

| Reagents<br>TMB : 1021<br>Fixative : 1081 | |
| --- | --- |
| | |
| sample | RU's |
| 44 (203 mg/L | 76 |
| | 70 |
| | 72 |
| | 80 |
| | 78 |
| Mean | 75 |
| SD | 4,1 |
| %CV | 5,5 |
| 35 (75 mg/L) | 102 |
| | 90 |
| | 91 |
| | 101 |
| | 104 |
| Mean | 98 |
| SD | 6,6 |
| %CV | 6,7 |
| 22 (24,7mg/L) | 118 |
| | 115 |
| | 120 |
| | 129 |
| | 132 |
| Mean | 123 |
| SD | 7,3 |
| %CV | 6,0 |
| 17 (12 mg/L) | 133 |
| | 129 |
| | 133 |
| | 154 |
| | 157 |
| Mean | 141 |
| SD | 13,1 |
| %CV | 9,3 |
| 2 (0,6 mg/L) | 162 |

(continued)

| sample | RU's |
|--------|------|
|  | 162 |
|  | 169 |
|  | 171 |
|  | 166 |
| Mean | 166 |
| SD | 4,0 |
| %CV | 2,4 |

| sample | RU's |
|--------|------|

**Table 8 f:** Evaluation of the inter-assay reproducibility

```
Reagents
Membrane      1065
Conjugate     1083
Diluent buffer 1081 &1111A
TMB           1021
Fixative      1081
```

| sample | RU's | standard | RU's |
|---|---|---|---|
| 02 (0,6 mg/L) | 173 | 1mg/L | 164 |
| | 172 | | 164 |
| | 175 | | 171 |
| | 175 | | 177 |
| Mean | 174 | Mean | 169 |
| SD | 1,5 | SD | 6,3 |
| %CV | 0,9 | %CV | 3,7 |
| | | | |
| 17 (12 mg/L) | 155 | 10mg/L | 147 |
| | 145 | | 148 |
| | 149 | | 140 |
| | 136 | | 143 |
| Mean | 146 | Mean | 145 |
| SD | 8,0 | SD | 3,7 |
| %CV | 5,5 | %CV | 2,6 |
| | | | |
| 22 (24,7 mg/L) | 115 | 25 mg/L | 110 |
| | 119 | | 136 |
| | 121 | | 119 |
| | 124 | | 119 |
| Mean | 120 | Mean | 121 |
| SD | 3,8 | SD | 10,8 |
| %CV | 3,2 | %CV | 8,9 |
| | | | |
| 35 (75 mg/L) | 92 | 75 mg/L | 98 |
| | 84 | | 98 |
| | 97 | | 92 |
| | 88 | | 106 |
| Mean | 90 | Mean | 99 |
| SD | 5,6 | SD | 5,7 |
| %CV | 6,2 | %CV | 5,8 |
| | | | |
| 44 (203 mg/L) | 87 | 200 mg/L | 84 |
| | 75 | | 84 |
| | 80 | | 83 |
| | 81 | | 87 |
| Mean | 81 | Mean | 85 |
| SD | 4,9 | SD | 1,7 |
| %CV | 6,1 | %CV | 2,0 |
| | | | |

**Assay protocol**

| STD (x) | lnx | Interpol. | Ecart% | Courbe moyenne | Y Moy. | Probit | Logit | Extrapol. | Measure (y) |
|---|---|---|---|---|---|---|---|---|---|
| | | 0 | 0 | 0 | -38 | | | 0 | |
| 1 | 0,000 | 1,1 | 5,3 | 169 | 90 | 9,154 | 2,214 | 169 | 169 |
| 10 | 2,303 | 8,0 | -19,7 | 145 | 72 | 2,568 | 0,943 | 141 | 145 |
| 25 | 3,219 | 28,5 | 13,8 | 121 | 54 | 1,164 | 0,152 | 124 | 121 |
| 75 | 4,317 | 84,2 | 12,3 | 99 | 37 | 0,590 | -0,527 | 101 | 99 |
| 200 | 5,298 | 185,0 | -7,5 | 85 | 27 | 0,381 | -1,019 | 84 | 85 |

**Table 9:** Evaluation of different TMB agents

**Table 9a:** Evaluation of different TMB agents (aim 1):

| CRP Concentration mg/L | Reflectance with TMB "A" | Reflectance with TMB " B" |
|---|---|---|
| 275 | 77.2 | 59.0 |
| 137.5 | 84.3 | 67.5 |
| 91 | 95.4 | 73.9 |
| 69 | 101.2 | 89.0 |
| 27.5 | 136.9 | 105.2 |
| 11 | 153.6 | 123.8 |

**Table 9 b:** Evaluation of 2 different lots of TMB (aim 2).

| Reagents | | | |
|---|---|---|---|
| Membrane | 1051 | | |
| *Conjugate* | *1051* | | |
| Diluent buffer | 1051 | | |
| *Fixative* | *1021* | | |
| | | | |
| *Standard* | *TMB lot 207 (ref.)* | *TMB lot 629* | *% ref.* |
| mg/L | Ru's | Ru's | Ratio 207/629 |
| 100 | 91 | 87 | 95,6 |
| 50 | 103 | 118 | 114,5 |
| 10 | 144 | 141 | 97,9 |
| 5 | 148 | 149 | 100,6 |
| + ctrl Nycomed | 137 | 121 | 88,3 |
| + sample dil.1/200 | 114 | 127 | 111,4 |
| | | | Mean: 101,4 +1,04% |

**Table 9c:** Evaluation of 2 different lot of TMB on samples

| Reagents | | | |
|---|---|---|---|
| Membrane | 1065 | | |
| Conjugate | 1083 | | |
| Diluent buffer | 111A | | |
| Fixative | 1081 | | |
| | | | |
| Samples | TMB lot 629 (ref) | TMB lot 917 | % diff. |
| | RU's | RU's | |
| *29 (48 mg/L)* | 113 | 125 | |
| | 127 | 117 | |
| | 113 | 132 | |
| *mean* | 118 | 125 | 5,4 % |
| *std dev.* | 8,0 | 7,5 | |
| *CV%* | 6,9 | 6,0 | |
| | | | |
| *50 (368 mg/L)* | 58 | 58 | |
| | 76 | 62 | |
| | 70 | 70 | |
| *mean* | 69 | 63 | 9,5% |
| *std dev.* | 10 | 6,1 | |
| *CV%* | 14,6 | 9,6 | |
| | | | |
| *Blank (0 mg/L)* | 185 | 176 | |

(continued)

| Samples | TMB lot 629 (ref) | TMB lot 917 | % diff. |
|---------|-------------------|-------------|---------|
|  | 178 | 175 |  |
|  | 179 | 175 |  |
| *mean* | 181 | 175 | 3,4 % |
| *std dev.* | 3,8 | 0,6 |  |
| *CV%* | 2,1 | 0,3 |  |
|  |  | Mean | 6,1 % |

**Table 10:** Determination of the lowest detection limit.

**Table 10 a:**

| Reagents (lot n°) | |
|---|---|
| Membrane | 1061 b |
| Conjugate | 1083 |
| Diluent buffer | 1111 |
| TMB | 1021 |
| Fixative | 1081 |
|  |  |
| Standard concentration | RU's |
| 0 mg/l | 184 |
|  | 191 |
|  | 181 |
|  | 189 |
| mean | 186,25 |
| Std dev.(SD) | 4,57 |
| CV% | 2,45 |
| 1,5 SD | 6,85 |
| Mean +/- 1.5 SD | 179,4 - 193,1 |
| 2 SD | 9,4 |
| Mean +/- 2SD | 176,9 - 195,7 |
|  |  |
| 1 mg/L | 160 |
|  | 162 |
|  | 172 |
|  | 159 |
| mean | 163,25 |
| Std dev.(SD) | 5,97 |
| CV% | 3,65 |

(continued)

| Standard concentration | RU's |
|---|---|
| 1,5 SD | 8,94 |
| Mean +/- 1.5 SD | 154,3 - 172,2 |
| 2 SD | 11,9 |
| Mean +/- 2SD | 151,4 - 175,5 |
| 10 mg/L | 143 |
| | 138 |
| | 133 |
| | 144 |
| mean | 139,5 |
| Std dev.(SD) | 5,1 |
| C V% | 3,6 |
| 1,5 SD | 7,6 |
| Mean +/- 1.5 SD | 131,9 - 147,1 |
| 2 SD | 10,1 |
| Mean +/- 2SD | 129,4 - 149,6 |

**Table 10 b:** Determination of the lowest detection limit.

| Reagents (lot n°) | |
|---|---|
| Membrane | 1106 |
| Conjugate | 1083 |
| Diluent buffer | 1081 |
| TMB | 1021 |
| Fixative | 1081 |
| | |

| Standard concentration | RU's |
|---|---|
| 0 mg/l | 178 |
| | 183 |
| mean | 180,5 |
| Std dev.(SD) | 3,53 |
| CV% | 1,95 |
| 1,5 SD | 5,3 |
| Mean +/- 1.5 SD | 175,2 - 185,8 |
| 2 SD | 7,1 |
| Mean +/- 2SD | 173,4 - 187,6 |
| | |
| 1 mg/L | 169 |
| | 173 |
| mean | 171,0 |

(continued)

| Standard concentration | RU's |
|---|---|
| Std dev.(SD) | 2,82 |
| CV% | 1,6 |
| 1,5 SD | 4,2 |
| Mean +/- 1.5 SD | 166,8 - 175,2 |
| 2 SD | 5,6 |
| Mean +/- 2SD | 165,4 - 176,6 |
| | |
| 10 mg/L | 145 |
| | 136 |
| mean | 140,05 |
| Std dev.(SD) | 6,4 |
| C V% | 4,5 |
| 1,5 SD | 9,5 |
| Mean +/- 1.5 SD | 131,0 - 150,0 |
| 2 SD | 12,7 |
| Mean +/- 2SD | 127,8 - 153,2 |

**Table 10 c:** Determination of the lowest detection limit.

| Reagents (lot n°) | |
|---|---|
| Membrane | 1071 |
| Conjugate | 1083 |
| Diluent buffer | 1081 |
| TMB | 1021 |
| Fixative | 1081 |
| | |
| Standard concentration | RU's |
| 0 mg/l | 171 |
| | 177 |
| mean | 174 |
| Std dev.(SD) | 4,2 |
| CV% | 2,4 |
| 1,5 SD | 6,4 |
| Mean +/- 1.5 SD | 167,6 - 180,4 |
| 2 SD | 8,4 |
| Mean +/- 2SD | 165,6 - 182,4 |
| | |
| 1 mg/L | 169 |
| | 171 |

(continued)

| Standard concentration | RU's |
|---|---|
| mean | 170 |
| Std dev.(SD) | 1,4 |
| CV% | 0,8 |
| 1,5 SD | 2,1 |
| Mean +/-1.5 SD | 167,9 - 172,1 |
| 2 SD | 2,8 |
| Mean +/- 2SD | 167,2 - 172,8 |
| | |
| 10 mg/L | 143 |
| | 142 |
| mean | 142,5 |
| Std dev.(SD) | 0,7 |
| C V% | 0,5 |
| 1,5 SD | 1,1 |
| Mean +/-1.5 SD | 141,4 - 143,6 |
| 2 SD | 1,4 |
| Mean +/- 2SD | 141,1 - 143,9 |

**Table 11:** Detection of anti-mitochondrial type M2 antibodies

**Table11 a:** 1° Comparison with the EUSA method

| *Reagents* Membrane: 1082 Conjugate: 1051 Diluent buffer: 1081 Fixative :1021 | | | | |
|---|---|---|---|---|
| | | | | |
| Sample n° | FT (Ru's) | Int. | ELISA (OD 450 nm) | Int. |
| *positive* | | | | |
| 34590 | 135 | + | 1,836 | + |
| 4820 | 128 | + | 1,905 | + |
| 26416 | 90 | + | 1,567 | + |
| 18133 | 119 | + | 1,481 | + |
| 8413 | 121 | + | 1,800 | + |
| 5276 | 90 | + | 1,510 | + |
| | | | | |
| *negative* | | | | |

(continued)

| Sample n° | FT (Ru's) | Int. | ELISA (OD 450 nm) | Int. |
|---|---|---|---|---|
| 49319 | 161 | - | 0,206 | - |
| 49556 | 154 | - | 0,178 | - |
| 3494 | 158 | - | 0,064 | - |
| 2364 | 149 | - | 0,049 | - |
| 49435 | 164 | - | 0,036 | - |
| 40855 | 147 | - | 0,075 | - |
| 3494 | 161 | - | 0,049 | - |
| 2364 | 154 | - | 0,064 | - |
| 46235 | 156 | - | 0,040 | - |
| *Cut-off* | *143* | - | *<0,300* | |
| Blank value | 164 | | | |

**Table 11b:** Detection of anti-mitochondrial type M2 antibodies.

| Study of the inter-assays reproducibility | | | | |
|---|---|---|---|---|
| *Reagents*<br>Membrane: 1081 / 1082 / 1083/ 1085<br>Conjugate: 1051      Diluent buffer: 1081      Fixative :1021 | | | | |
| | | | | |
| Positive sample | RU's | RU's | RU's | RU's |
| *Lot membrane* | 1081 | 1082 | 1083 | 1085 |
| | | | | |
| 5276 | 90 | 80 | np | 80 |
| | | 89 | | |
| | | 90 | | |
| *Mean* | 86 | | | |
| *Std Dev.* | 5,3 | | | |
| *CV %* | 6,2 | | | |
| | | | | |
| 26416 | 89 | 106 | 95 | 82 |
| | 90 | | | |
| *Mean* | 92 | | | |
| *Std Dev.* | 8,9 | | | |
| *CV %* | 9,6 | | | |
| | | | | |
| 18133 | 119 | 104 | 109 | 98 |
| *Mean* | 107 | | | |
| *Std Dev.* | *7,7* | | | |

(continued)

| Positive sample | RU's | RU's | RU's | RU's |
|---|---|---|---|---|
| *CV %* | *7,2* | | | |
| | | | | |
| Negative samples | RU's | RU's | RU's | |
| *Lot membrane* | 1081 | 1082 | 1083 | |
| | | | | |
| 3494 | 161 | 152 | 167 | |
| | | | | |
| *Mean* | 160 | | | |
| *Std Dev.* | 7,5 | | | |
| *CV %* | 4,7 | | | |
| | | | | |
| 2364 | 153 | 159 | 148 | |
| *Mean* | 153 | | | |
| *Std Dev.* | 5,5 | | | |
| *CV %* | 3,6 | | | |
| | | | | |
| 49435 | 153 | 140 | 153 | |
| *Mean* | 149 | | | |
| *Std Dev.* | 7,5 | | | |
| *CV %* | 5,0 | | | |

**Table 12: Detection of *Salmonella.***

| Feasibility of quantification | | | |
|---|---|---|---|
| Dilution factor | Counting of bacteria /ml | Reflectance units | Reflectance units |
| | | a) Volumes $2 \times 250\ \mu l$ | b) Volume $1 \times 500\ \mu l$ |
| 10-1 | $5,30 \times 10^7$ | 92 | 25 |
| 10-2 | $5,0 \times 10^8$ | 67 | |
| 10-3 | $5,80 \times 10^5$ | np | |
| 10-4 | $6,3 \times 10^4$ | 106 | 50 |
| 10-5 | $5,6 \times 10^3$ | 107 | |
| 10-6 | $5,9 \times 10^2$ | np | |
| 10-7 | $6,5 \times 10^1$ | np | |
| 10-8 | 7,0 | np | 96 |
| blank | | 170 | 170 |

**Table 13:** influence of the composition of the diluent buffer

| Sample | Buffer 1111 (A) | Buffer 1112 (A) | Buffer 1051 (B) | Buffer 1113 (C) |
|---|---|---|---|---|
| | Ru's | Ru's | Ru's | Ru's |
| 3 (1,2 mg/L) | 175 | 171 | 172 | 182 |
| | 166 | 175 | 177 | 182 |
| | 167 | 165 | 174 | 177 |
| | 74 | 169 | 170 | 178 |
| Mean | 170 | 170 | 176 | 180 |
| Std dev. | 4,9 | 4,2 | 3,0 | 2,6 |
| CV's % | 2,8 | 2,4 | 1,7 | 1,5 |
| | | | | |
| 15 (9,3 mg/L) | 152 | 151 | 157 | 157 |
| | 155 | 162 | 157 | 161 |
| | 159 | 153 | 152 | 160 |
| | 165 | 154 | 160 | 150 |
| Mean | 158 | 155 | 157 | 157 |
| Std dev. | 5,6 | 4,8 | 3,3 | 5,0 |
| CV's % | 3,6 | 3,1 | 2,1 | 3,2 |
| | | | | |
| 21 (20 mg/L) | 142 | 137 | 119 | 123 |
| | 128 | 142 | 137 | 136 |
| | 133 | 141 | 141 | 143 |
| | 137 | 136 | 122 | 130 |
| Mean | 135 | 139 | 130 | 132 |
| Std dev. | 5,9 | 2,9 | 1 0,8 | 9,7 |
| CV's % | 4,4 | 2,1 | 8,4 | 7,4 |
| | | | | |
| 36 (84 mg/L) | 101 | 103 | 102 | 89 |
| | 103 | 115 | 108 | 95 |
| | 102 | 117 | 99 | 94 |
| | 101 | 112 | 112 | 101 |
| Mean | 102 | 112 | 105 | 95 |
| Std dev. | 0,9 | 6,2 | 5,9 | 4,9 |
| CV's % | 0,9 | 5,5 | 5,6 | 5,2 |
| | | | | |
| 50 (268 mg/L) | 80 | 68 | 71 | 74 |
| | 79 | 70 | 91 | 69 |
| | 81 | 74 | 85 | 71 |
| | 77 | 71 | 80 | 65 |
| Mean | 79 | 71 | 82 | 70 |

(continued)

| Sample | Buffer 1111 (A) | Buffer 1112 (A) | Buffer 1051 (B) | Buffer 1113 (C) |
|---|---|---|---|---|
| Std dev. | 1,7 | 2,5 | 8,5 | 3,8 |
| CV's % | 2,2 | 3,5 | 10,3 | 5,4 |

**Table 14:** Correlation with another rapid method for the detection of CRP by immunoturbidimetry

**Table 14a:** Correlation on negative samples

| Number of samples | BIO ART conc. mg/L | ORION conc. mg/L |
|---|---|---|
| 12 | 0-9 | < 8 |
| 15 | 10 | < 8 |
| 3 | 10-25 | < 8 |

**Table 14b:** Correlation on low positive samples (conc. from 9 to 26 mg/L)

| 1. Sample n° | 2. BIO ART conc. mg/L | 3. ORION conc. mg/L | 4. correlation |
|---|---|---|---|
| 5. 5 | 6. 25 | 7. 19 | 8. Ok |
| 9. 7 | 10.10 | 11.9 | 12. Ok |
| 13. 10 | 14. 25 | 15. 16 | 16. Ok |
| 17. 11 | 18. 25 | 19. 19 | 20. Ok |
| 21.13 | 22.10 | 23.12 | 24. Ok |
| 25. 21 | 26. 10-25 | 27. 11 | 28. Ok |
| 29. 22 | 30.10-25 | 31. 26 | 32. Ok |
| 33. 26 | 34. 25 | 35.15 | 36. Ok |
| 37.30 | 38. 10-25 | 39. 13 | 40. Ok |
| 41. 31 | 42. 25-50 | 43. 25 | 44. Ok |
| 45.33 | 46.25 | 47.10 | 48. Ok |
| 49.42 | 50. 10 | 51. 18 | 52. Ok |

**Table 14c:** Correlation on positive samples between 27 and 100 mg/L

| Sample n° | BIO ART conc. mg/L | Orion conc. mg/L | Correlation |
|---|---|---|---|
| 14 | 75-100 | 77 | ok |
| 15 | 25-50 | 27 | ok |
| 18 | 25-50 | 50 | ok |
| 40 | 50 | 44 | ok |

**Table 14d**

| Sample n° | BIO ART conc. mg/L | Orion conc. mg/L |
|---|---|---|
| 37 | 200 | 112 |

**Table 14e:** general conclusion

| | | ORION * | |
|---|---|---|---|
| | | + results | - results |
| **BIO ART** | + results | 17 | 3 |
| | - results | 0 | 27 |
| Total | | 17 | 30 |
| Sensitivity : | | 17/17 100% | Specificity: 27/30 90% |

**Claims**

1. A method to coat an insoluble porous material with an analyte binding compound or complex comprising the steps of:

   a) immersing said material in a solution comprising said analyte binding compound or complex,
   b) immersing said material in a blocking-solution,
   c) drying the porous material,

   wherein said steps are performed in the absence of any intervening washing-offs.

2. A method for testing the presence of, or to quantify, an analyte in a sample, comprising the steps of:

   a) coating an insoluble porous material according to the method of claim 1,
   b) preparing a multilayer device comprising the porous material of step a),
   c) applying the sample onto the device of step b), wherein the (first) analyte binding compound or analyte binding complex binds the analyte present in said sample,
   d) forming a sandwich, wherein said analyte binds a second enzyme-labelled analyte binding compound or enzyme-labelled analyte binding complex,
   e) generating a colored deposit upon contact with a suitable precipitating substrate for said enzyme-label in a one step procedure, and,
   f) adding a fixing solution to stop the enzymatic reaction and allow the permanent retention of the resulting color.

3. The method according to claim 2, wherein the sample is applied in step c) to said test zone.

4. The method according to claim 2 wherein steps c) to f) are performed in the absence of intervening wash steps.

5. The method according to claim 2, wherein said enzyme label is HRP and said precipitating substrate is chosen from the group consisting of TMB (tetramethylbenzidine) or AEC (3-amino-9 ethylcarbazole).

6. The method according to claim 2, wherein said enzyme label is alkaline phosphatase and said substrate is chosen from the group consisting of BCIP (5-bromo-4-chloro-3-indolyl phosphate) or BCIP-NBT (5-bromo-4-chloro-3-indolyl phosphate-nitro blue tetrazolium).

7. The method according to claim 2, wherein said enzyme label is dehydrogenase and said substrate is NBT (nitro blue tetrazolium).

8. The method according to claim 5, wherein the formed TMB precipitate is able to be fixed using a reagent chosen from the group consisting of polyvinylic alcohol supplemented with dioctyl sulfosuccinate or dimethyl formamide.

9. The method according to any of claims 2 to 8, whereby volumes of 5 to 500 microlitres are applied to induce a homogenous coloured spot when a hole of 3-4 mm of diameter is present in the cover layer of said device.

10. The method according to claim 9, wherein 1% to 40% sucrose is added to the sample when the test zone is larger than 4 mm or if the sample volume is larger than 100 microlitres.

11. The method according to claims 9 or 10 wherein a zone, limited by the hole, has a diameter of 1, 2, 3, 4, 5, 6, 7, 8, 9, 10, 11, 12, 13, 14, 15, 16, 17, 18, 19 or 20 mm and
wherein the sample volume is 5 to 2000 microlitres.

12. The method according to claim 2, wherein water or food samples are controlled, tested and/or determined.

13. The method according to claim 2, wherein food-borne pathogens are detected and/or identified.

14. The method according to claim 2, used to detect an auto-immune disease marker, infectious disease marker, allergen, cardiac disease marker, inflammation marker, or bacterial infection marker.

15. The method according to claim 2, applied to the diagnosis and/or monitoring of treatment of diseases.

16. The method according to claim 15, wherein said diseases include auto-immune diseases, infectious diseases, allergens, cardiac diseases, inflammation and bacterial infections.

17. The method according to any of the claims 1 to 16, wherein the blocking solution contains 0.2-10% blocking agent.

18. The method according to claim 17, wherein the blocking agent is BSA.

**Patentansprüche**

1. Verfahren zum Beschichten eines unlöslichen porösen Materials mit einer/einem Analyt-bindenden Verbindung oder Komplex, umfassend die Schritte:

   a) Eintauchen des Materials in eine Lösung, die die/den Analyt-bindende(n) Verbindung oder Komplex umfasst,
   b) Eintauchen des Materials in eine blockierende Lösung,
   c) Trocknen des porösen Materials,

   wobei die Schritte in Abwesenheit jeglicher zwischenzeitlicher Waschungen durchgeführt werden.

2. Verfahren zum Prüfen des Vorhandenseins von oder zum Quantifizieren eines Analyten in einer Probe, umfassend die Schritte:

   a) Beschichten eines unlöslichen porösen Materials gemäß dem Verfahren nach Anspruch 1,
   b) Herstellen einer mehrlagigen Vorrichtung, die das poröse Material aus Schritt a) umfasst,
   c) Aufbringen der Probe auf die Vorrichtung aus Schritt b), wobei die (erste) Analyt-bindende Verbindung oder der (erste) Analyt-bindende Komplex den in der Probe vorhandenen Analyt bindet,
   d) Bilden eines Sandwiches, wobei der Analyt eine zweite enzymmarkierte Analyt-bindende Verbindung oder einen zweiten enzymmarkierten Analyt-bindenden Komplex bindet,
   e) Erzeugen eines farbigen Niederschlags nach Kontakt mit einem geeigneten präzipitierenden Substrat für die Enzymmarkierung in einem Einschritt-Verfahren, und,
   f) Zufügen einer fixierenden Lösung, um die enzymatische Reaktion zu stoppen und die dauerhafte Erhaltung der resultierenden Farbe zu erlauben.

3. Verfahren nach Anspruch 2, wobei die Probe in Schritt c) auf den Testbereich aufgetragen wird.

4. Verfahren nach Anspruch 2, wobei die Schritte c) bis f) in Abwesenheit von zwischenzeitlichen Waschschritten durchgeführt werden.

5. Verfahren nach Anspruch 2, wobei die Enzymmarkierung HRP ist und das präzipitierende Substrat ausgewählt ist aus TMB (Tetramethylbenzidin) oder AEC (3-Amino-9-ethylcarbazol).

6. Verfahren nach Anspruch 2, wobei die Enzymmarkierung alkalische Phosphatase ist und das Substrat ausgewählt ist aus BCIP (5-Brom-4-chlor-3-indolylphosphat) oder BCIP-NBT (5-Brom-4-chlor-3-indolylphosphat-Nitroblautetrazolium).

**7.** Verfahren nach Anspruch 2, wobei die Enzymmarkierung Dehydrogenase ist und das Substrat NBT (Nitroblautetrazolium) ist.

**8.** Verfahren nach Anspruch 5, wobei das erzeugte TMB-Präzipitat fixiert werden kann unter Verwendung eines Reagens ausgewählt aus Polyvinylalkohol, ergänzt mit Dioctylsulfosuccinat oder Dimethylformamid.

**9.** Verfahren nach einem der Ansprüche 2 bis 8, wobei Volumina von 5 bis 500 Mikrolitern angewendet werden, um einen homogenen farbigen Fleck zu erzeugen, wenn in der Deckschicht der Vorrichtung ein Loch von 3 bis 4 mm Durchmesser vorhanden ist.

**10.** Verfahren nach Anspruch 9, wobei 1% bis 40% Saccharose der Probe zugeführt wird, wenn der Testbereich größer als 4 mm ist oder wenn das Probenvolumen größer als 100 Mikroliter ist.

**11.** Verfahren nach den Ansprüchen 9 oder 10, wobei ein Bereich, begrenzt durch das Loch, einen Durchmesser von 1, 2, 3, 4, 5, 6, 7, 8, 9, 10, 11, 12, 13, 14, 15, 16, 17, 18, 19 oder 20 mm aufweist und wobei das Probenvolumen 5 bis 2000 Mikroliter beträgt.

**12.** Verfahren nach Anspruch 2, wobei Wasser oder Nahrungsproben kontrolliert, getestet und/oder bestimmt werden.

**13.** Verfahren nach Anspruch 2, wobei durch Nahrung übertragene Krankheitserreger entdeckt und/oder identifiziert werden.

**14.** Verfahren nach Anspruch 2, verwendet um einen Autoimmunerkrankungsmarker, Infektionserkrankungsmarker, ein Allergen, einen Herzerkrankungsmarker, Entzündungsmarker oder bakteriellen Infektionsmarker zu ermitteln.

**15.** Verfahren nach Anspruch 2, angewendet zur Diagnose und/oder Überwachung einer Behandlung von Krankheiten.

**16.** Verfahren nach Anspruch 15, wobei die Krankheiten Autoimmunerkrankungen, Infektionserkrankungen, Allergene, Herzerkrankungen, Entzündung und bakterielle Infektionen einschließen.

**17.** Verfahren nach einem der Ansprüche 1 bis 16, wobei die blockierende Lösung 0,2 - 10% Blockierungsmittel enthält.

**18.** Verfahren nach Anspruch 17, wobei das Blockierungsmittel BSA ist.

**Revendications**

**1.** Procédé d'enduction d'un matériau poreux insoluble avec un composé ou complexe se liant à un analyte, qui comprend les étapes suivantes :

    a) immerger ledit matériau dans une solution comprenant ledit composé ou complexe se liant à un analyte,
    b) immerger ledit matériau dans une solution de blocage,
    c) sécher le matériau poreux,

dans lequel lesdites étapes sont réalisées en l'absence de tout lavage intermédiaire.

**2.** Procédé de test en présence de, ou de quantification, d'un analyte dans un échantillon, qui comprend les étapes suivantes :

    a) enduire un matériau poreux insoluble selon le procédé de la revendication 1,
    b) préparer un dispositif à couches multiples comprenant le matériau poreux de l'étape a),
    c) appliquer l'échantillon sur le dispositif de l'étape b), dans lequel le (premier) composé se liant à un analyte ou complexe se liant à un analyte se lie à l'analyte présent dans ledit échantillon,
    d) former un sandwich, dans lequel ledit analyte se lie à un second composé se liant à un analyte marqué par une enzyme ou complexe se liant à un analyte marqué par une enzyme,
    e) générer, lors du contact avec un substrat précipitant adapté pour ledit marqueur enzymatique, un dépôt coloré en une procédure en une étape, et,

f) ajouter une solution de fixation pour mettre un terme à la réaction enzymatique et permettre la rétention permanente de la couleur résultante.

3. Procédé selon la revendication 2, dans lequel l'échantillon est appliqué dans l'étape c) à ladite zone de test.

4. Procédé selon la revendication 2, dans lequel les étapes c) à f) sont réalisées en l'absence d'étapes de lavage intermédiaires.

5. Procédé selon la revendication 2, dans lequel ledit marqueur enzymatique est la HRP et ledit substrat précipitant est choisi dans le groupe constitué par la TMB (tétraméthylbenzidine) ou l'AEC (3-amino-9-éthylcarbazole).

6. Procédé selon la revendication 2, dans lequel ledit marqueur enzymatique est une phosphatase alcaline et ledit substrat est choisi dans le groupe constitué par le BCIP (5-bromo-4-chloro-3-indolyl phosphate) ou le BCIP-NBT (5-bromo-4-chloro-3-indolyl phosphate-nitrobleu de tétrazolium).

7. Procédé selon la revendication 2, dans lequel ledit marqueur enzymatique est une déshydrogénase et ledit substrat est le NBT (nitrobleu de tétrazolium).

8. Procédé selon la revendication 5, dans lequel le précipité de TMB formé est capable d'être fixé en utilisant un réactif choisi dans le groupe constitué d'alcool polyvinylique supplémenté de dioctyl sulfosuccinate ou de diméthylformamide.

9. Procédé selon l'une quelconque des revendications 2 à 8, moyennant lequel des volumes de 5 à 500 microlitres sont appliqués pour induire une tache colorée homogène lorsqu'un trou de 3 à 4 mm de diamètre est présent dans la couche de couverture dudit dispositif.

10. Procédé selon la revendication 9, dans lequel de 1 % à 40 % de sucrose sont ajoutés à l'échantillon lorsque la zone de test est plus grande que 4 mm ou si le volume de l'échantillon est plus grand que 100 microlitres.

11. Procédé selon les revendications 9 ou 10, dans lequel une zone, limitée par le trou, a un diamètre de 1, 2, 3, 4, 5, 6, 7, 8, 9, 10, 11, 12, 13, 14, 15, 16, 17, 18, 19 ou 20 mm et dans lequel le volume de l'échantillon est de 5 à 2000 microlitres.

12. Procédé selon la revendication 2, dans lequel des échantillons d'eau ou de nourriture sont contrôlés, testés et/ou déterminés.

13. Procédé selon la revendication 2, dans lequel des pathogènes d'origine alimentaire sont détectés et/ou identifiés.

14. Procédé selon la revendication 2, utilisé pour détecter un marqueur de maladie auto-immune, un marqueur de maladie infectieuse, un allergène, un marqueur de maladie cardiaque, un marqueur d'inflammation, ou un marqueur d'infection bactérienne.

15. Procédé selon la revendication 2, appliqué au diagnostic et/ou à la surveillance du traitement de maladies.

16. Procédé selon la revendication 15, dans lequel ladite maladie comprend les maladies auto-immunes, les maladies infectieuses, les allergènes, les maladies cardiaques, les inflammations et les infections bactériennes.

17. Procédé selon l'une quelconque des revendications 1 à 16, dans lequel la solution de blocage contient de 0,2 à 10 % d'agent de blocage.

18. Procédé selon la revendication 17, dans lequel l'agent de blocage est la BSA.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0207152 A
- US 5958790 A
- US 5616467 A
- EP 0458231 A
- EP 0125118 A

### Non-patent literature cited in the description

- **KOHLER, F. ; MILSTEIN, C.** Continuous cultures of fused cells secreting antibody of predefined specificity. *Nature,* 1995, vol. 256, 495-497
- **MERRIFIELD, B.** Concept and early development of solid-phase peptide synthesis. *Methods Enzymol.,* 1997, vol. 289, 3-13
- **LEMOINE F.M.** Intérèts clinique de la recherche des auto-anticorps. *Medical trends,* 1992, vol. 6
- Centre hospitalier du Luxembourg. **HUMBEL RL.** Cources related to auto-immunity. Université de Liège, 1990
- **ABUAF N. ; JOHANET C. ; HOMBERG JC.** Les autoanticorps anti-tissues non spécifiques d'organe. *2e journée d'immunopathologie de l'hôpital Rothschild OPTION BIO n°51*
- **STURGESS, A.** *Recently characterised autoantibodies and their clinical significance Aust NZ J Med,* 1992, vol. 22, 279-289
- **MC NEIL HP ; KRILIS SA.** *Antiphospholipid antibodies Aust Nz J Med,* 1991, vol. 21, 463-475
- **DE ROOIJ et al.** Use of recombinant RNP peptides 70K and A in an ELISA for measurement of antibodies in mixed connective tissue disease : a longitudinal follow up of 18 patients. *Ann Rheum Dis,* 1990, vol. 49, 391-395
- **WHITTIGHAM S. et al.** Serological diagnosis of primary Sjögren syndrome by means of recombinant La (SSB) as nuclear antigen. *Lancet,* 1987, vol. 8549, 1-3
- **MATHEWS MB ; BERNSTEIN RM.** Myositis autoantibody inhibits histidyl tRNA synthetase. A model for autoimmunity. *Nature,* 1983, vol. 304, 177-179
- **LE ROY et al.** Scleroderma (systemic sclerosis); classification, subsets and pathogenesis. *J Rheumatol.,* 1988, vol. 15, 202-205
- **TAN et al.** Anti-nuclear antibodies (ANA's) : diagnosistically specific immune markers and clues towards the understanding of systemic auto-immunity. *Clin Immunol Immunopath,* 1988, vol. 47, 121-141
- Antinuclear antibodies : diagnostic markers for auto-Immune diseases and probes for cell biology. **TAN ENG.** Advances in Immunology. Academic Press, 1989, vol. 44, 93-151
- **HOCHBERG MC et al.** Systemic Lupus Erythematosus : a review of clinico-laboratory features and immunogenetic markers in 150 patients with emphasis on demographic subsets. *Medicine,* 1985, vol. 64, 285-295
- **LINDBACK S et al.** The value of C-reactive protein as a marker of bacterial infection in patients with septicaemia, endocarditis and influenza. *Scand J Infect Dis,* 1989, vol. 21, 543-9
- **MORLEY J ; KUSHNER I.** Serum C-reactive protein levels in disease. *Ann NY Acad Sci,* 1982, vol. 389, 406-18
- **WHICHER J et al.** Acute phase response of serum amyloid A protein and C-reactive protein to the common cold and influenza. *J Clin Pathol,* 1985, vol. 38, 312-6
- **PEPYS M ; BALTZ M.** Acute phase proteins with special reference to C-reactive protein and related proteins (pentaxins) and serum amyloid A protein. *Adv Immunol,* 1983, vol. 34, 141-212
- **CHAMBERS RE et al.** Acute phase protein in inflammatory disease. *Lab,* 1988, vol. 1, 29-37
- **MC CABE RE ; REMINGTON JS.** C-reactive protein in patients which bacteremia. *J Clin Microbiol,* 1984, vol. 20, 317-9
- **MACKIE et al.** C-reactive protein for rapid diagnosis of infection in leukaemia. *J Clin Pathol,* 1979, vol. 32, 1253-6
- **ANGERMAN N et al.** *C-reactive protein in the evaluation of antibiotic therapy for pelvic infections J Reprod Med,* 1980, vol. 25, 65-7
- **PHILIPS A ; ANDREWS P.** apid determination of C-reactive protein levels; semi-quantitative versus quantitative. *J. Paediatrics,* 1987, vol. 110, 263-8
- **KOMORSKI et al.** Quantitative measurement of C-reactive protein in acute otitis media. *Clin Lab Observ,* 1987, vol. 111, 81-4

- **CLAUS DR et al.** Radioimmunoassay of human C-reactive protein and levels in normal sera. *J lab Clin Med,* 1976, vol. 87, 120-8
- **GRÜTZEIMER S ; VON SCHENK H.** Four immuno-chemical methods for measuring C-reactive protein in plasma compared. *Clin Chem,* 1989, vol. 35, 461-3
- **WADSWORTH C ; WADSWORTH E.** Efficacy of latex agglutination and quantification methods for determination of C-reactive protein in pediatric sera. *Clin Chem Acta,* 1984, vol. 138, 309-18
- **COLLET-CASSART D et al.** A quantitative C-reactive protein assay using latex agglutination in microtiter plates. *J Immunol Methods,* 1989, vol. 125, 137-41
- **HULMAN G et al.** An accurate, simple and rapid test for detecting elevated levels of C-reactive protein in serum by agglutination of fat emulsion. *Clin Chim Acta,* 1986, vol. 156, 337-40
- **PRICE CP et al.** Development and validation of a particle enhanced turbidimetric assay for C-reactive protein. *J Immunol Methods,* 1987, vol. 99, 205-11
- **HIGHTON J ; HESSIAN P.** A solid-phase enzyme immunoassy for C-reactive protein : clinical value and the effect of rheumatoid factor. *J Immunol Methods,* 1984, vol. 68, 185-92
- **KÄPYAHO K.** Rapid determination of C-reactive protein by enzyme immunoassay using two monoclonal antibodies. *Scand J Clin Lab Invest,* 1989, vol. 49, 389-93
- **BUITAGO MG et al.** Clinical evaluation of a fluorescence polarization immunoassay for quantifying C-reactive protein (Tech Brief. *Clin Chem,* 1988, vol. 34, 595-6
- **HJORTDAHL P et al.** C-reactive protein : a new rapid assay for managing infectious disease in primary health care. *Scand J Prim Health Care,* 1991, vol. 8, 20-6
- **URDAL P et al.** Rapid Immunometric measurement of C-reactive protein in whole blood. *Clin Chem,* 1992, vol. 38, 580-4
- Autoantibody patterns in Primary Biliary Cirrhosis. **P.A. BERG ; R. KLEIN.** Auto-immune liver diseases. Raven Press, 1991, 121-142
- **BERG E.L ; R. KLEIN.** Antimitochondrial antibodies in primary Biliary Cirrhosis and other disorders: definition and clinical relevance. *Dig. Dis.,* 1992, vol. 10, 857-907
- **BAMPTON et al.** Measurement of Rheumatoid factors by an anzyme-linked immunosorbent assay and comparison with other methods. *Annals of Rheumatic Diseases,* 1985, vol. 44, 13-19
- **STONE R. et al.** Clinical value of ELISA assays for IgM and IgG Rgeumatic factors. *J. Clin. Pathol,* 1987, vol. 40, 107-111
- **KUBY.** J. Immunology. W.H. Freeman and Company, 1992
- **MYGIND N.** Essential Allergy. *Blackwell Scientific Publications,* 1986
- Dodet Allergy: une cascade de reactions. *BIOFUTUR,* July 1989
- **DAVID B.** Allergenes et desensibilisation. *Rev.Fr.Allergol.,* 1989, vol. 26, 29-37
- **O'BYRNE P.M.** Allergen-induced airway hyperresponsiveness. *J.Allergy Clin Immunol.,* 1988, vol. 81, 119-127
- **COCKCROFT DW et al.** Allergen-induced increase in non-allergic bronchial reactivity. *Clin Allergy,* 1977, vol. 7, 503-513
- **PLATTS-MILLS TAE et al.** Bronchial hyper-reactivity and allergen exposure. *Prog.Resp.Res.,* 1985, vol. 19, 276-284
- **BELLANTI JA.** Prevention of food-allergies. *Ann of Allergy,* 1984, vol. 53, 683-688
- Validation of analyses methods in food microbiology. **LECLERCQ, A.** Proceedings of the sixth conference in food microbiology. University of Liège, 21 June 2001
- Recherche et dénombrement de Listeria monocytogenes : critères microbiologiques et méthodes. **LECLERC,V ; VINCENT-RACE,C et al.** Proceedings of the sixth conference in food microbiology. University of Liège, 21 June 2001
- Travaux de l'Agence française de sécurité sanitaire des aliments sur les tests de croissance de Listeria monocytogenes dans les denrées alimentaires. **DANAN,C ; CORNU,M et al.** Proceedings of the sixth conference in food microbiology. University of Liège, 21 June 2001
- **COIA, J.** Clinical, microbiological and epidemiological aspects of Escherichia coli O157 infection. *FEMS Immunology and Medical Microniology,* 1998, vol. 20, 1-9
- **KERR, M. ; FITZGERALD, M. et al.** *Survival of Escherichia coli 0157:H7 in bottled natural mineral water J.Appl.Microbiol.,* vol. 87, 833-841
- **SMITH, P. ; ILLINGWORTH,D.S. ; COATES, D.** Evaluation of the EiaFoss assay for the detection of the Escherichia coli 0157 in food, faeces and environmental samples. Conference proceedings on ''methods for Verocytotoxigenic E. coli'' organised by an EU concerted Action on VTEC (CT 98-3935. Western General Hospital, 25 November 1998
- **VERNOZY-ROZAND.** C. Detection of Escherichia coli O157:H7 and other verocytotoxin producing E.coli (VTEC) in food. *J.Appl.Microb.,* vol. 82, 537-551
- Salmonella spp. detection In foods : validation study of the Transla ™ plate salmonella Gold. **FRANÇOIS, JY ; JACOB, B et al.** Proceedings of the sixth conference in food microbiology. University of Liège, 21 June 2001
- **VAN DER ZEE H ; HUIS IN'T VELD JHJ.** Rapid and alternative screening methods for microbiological analysis. *J AOAC Int,* 1997, vol. 4, 934-940

- **ROMBOUTS FM et al.** Rapid detection of foodborne pathogens. *Med Fac Landbouww,* 1995, 1771-1776
- **PAFFARD SM ; MILES RJ et al.** A rapid and sensitive enzyme linked immunofilter assay (ELIFA) for whole bacterial cells. *Journal of Immunological methods,* 1996, vol. 192, 133-136
- **BERNAL CS ; ROBINSON G et al.** Development of a sensitive and quantitative enzyme-linked immunofilter assay (ELIFA) for whole bacterial cells. *Journal of microbiological methods,* 1994, vol. 19, 135-143
- **ANDREWS W.H.** Evolution of methods for the detection of Salmonella in food. *J AOAC Int 1,* 1996, 4-12
- **BLACKBUM C.** Rapid and alternative method for the detection of Salmonella in food. *J App Bacteriol,* 1993, vol. 75, 199-215
- **FELDSLNE, PT ; FALBO-NELSON, MT et al.** Visual immunoprecipitate assay (VIP) for the detection of enterohemorrhagic Escherichia Coli (EHEC) 0157:H7 in selected foods : collaborative study. *J AOAC Int,* 1997, vol. 80, 517-529
- **CHAPAMN PA ; ELLIN M ; ASHTON R.** *A comparison of immunomagnetic separation and culture Reveal and VIP for the detection of E.coli 0157 in enrichment cultures of naturally-contaminated raw beef, lamb and mixed meat products*
- **FELDSINE, PT;LIENAU et al.** Visual immunoprecipitate assay (VIP) for the detection for Listeria monocytogenes and related Listeria species in selected food. : collaborative study. *J AOAC Int,* 1997, vol. 80, 517-529
- **BRAKSTAD OG ; MAELAND JA.** Detection of staphylococcus aureus with biotinylated monoclonal antibodies directed against staphylococcal Tnase complexed to avidin-peroxidase in a rapid sandwich linked immunofiltration assay (sELIFA. *J Med Microbiol,* 1993, vol. 39, 128-134
- **DUPONT H ; THERASSE J et al.** Detection of staphylococcal enterotoxin - B A comparative study of ELISA and ELIFA systems. *Journal Of Immunological methods,* 1990, vol. 128, 287-291
- **SCHENIDER E ; USLEBER E ; MARTLAUBER E.** Rapid detection of Fumonisin B-1 in corn-based food by competitive direct dipstick enzyme-immunoassay, enzyme-linked immunofiltration assay with integrated negative control reaction. *Journal of Agricultural and Food Chemistry,* 1995, vol. 43, 2548-2552
- **AUBERT D ; LEPAN H et al.** Rapid detection of toxoplasmic nucleic-acid by enzyme-linked immunofiltration- assay after membrane transfer. *Electrophoresis,* 1995, vol. 16, 354-356
- **AUBERT D ; TOUBAS D et al.** Accelerated detection of DNA on membranes by automated enzyme-linked immunofiltration assay. *Analytical Biochemistry,* 1997, vol. 247, 25-29
- **RUBIN II.** *Handbook of plastic materials and technology,* 1990, 575-589
- **SCHOUTEN AE. ; VAN DER VEGT.** Plastics. Delta Press, 1987, 106-124